# EUROPEAN PATENT APPLICATION

(11) **EP 4 825 864 A1**
(43) Date of publication of application: **21.10.2026**
(21) Application number: 24867060.6
(22) Date of filing: 18.07.2024
(51) Int. Cl.: C07H 19/067, C07H 21/02, C07H 1/00, A61K 31/7084, A61P 25/00, A61P 37/06, A61P 31/12

(54) **MODIFIED NUCLEOTIDE COMPOUND, OLIGONUCLEOTIDE THEREOF, AND USE THEREOF**

(30) Priority: 18.09.2023 CN 202311202774; 07.02.2024 CN 202410174938
(71) Applicant: BeBetter Med Inc., Guangzhou, Guangdong 510660 (CN)
(72) Inventor: LIU, Xinjian, Guangzhou, Guangdong 510660 (CN); FAN, Fushun, Guangzhou, Guangdong 510660 (CN); CAI, Xiong, Guangzhou, Guangdong 510660 (CN); QING, Yuanhui, Guangzhou, Guangdong 510660 (CN); WENG, Yunwo, Guangzhou, Guangdong 510660 (CN); GAO, Mingzhang, Guangzhou, Guangdong 510660 (CN); QIAN, Changgeng, Guangzhou, Guangdong 510660 (CN)
(74) Representative: Fuchs Patentanwälte Partnerschaft mbB
(86) International application number: PCT/CN2024/106181
(87) International publication number: WO 2025/060642

(57) **Abstract**

The present invention discloses a modified nucleotide compound having a structure shown in formula (I) or a pharmaceutically acceptable salt thereof or a stereoisomer thereof, an oligonucleotide thereof, and use thereof. Connecting the modified nucleotide compound provided herein to the 5'-terminus of an oligonucleotide can enhance the activity of the oligonucleotide in inhibiting gene expression and can improve the stability of the oligonucleotide *in vivo,* thereby effectively improving its druggability *in vivo.*

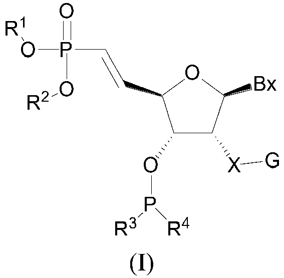

## Description

The present invention claims priority to Chinese Patent Application No. 202311202774.8, entitled "MODIFIED NUCLEOTIDE COMPOUND, OLIGONUCLEOTIDE THEREOF AND USE THEREOF" and filed with China National Intellectual Property Administration on September 18, 2023, and Chinese Patent Application No. 202410174938.9, entitled "MODIFIED NUCLEOTIDE COMPOUND, OLIGONUCLEOTIDE THEREOF AND USE THEREOF" and filed with China National Intellectual Property Administration on February 07, 2024, the entire contents of which are incorporated herein by reference.

### TECHNICAL FIELD

The present invention belongs to the field of biomedicine, and particularly relates to a modified nucleotide compound, an oligonucleotide thereof and use thereof.

### BACKGROUND ART

In 1998, Fire and Mello discovered RNA interference (RNAi) is a very promising gene therapy tool. Small nucleic acid therapies such as small interfering RNA (siRNA), microRNA (miRNA), and antisense oligonucleotides (ASOs) are all potential gene silencing tools and have shown therapeutic prospects for a wide range of diseases. Exogenously introduced double-stranded siRNAs are composed of a sense strand and an antisense strand, which can be recognized by the endogenous RNAi mechanism to exert gene silencing function. siRNA binds to Argonaute 2 protein (AGO2) and other auxiliary protein residues to form an RNA-induced silencing complex (RISC). The activated RISC contains an antisense strand that specifically targets the target mRNA, and then AGO2 cuts the mRNA to achieve effective gene silencing (Matzke et al. Nature reviews. Genetics vol. 6,1 (2005): 24-35.). RNAi-based therapy is the most effective and reliable tool for inhibiting target genes. Currently, many diseases including cancer, neurodegenerative diseases, autoimmune diseases and viral infections have been explored using siRNA therapy (Gangopadhyay S et al. RNA Biol. 2022;19(1):452-467.).

There are still several challenges in siRNA therapy, including limited tolerance to nucleases in the body, off-target effects, difficulty in specific delivery to specific tissues or organs, and poor drug properties (Hammond SM et al., EMBO Mol Med. 2021;13(4):e13243.). To address these challenges, chemical modifications have made significant contributions to the drug properties of siRNA and played an indispensable role in its clinical success. For example, nearly all recently approved siRNA drugs have undergone extensive ribose 2' position modifications, including 2'-O-methylation and 2'-fluoro modification, which have a strong impact on the physicochemical properties and overall efficacy of siRNA. In addition, the delivery of siRNA drugs is also an important guarantee for RNAi therapy. Currently, the most mature delivery system is N-acetylgalactosamine (GalNAc). GalNAc can bind to the asialoglycoprotein receptor (ASGPR) specifically expressed by hepatocytes. Conjugation of GalNAc with siRNA enables active delivery to hepatocytes (Debacker, Alexandre J et al. Molecular Therapy vol. 28,8 (2020): 1759-1771).

Notably, when double-stranded siRNA is loaded into RISC, the sense strand is removed while the antisense strand remains stably bound to the RISC. The 5'-phosphate (5'-P) of the antisense strand can anchor to a binding pocket in the middle domain of Ago2 protein and is considered a key factor for efficient siRNA loading into RISC (Schirle NT et al., Science. 2012;336(6084):1037-1040.). However, the above chemical modifications, especially the GalNAc delivery system, will cause the natural 5'-P to be rapidly hydrolyzed by lysosomal acid phosphatase during endocytic transport. Therefore, it is urgent to modify 5'-P of the antisense strand to improve its metabolic stability (Nair JK et al., J Am Chem Soc. 2014;136(49):16958-16961.). It has been reported that 5'-P analog 5'-(E)-vinylphosphonate (5'-VP) is a modification that can be used to stabilize single-stranded RNAi (ssRNAi) (Prakash TP et al., Nucleic Acids Res. 2015;43(6):2993-3011.). Subsequently, researchers tested four siRNA-GalNAc conjugates targeting apolipoprotein B (ApoB) and found that replacing 5'-P with the stable analog 5'-VP could increase siRNA silencing effect by up to 20-fold *in vitro;* in mice, 5'-VP could increase the *in vivo* activity of phosphate-dependent conjugates by up to 3-fold (Parmar R et al., Chembiochem. 2016;17(11):985-989.). It is evident that the metabolically stable 5'-VP can improve the stability of siRNA by promoting siRNA loading into RISC, thereby enhancing the *in vivo* activity of siRNA-GalNAc conjugates, especially phosphate-dependent sequences. Currently, the most widely used 5'-VP modified nucleotides are mostly modified with 2'-O-methyl.

### SUMMARY OF INVENTION

Based on these findings, the present invention provides a new class of 5'-VP modified nucleotide compounds that can enhance the activity of oligonucleotides in inhibiting gene expression.

In one aspect, the present invention provides a modified nucleotide compound having a structure shown in formula (I) or a pharmaceutically acceptable salt thereof or a stereoisomer thereof, 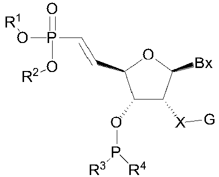 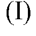 wherein,
R¹ and R² are each independently selected from: H, C₁-C₆ alkyl, C₂-C₆ alkenyl, C₂-C₆ alkynyl, C₃-C₈ cycloalkyl, and C₃-C₈ cycloalkylmethyl;
R³ is selected from: -OR⁵;
R⁴ is selected from: -N(R⁶)(R⁷);
R⁵ is selected from: H, C₁-C₆ alkyl, C₁-C₆ alkyl substituted with cyano, C₁-C₆ alkyl substituted with halogen, C₁-C₆ alkyl substituted with hydroxyl, C₁-C₆ alkyl substituted with C₁-C₆ alkoxy, C₁-C₆ alkyl substituted with amino, and C₁-C₆ alkyl substituted with C₁-C₆ alkylamino;
R⁶ and R⁷ are each independently selected from: C₁-C₆ alkyl, C₂-C₆ alkenyl, C₂-C₆ alkynyl, C₃-C₈ cycloalkyl, and C₃-C₈ cycloalkylmethyl;
X is selected from: -C(R⁸)(R⁹)-, -S-;
R⁸ and R⁹ are each independently selected from: H, C₁-C₆ alkyl, and halogen;
G is selected from: H, halogen, cyano, C₁-C₁₀ alkyl, C₂-C₁₀ alkenyl, C₂-C₁₀ alkynyl, C₃-C₈ cycloalkyl, C₃-C₈ cycloalkylmethyl, C₁-C₁₀ alkoxy, C₁-C₁₀ alkyl substituted with halogen, C₁-C₁₀ alkyl substituted with hydroxyl, C₁-C₁₀ alkyl substituted with C₁-C₆ alkoxy, C₁-C₁₀ alkyl substituted with amino, C₁-C₁₀ alkyl substituted with C₁-C₆ alkylamino, and C₁-C₁₀ alkyl substituted with cyano; Bx is a heterocyclic base.

In some embodiments, R¹ and R² are each independently selected from: H, C₁-C₃ alkyl, C₂-C₃ alkenyl, C₂-C₃ alkynyl, C₃-C₆ cycloalkyl, and C₃-C₆ cycloalkylmethyl;

In some embodiments, R¹ and R² are each independently selected from: H, methyl, ethyl, n-propyl, and isopropyl.

In some embodiments, R⁵ is selected from: H, C₁-C₃ alkyl, C₁-C₃ alkyl substituted with cyano, C₁-C₃ alkyl substituted with halogen, C₁-C₃ alkyl substituted with hydroxyl, C₁-C₃ alkyl substituted with C₁-C₃ alkoxy, C₁-C₃ alkyl substituted with amino, and C₁-C₃ alkyl substituted with C₁-C₃ alkylamino. In some embodiments, R⁵ is selected from: H, methyl, ethyl, n-propyl, isopropyl, methyl substituted with cyano, ethyl substituted with cyano, and n-propyl substituted with cyano.

In some embodiments, R⁶ and R⁷ are each independently selected from: C₁-C₃ alkyl, C₂-C₃ alkenyl, C₂-C₃ alkynyl, C₃-C₆ cycloalkyl, and C₃-C₆ cycloalkylmethyl;

In some embodiments, R⁶ and R⁷ are each independently selected from: methyl, ethyl, n-propyl, and isopropyl.

In some embodiments, X is selected from: -S-, -CH₂-, -C(H)(F)-, and -CF₂-.

In some embodiments, G is selected from: H, halogen, cyano, C₁-C₆ alkyl, C₂₋C₆ alkenyl, C₂-C₆ alkynyl, C₃-C₆ cycloalkyl, C₃-C₆ cycloalkylmethyl, C₁-C₆ alkoxy, C₁-C₆ alkyl substituted with halogen, C₁-C₆ alkyl substituted with hydroxyl, C₁-C₆ alkyl substituted with C₁-C₆ alkoxy, C₁-C₆ alkyl substituted with amino, C₁-C₆ alkyl substituted with C₁-C₆ alkylamino, and C₁-C₆ alkyl substituted with cyano;

In some embodiments, G is selected from: H, halogen, cyano, C₁-C₃ alkyl, C₁-C₃ alkyl substituted with halogen, C₂-C₃ alkenyl, C₂-C₃ alkynyl, C₁-C₃ alkoxy, and C₁-C₃ alkyl substituted with C₁-C₃ alkoxy.

In some embodiments, G is selected from: H, methyl, ethyl, propyl, isopropyl, monofluoromethyl, difluoromethyl, trifluoromethyl, monofluoroethyl, difluoroethyl, vinyl, propenyl, methoxy, ethoxy, methoxymethyl, methoxyethyl, cyano, fluorine, and chlorine.

In some embodiments, X is -S-; G is selected from: C₁-C₃ alkyl.

In some embodiments, X is -CH₂-; G is selected from: C₁-C₃ alkyl, C₂-C₃ alkenyl, C₂-C₃ alkynyl, C₁-C₂ alkyl substituted with C₁-C₃ alkoxy, methyl substituted with fluorine, cyano, fluorine, and chlorine.

In some embodiments, the -X-G formed by X and G together is selected from: methylthio, ethylthio, propylthio, methyl, ethyl, propyl, butyl, allyl, methoxymethyl, methoxyethyl, methoxypropyl, ethoxymethyl, ethoxyethyl, ethoxypropyl, methyl substituted with cyano, ethyl substituted with cyano, monofluoromethyl, difluoromethyl, monofluoroethyl, and difluoroethyl.

In some embodiments, Bx is selected from: adenine, guanine, cytosine, uracil, thymine, methyladenine, 1-methylguanine, isoguanine, 7-methylguanine, methylcytosine, isocytosine, pseudouracil, 5-hydroxymethylcytosine, hypoxanthine, Q-substituted adenine, Q-substituted guanine, Q-substituted cytosine, Q-substituted uracil, and Q-substituted thymine; wherein Q is selected from: H, benzoyl, and benzyloxymethyl.

In some embodiments, the modified nucleotide compound is selected from: 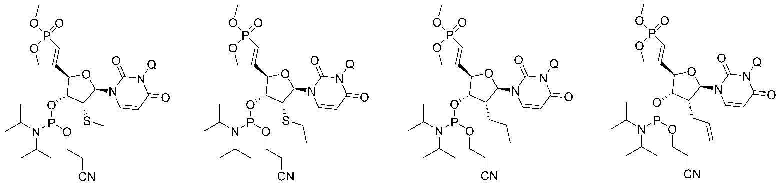 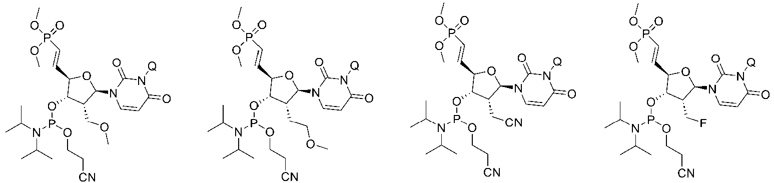 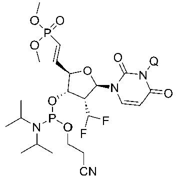 wherein, Q is selected from: H, benzoyl, and benzyloxymethyl.

In a second aspect, the present invention provides an oligonucleotide, wherein at least one 5'-terminal nucleotide monomer is connected to the oligonucleotide by the modified nucleotide compound via a linking group; wherein the oligonucleotide has the structural formula as follows: 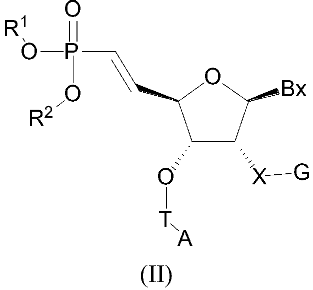 wherein, R¹, R², Bx, X, and G are as described above;
A is the nucleotide chain in the oligonucleotide excluding the 5'-terminal nucleotide monomer;
T is the linking group connecting the modified nucleotide compound to the internucleoside of the oligonucleotide.

In some embodiments, T is selected from: a phosphodiester bond, and a phosphorothioate bond.

In some embodiments, the oligonucleotide is selected from: siRNA, shRNA, antisense oligonucleotide, microRNA, anti-microRNA, antimir, supermir, antagomir, ribozyme, triplex-forming oligonucleotide, bait oligonucleotide, splice-switching oligonucleotide, immunostimulatory oligonucleotide, RNA activator, U1 adaptor and CRISPR Cas, or a combination thereof.

In some embodiments, the oligonucleotide is siRNA.

In some embodiments, the modified nucleotide compound is connected to the 5'-terminus of the siRNA antisense strand via a T linking group.

In a third aspect, the present invention provides use of the modified nucleotide compound and the oligonucleotide, including:
Use of the modified nucleotide compound in the preparation of an oligonucleotide for inhibiting or activating gene expression.

In some embodiments, the oligonucleotide is selected from: siRNA, shRNA, antisense oligonucleotide, microRNA, anti-microRNA, antimir, supermir, antagomir, ribozyme, triplex-forming oligonucleotide, bait oligonucleotide, splice-switching oligonucleotide, immunostimulatory oligonucleotide, RNA activator, U1 adaptor and CRISPR Cas, or a combination thereof.

In some embodiments, the oligonucleotide is siRNA.

In some embodiments, the modified nucleotide compound is connected to the 5'-terminus of the siRNA antisense strand via a covalent linking group.

In some embodiments, the covalent linking group is a phosphodiester bond or a phosphorothioate bond.

Use of the modified nucleotide compound described herein, or the oligonucleotide described herein, in the preparation of a drug for inhibiting or activating gene expression.

In a fourth aspect, the present invention provides a pharmaceutical preparation for inhibiting or activating gene expression, which is prepared from an active ingredient and a pharmaceutically acceptable excipient, wherein the active ingredient comprises the oligonucleotide described herein.

In a fifth aspect, the present invention provides a method for inhibiting or activating gene expression, which comprises the following steps: administering a safe and effective amount of the oligonucleotide described herein and/or the pharmaceutical preparation described herein.

The present invention obtains a new class of 5'-VP modified nucleotide compounds by performing different 2'-modifications on 5'-VP modified nucleotide compounds. The present invention finds that 2'-modification has a great influence on the druggability of oligonucleotides. Connecting the modified nucleotide compound provided herein to the 5'-terminus of an oligonucleotide can enhance the activity of the oligonucleotide in inhibiting gene expression and can improve the stability of the oligonucleotide *in vivo*, thereby effectively improving its druggability *in vivo*.

### BRIEF DESCRIPTION OF DRAWINGS

Figure 1 shows the effects of differently modified 11040 sequences on the expression of hPCSK9 in the serum of AAV8-hPCSK9 mice.
Figure 2 shows the effects of differently modified W4005 sequences on the expression of hCIDEB in the liver of AAV8-hCIDEB mice.

### DESCRIPTION OF EMBODIMENTS

The technical solution of the present invention is further illustrated by specific embodiments below. Those skilled in the art should understand that the embodiments are only to help understand the present invention and should not be regarded as specific limitations of the present invention.

Unless otherwise defined, all technical and scientific terms used in the present invention have the same meaning as those commonly understood by those skilled in the art of the present invention. The terms used in the Description of the present invention are only for the purpose of describing specific embodiments and are not intended to limit the present invention.

The terms "including" and "having" and any variations thereof used in the present invention are intended to cover a non-exclusive inclusion. For example, a process, method, device, product, or equipment comprising a series of steps is not limited to the listed steps or modules, but may optionally further include steps not listed, or may optionally further include other steps inherent to the process, method, product, or equipment.

The term "multiple" as used herein refers to two or more. The term "and/or" describes the association relationship of the associated objects, indicating that three relationships may exist. For example, A and/or B can represent: A exists alone, A and B exist at the same time, and B exists alone. The character "/" generally indicates that the associated objects are in an "or" relationship.

In the compounds described herein, when any variable occurs more than once in any component, each occurrence of that variable is defined independently of every other occurrence. Likewise, combinations of substituents and variables are permitted, provided such combinations render the compound stable. A line drawn from a substituent to a ring system indicates that the indicated bond may be attached to any substitutable atom of the ring. If the ring system is polycyclic, this means that the bond is attached only to any suitable carbon atom of the adjacent ring. It is to be understood that ordinarily skilled persons in the art may select substituents and substitution patterns for the compounds of the present invention to provide chemically stable compounds that can be readily synthesized from readily available raw materials by techniques of the art and the methods described below. If a substituent is itself substituted with more than one group, it is to be understood that these groups may be on the same carbon atom or on different carbon atoms, provided that the structure remains stable.

The term "alkyl" as used herein is intended to include branched and straight-chain saturated aliphatic hydrocarbon groups having a specific number of carbon atoms. For example, "C₁-C₆ alkyl" includes groups having 1, 2, 3, 4, 5 or 6 carbon atoms in a straight-chain or branched arrangement. For example, "C₁-C₆ alkyl" specifically includes methyl, ethyl, n-propyl, isopropyl, n-butyl, tert-butyl, isobutyl, pentyl and hexyl.

The term "cycloalkyl" refers to a monocyclic saturated aliphatic hydrocarbon group having a specific number of carbon atoms. For example, "cycloalkyl" includes cyclopropyl, cyclobutyl, cyclopentyl, or cyclohexyl.

The term "alkoxy" refers to a group in which an alkyl group is directly linked to oxygen, i.e., a group having an -O-alkyl structure, such as -OCH₃, -OCH₂CH₃, -OCH₂CH₂CH₃, -O-CH₂CH(CH₃)₂, -OCH₂CH₂CH₂CH₃ and -O-CH(CH₃)₂.

The term "heterocyclic base" refers to the base moiety constituting a nucleoside, most commonly purines and pyrimidines, such as adenine, guanine, cytosine, uracil and thymine, derivatives thereof such as methyladenine, 1-methylguanine, isoguanine, 7-methylguanine, methylcytosine, isocytosine, pseudouracil, 5-hydroxymethylcytosine and hypoxanthine, and substitutes thereof such as substituted adenine, substituted guanine, substituted cytosine, substituted uracil and substituted thymine.

The term "substituted" refers to the replacement of a hydrogen atom in a specific structure with a specified substituent group.

As will be appreciated by those skilled in the art, the terms "halogen" or "halo" as used herein refer to chlorine, fluorine, bromine and iodine.

The present invention includes free forms of the compound of formula (I), as well as pharmaceutically acceptable salts thereof and stereoisomers thereof. Stereoisomers described herein refer to (depending on their structure) enantiomers, diastereomers, syn-/anti-isomers, cis-/trans-isomers, epimers, and (E)/(Z)-isomers. The compound of formula I may be used in the context of the present invention in the form of pure stereoisomers or in the form of any mixture of stereoisomers.

The term "free form" refers to an amine compound in a non-salt form. The included pharmaceutically acceptable salts refer not only to exemplary salts of the specific compounds described herein, but also to all typical pharmaceutically acceptable salts of the free forms of the compound of formula (I) or (II). The free form of the specific salt of the compound may be isolated using techniques known in the art. For example, the free form can be regenerated by treating the salt with a suitable dilute aqueous solution of a base, such as a dilute aqueous solution of NaOH, a dilute aqueous solution of potassium carbonate, a dilute aqueous solution of ammonia, and a dilute aqueous solution of sodium bicarbonate. The free form is somewhat different from its respective salt form in certain physical properties, such as solubility in polar solvents, but for the purpose of the present invention, such acid and base salts are equivalent to their respective free forms in other pharmaceutical aspects.

Pharmaceutically acceptable salts of the present invention can be synthesized from compounds of the present invention containing a basic or acidic moiety by conventional chemical methods Typically, salts of basic compounds are prepared by ion exchange chromatography or by reacting a free base with a stoichiometric or excess amount of an inorganic or organic acid in the desired salt form in an appropriate solvent or combination of solvents. Similarly, salts of acidic compounds are formed by reacting with an appropriate inorganic or organic base.

Therefore, pharmaceutically acceptable salts of the compounds of the present invention include conventional non-toxic salts of the compounds of the present invention formed by reacting basic compounds of the present invention with inorganic or organic acids. For example, conventional non-toxic salts include salts derived from inorganic acids such as hydrochloric acid, hydrobromic acid, sulfuric acid, sulfamic acid, phosphoric acid and nitric acid, and also include salts prepared from organic acids such as acetic acid, propionic acid, succinic acid, glycolic acid, stearic acid, lactic acid, malic acid, tartaric acid, citric acid, ascorbic acid, pamoic acid, maleic acid, hydroxymaleic acid, phenylacetic acid, glutamic acid, benzoic acid, salicylic acid, p-aminobenzenesulfonic acid, 2-acetoxybenzoic acid, fumaric acid, toluenesulfonic acid, methanesulfonic acid, ethanedisulfonic acid, oxalic acid, isethionic acid and trifluoroacetic acid.

If the compound of the present invention is acidic, then suitable "pharmaceutically acceptable salts" refer to salts prepared by pharmaceutically acceptable non-toxic bases, including inorganic and organic bases, and salts derived from inorganic bases include aluminum salts, ammonium salts, calcium salts, copper salts, iron salts, ferrous salts, lithium salts, magnesium salts, manganese salts, manganous salts, potassium salts, sodium salts and zinc salts. Ammonium salts, calcium salts, magnesium salts, potassium salts, and sodium salts are particularly preferred. Salts derived from pharmaceutically acceptable organic non-toxic bases include salts of primary, secondary and tertiary amines; substituted amines include naturally occurring substituted amines, cyclic amines, and basic ion exchange resins, such as arginine, betaine, caffeine, choline, N,N'-dibenzylethylenediamine, diethylamine, 2-diethylaminoethanol, 2-dimethylaminoethanol, aminoethanol, ethanolamine, ethylenediamine, N-ethylmorpholine, N-ethylpiperidine, glucosamine, histidine, hydroxocobalamin, isopropylamine, lysine, methylglucosamine, morpholine, piperazine, piperidine, guanidine, polyamine resin, procaine, purine, theobromine, triethylamine, trimethylamine, tripropylamine and tromethamine.

Berg et al., "Pharmaceutical Salts," J. Pharm. Sci. 1977: 66: 1-19 describes in more detail the preparation of the pharmaceutically acceptable salts described above and other typical pharmaceutically acceptable salts.

Since under physiological conditions, the deprotonated acid moieties such as carboxyl groups in the compounds may be anionic, and this charge may then be balanced by internal protonated or alkylated basic moieties carrying cations such as the quaternary nitrogen atom, it should be noted that the compounds of the present invention are potential inner salts or zwitterions.

The pharmaceutical preparation or method for inhibiting or activating gene expression provided by the present invention comprises (administering to a patient or subject) an active ingredient (i.e., the oligonucleotide described herein) within a safe and effective amount, and a pharmaceutically acceptable excipient. When administering the drug, a safe and effective amount of the active ingredient is administered to a mammal (such as a human) in need of treatment, where the dosage administered is a pharmaceutically effective dosage. Of course, the specific dosage should also take into account factors such as the route of administration and the patient's health status, which are all within the skill range of skilled physicians.

Among them, "safe and effective amount" means: the amount of active ingredient is sufficient to significantly improve the condition without causing serious side effects. "Pharmaceutically acceptable excipient" refers to one or more compatible solid or liquid fillers or gelling substances that are suitable for human use and must have sufficient purity and sufficiently low toxicity. "Compatibility" as used herein means that the components in the composition can be mixed with the active ingredient (oligonucleotide) of the present invention and with each other without significantly reducing the efficacy of the active ingredient.

The pharmaceutically acceptable excipient includes, but is not limited to, one or more of the following materials: at least one of solvents, vehicles, fillers, extenders, binders, humectants, disintegrants, release-retarding agents, absorption accelerators, adsorbents, diluents, solubilizers, emulsifiers, lubricants, wetting agents, suspending agents, flavoring agents, and fragrances.

Examples of pharmaceutically acceptable excipient include cellulose and its derivatives (such as sodium carboxymethyl cellulose, sodium ethyl cellulose and cellulose acetate), gelatin, talc, solid lubricants (such as stearic acid and magnesium stearate), calcium sulfate, vegetable oils (such as soybean oil, sesame oil, peanut oil and olive oil), polyols (such as propylene glycol, glycerol, mannitol and sorbitol), emulsifiers (such as Tween^{®}), wetting agents (such as sodium dodecyl sulfate), colorants, flavoring agents, stabilizers, antioxidants, preservatives, and pyrogen-free water.

The targeted delivery ligand or other types of delivery carriers of the present invention are preferably N-acetylgalactosamine derivatives (GalNAc carriers). Other types of delivery carriers, such as liposomes that specifically deliver siRNA to hepatocytes, may also be used in the present invention as long as they can achieve siRNA delivery.

Currently, GalNAc carriers have been extensively studied. GalNAc-nucleic acid is a monoconjugate formed by a carbohydrate with a nucleic acid. N-acetylated galactosamine is covalently conjugated to the 3'- and/or 5'-terminus of the sense strand of siRNA of different sequences in a trivalent state to form a GalNAc-siRNA drug, thereby achieving specific delivery to hepatocytes and allowing the drug to enter the cells and exert its function through endocytosis.

In the following embodiments, the exemplary structures of the GalNAc ligand and GalNAc-siRNA conjugate (the ligand is conjugated to the 3'-terminus of the sense strand) are as follows:

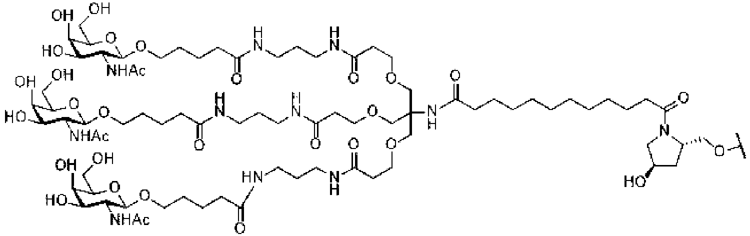

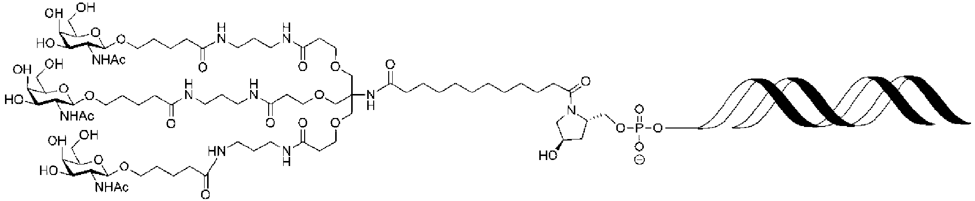

The above 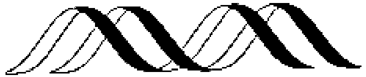 represents double-stranded RNA, such as the siRNA of the present invention.

In the nucleic acid sequence listing of the present invention, the abbreviations and structures of the nucleotide monomers used are as follows:

| | |
|---|---|
| A | Adenosine-3'-phosphate |
| T | 5'-Methyluridine-3'-phosphate |
| C | Cytidine-3'-phosphate |
| G | Guanosine-3'-phosphate |
| U | Uridine-3'-phosphate |
| dA | 2'-Deoxyadenosine-3'-phosphate |
| dT | 2'-Deoxythymidine-3'-phosphate |
| dC | 2'-Deoxycytidine-3'-phosphate |
| dG | 2'-Deoxyguanosine-3'-phosphate |
| dU | 2'-Deoxyuridine-3'-phosphate |
| mA | 2'-O-methyladenosine-3'-phosphate |
| mC | 2'-O-Methylcytidine-3'-phosphate |
| mG | 2'-O-methylguanosine-3'-phosphate |
| mU | 2'-O-methyluridine-3'-phosphate |
| fA | 2'-Fluoroadenosine-3'-phosphate |
| fC | 2'-Fluorocytidine-3'-phosphate |
| fG | 2'-Fluoroguanosine-3'-phosphate |
| fU | 2'-Fluorouridine-3'-phosphate |
| VPU | 2'-O-methyluridine-5'-(E)-vinylphosphonate-3'-phosphate |
| VPU-S | 2'-S-methyluridine-5'-(E)-vinylphosphonate-3'-phosphate |
| VPU-6 | 2'-Propyluridine-5'-(E)-vinylphosphonate-3'-phosphate |
| VPU-7 | 2'-Allyluridine-5'-(E)-vinylphosphonate-3'-phosphate |
| VPU-8 | 2'-Methoxymethyluridine-5'-(E)-vinylphosphonate-3'-phosphate |
| VPU-9 | 2'-Cyanomethyluridine-5'-(E)-vinylphosphonate-3'-phosphate |
| VPU-10 | 2'-Fluoromethyluridine-5'-(E)-vinylphosphonate-3'-phosphate |
| VPU-11 | 2'-Methoxyethyluridine-5'-(E)-vinylphosphonate-3'-phosphate |
| VPU-12 | 2'-S-ethyluridine-5'-(E)-vinylphosphonate-3'-phosphate |
| VPU-13 | 2'-Fluoroethyluridine-5'-(E)-vinylphosphonate-3'-phosphate |
| VPU-14 | 2'-Difluoroethyluridine-5'-(E)-vinylphosphonate-3'-phosphate |
| isoGNA-A | Adenosine-isoglycerol nucleotide |
| isoGNA-T | Thymidine-isoglycerol nucleotide |
| isoGNA-C | Cytidine-isoglycerol nucleotide |
| isoGNA-G | Guanosine-isoglycerol nucleotide |
| isoGNA-U | Uridine-isoglycerol nucleotide |
| Invab | Inverted abasic residue |
| UNA | Unlocked nucleic acid nucleotide |
| * | Phosphorothioate modifications |

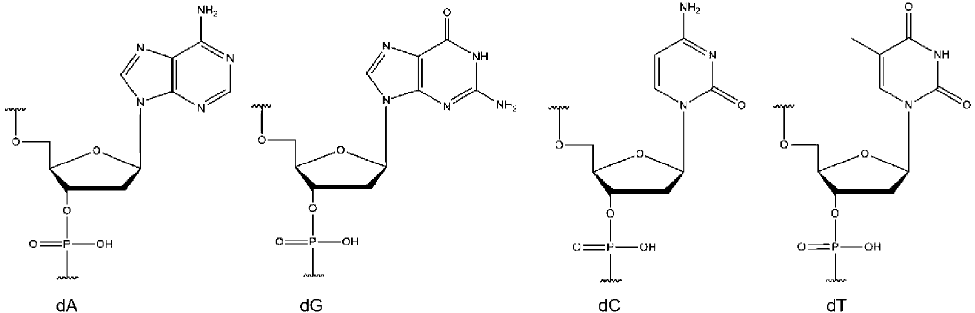

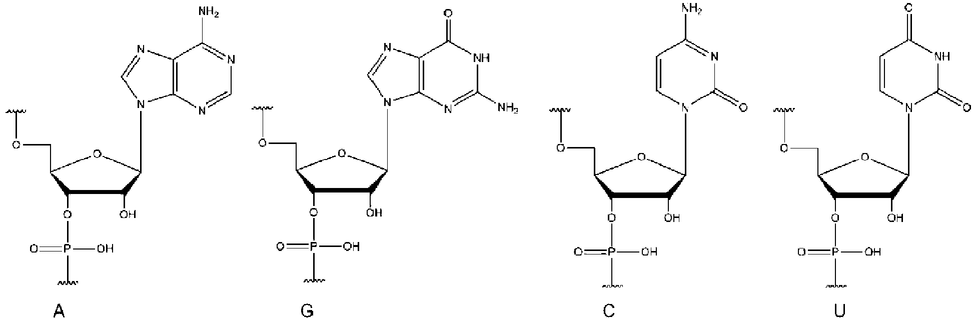

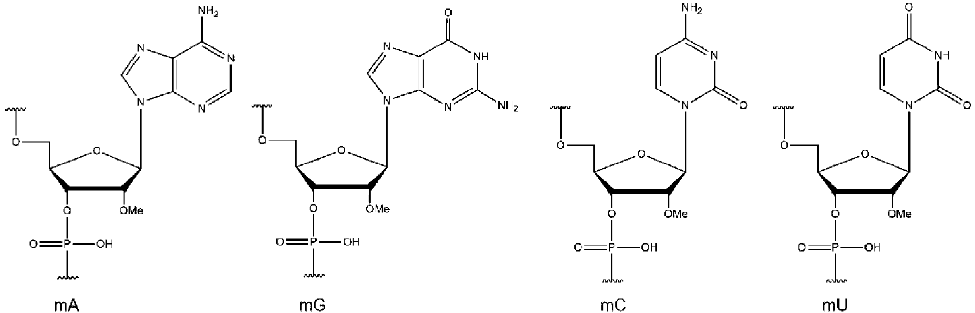

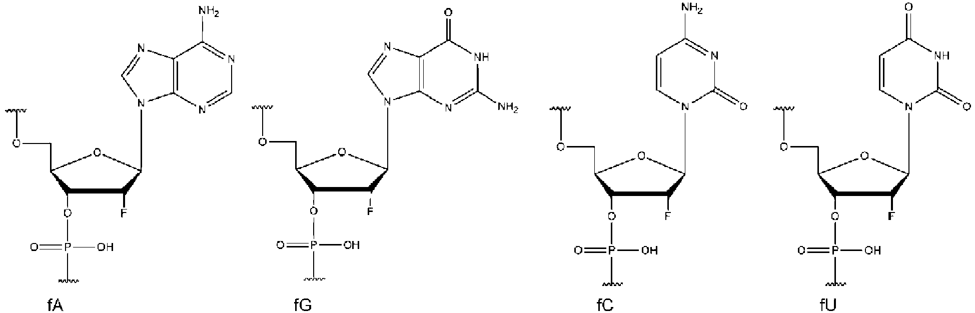

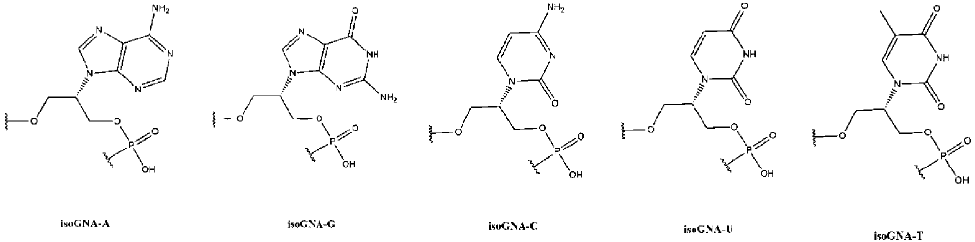

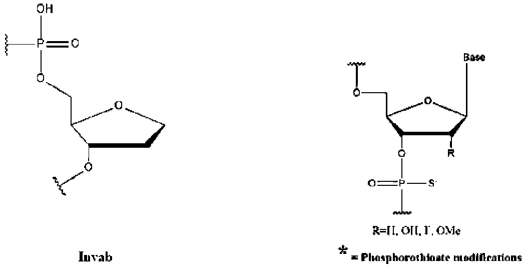

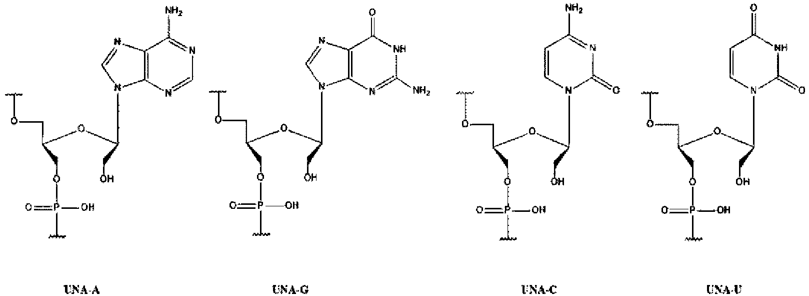

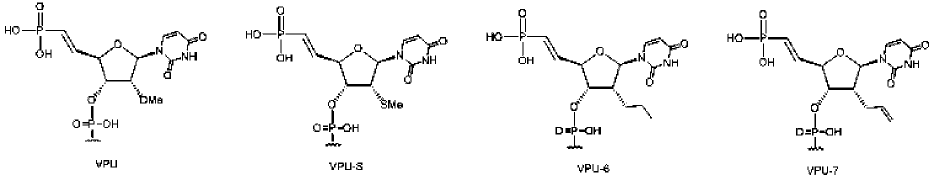

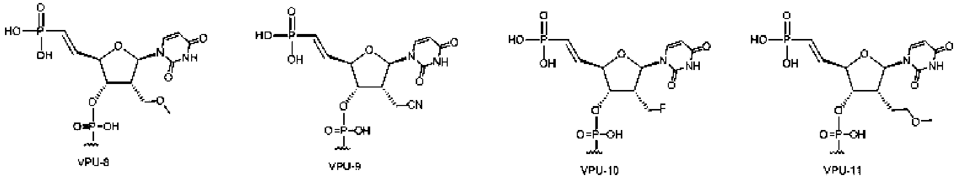

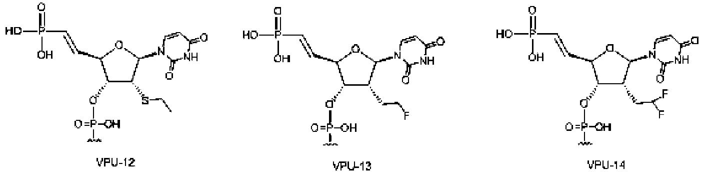

The following are specific embodiments.

### Embodiment 1: Preparation of (2R,3R,4R,5R)-5-(3-benzoyl-2,4-dioxo-3,4-dihydropyrimidin-1(2H)-yl)-2-((E)-2-(dimethoxyph osphoryl)vinyl)-4-(methylthio)tetrahydrofuran-3-yl(2-cyanoethyl)diisopropylphosphoramidite (Compound 1)

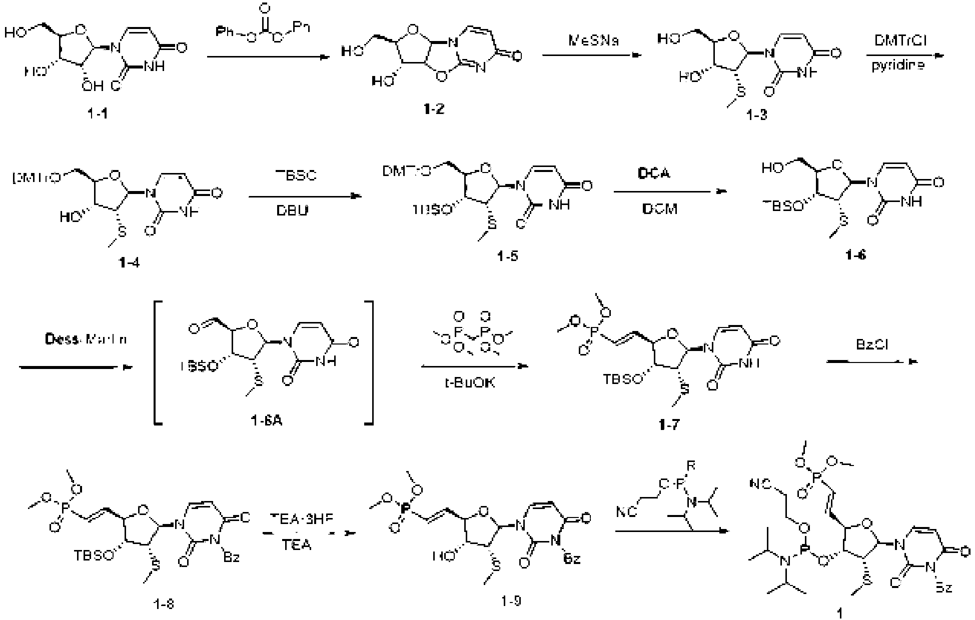

Step 1a: Preparation of (2R,3R,9aR)-3-hydroxy-2-(hydroxymethyl)-2,3,3a,9a-tetrahydro-6H-furo[2',3':4,5]oxazolo[3,2-a]p yrimidin-6-one (Compound **1-2**): A mixture of 1-((2R,3R,4S,5R)-3,4-dihydroxy-5-(hydroxymethyl)tetrahydrofuran-2-yl)pyrimidine-2,4(1H,3H)-di one (Compound **1-1**) (30 g, 122.9 mmol), diphenyl carbonate (31.6 g, 147.5 mmol) and sodium bicarbonate (825 mg, 9.8 mmol) in N,N-dimethylformamide (150 mL) was stirred at 100°C for 4 h. After cooling to room temperature, the mixture was added dropwise to methyl tert-butyl ether (1.5 L) and stirred for 15 min. The solid was filtered, dispersed in methanol (150 mL) and stirred for 15 min. The solid was filtered and dried in vacuo to give (2R,3R,9aR)-3-hydroxy-2-(hydroxymethyl)-2,3,3a,9a-tetrahydro-6H-furo[2',3':4,5]oxazolo[3,2-a]p yrimidin-6-one as an off-white solid (23.0 g, yield: 83.0%). LCMS (ESI): m/z=227[M+1]⁺.

### Step 1b: Preparation of 1-((2R,3R,4R,5R)-4-hydroxy-5-(hydroxymethyl)-3-(methylthio)tetrahydrofuran-2-yl)pyrimidine-2, 4(1H,3H)-dione (Compound 1-3):

(2R,3R,9aR)-3-hydroxy-2-(hydroxymethyl)-2,3,3a,9a-tetrahydro-6H-furo[2',3':4,5]oxazolo[3,2-a]p yrimidin-6-one (Compound **1-2**) (10 g, 44.2 mmol) was dissolved in N,N-dimethylformamide (100 mL), sodium thiomethoxide (14.8 g, 212.4 mmol) was added and the mixture was stirred at room temperature for 1 h. 6 mol/L aqueous hydrochloric acid solution was added to adjust the pH to 6-7.

The mixture was filtered, the filtrate was concentrated, and the residue was purified by silica gel column chromatography (eluent: dichloromethane/methanol = 20:1) to give 1-((2R,3R,4R,5R)-4-hydroxy-5-(hydroxymethyl)-3-(methylthio)tetrahydrofuran-2-yl)pyrimidine-2, 4(1H,3H)-dione as a light yellow solid (12 g, yield: 99.0%). LCMS (ESI): m/z=275[M+1]⁺.

Step 1c: Preparation of 1-((2R,3R,4R,5R)-5-((bis(4-methoxyphenyl)(phenyl)methoxy)methyl)-4-hydroxy-3-(methylthio)tet rahydrofuran-2-yl)pyrimidine-2,4(1H,3H)-dione (Compound **1-4**): Under a nitrogen atmosphere, 1-((2R,3R,4R,5R)-4-hydroxy-5-(hydroxymethyl)-3-(methylthio)tetrahydrofuran-2-yl)pyrimidine-2, 4(1H,3H)-dione (Compound **1-3**) (12 g, 43.8 mmol) was dissolved in anhydrous pyridine (120 mL), 4,4'-dimethoxytrityl chloride (17.8 g, 52.6 mmol) was added and the mixture was stirred at room temperature for 1 h. The reaction mixture was concentrated. The residue was dissolved in dichloromethane, washed with water, dried over anhydrous sodium sulfate, and concentrated in vacuo. The residue was purified by silica gel column chromatography (eluent: petroleum ether/dichloromethane/ethyl acetate = 1:1:1) to give 1-((2R,3R,4R,5R)-5-((bis(4-methoxyphenyl)(phenyl)methoxy)methyl)-4-hydroxy-3-(methylthio)tet rahydrofuran-2-yl)pyrimidine-2,4(1H,3H)-dione as a yellow foamy solid (13.4 g, yield: 53.1%). LCMS(ESI): m/z=575[M-1]⁻. ¹H NMR (500 MHz, DMSO-d6) δ 11.35 (s, 1H), 7.55 (d, *J* = 8.1 Hz, 1H), 7.42 (d, *J* = 7.7 Hz, 2H), 7.28 (tt, *J* = 20.0, 7.4 Hz, 7H), 6.96 - 6.87 (m, 4H), 5.97 (d, *J* = 5.2 Hz, 1H), 5.71 (d, *J* = 4.2 Hz, 1H), 5.37 (d, *J* = 8.1 Hz, 1H), 4.54 (td, *J* = 6.3, 3.2 Hz, 1H), 4.19 (q, *J* = 4.8 Hz, 1H), 3.74 (s, 6H), 3.40 - 3.35 (m, 1H), 3.30 - 3.24 (m, 2H).

Step 1d: Preparation of 1-((2R,3R,4R,5R)-5-((bis(4-methoxyphenyl)(phenyl)methoxy)methyl)-4-((tert-butyldimethylsilyl)o xy)-3-(methylthio)tetrahydrofuran-2-yl)pyrimidine-2,4(1H,3H)-dione (Compound **1-5**): 1-((2R,3R,4R,5R)-5-((bis(4-methoxyphenyl)(phenyl)methoxy)methyl)-4-hydroxy-3-(methylthio)tet rahydrofuran-2-yl)pyrimidine-2,4(1H,3H)-dione (Compound **1-4**) (13.4 g, 23.3 mmol) and 1,8-diazabicyclo[5.4.0]undec-7-ene (7.3 mL, 48.8 mmol) were dissolved in anhydrous dichloromethane (270 mL), tert-butyldimethylsilyl chloride (7.0 g, 46.5 mmol) was added and the mixture was stirred overnight at room temperature. The mixture was washed with water, dried over anhydrous sodium sulfate and concentrated in vacuo. The residue was purified by silica gel column chromatography (eluent: petroleum ether/ethyl acetate = 10:1) to give 1-((2R,3R,4R,5R)-5-((bis(4-methoxyphenyl)(phenyl)methoxy)methyl)-4-((tert-butyldimethylsilyl)o xy)-3-(methylthio)tetrahydrofuran-2-yl)pyrimidine-2,4(1H,3H)-dione as a white foamy solid (10.2 g, yield: 63.3%). LCMS(ESI): m/z=689[M-1]⁻. ¹H NMR (500 MHz, CDCl₃) δ 8.62 (d, *J* = 11.6 Hz, 1H), 7.50 (d, *J* = 8.2 Hz, 1H), 7.41 - 7.31 (m, 2H), 7.23 (t, *J* = 8.8 Hz, 3H), 7.19 - 7.15 (m, 2H), 7.11 (dd, *J* = 13.0, 5.8 Hz, 1H), 7.04 (d, *J* = 8.7 Hz, 1H), 6.77 - 6.66 (m, 4H), 5.63 (t, *J* = 2.9 Hz, 1H), 5.28 (dd, *J* = 8.1, 2.2 Hz, 1H), 4.57 - 4.39 (m, 1H), 4.28 - 4.19 (m, 1H), 3.67 (s, 6H), 3.44 (dd, *J* = 10.7, 5.7 Hz, 1H), 3.28 (dd, *J* = 10.6, 3.1 Hz, 1H), 3.00 (ddd, *J* = 78.1, 6.6, 4.5 Hz, 1H), 1.92 (d, *J* = 12.7 Hz, 3H), 0.77 (d, *J* = 9.6 Hz, 9H), 0.06 - -0.10 (m, 6H).

Step 1e: Preparation of 1-((2R,3R,4R,5R)-4-((tert-butyldimethylsilyl)oxy)-5-(hydroxymethyl)-3-(methylthio)tetrahydrofura n-2-yl)pyrimidine-2,4(1H,3H)-dione (Compound **1-6**): 1-((2R,3R,4R,5R)-5-((bis(4-methoxyphenyl)(phenyl)methoxy)methyl)-4-((tert-butyldimethylsilyl)o xy)-3-(methylthio)tetrahydrofuran-2-yl)pyrimidine-2,4(1H,3H)-dione (Compound **1-5**) (9.18 g, 13.3 mmol) was dissolved in dichloromethane (92 mL), dichloroacetic acid (4.4 mL, 53.2 mmol) was added and the mixture was stirred at room temperature for 1 h. A saturated aqueous sodium bicarbonate solution was added to adjust the reaction mixture to pH 7-8. The layers were separated. The organic layer was washed with a saturated aqueous sodium chloride solution, dried over anhydrous sodium sulfate and concentrated in vacuo. The residue was purified by silica gel column chromatography (eluent: dichloromethane/methanol = 30:1) to give 1-((2R,3R,4R,5R)-4-((tert-butyldimethylsilyl)oxy)-5-(hydroxymethyl)-3-(methylthio)tetrahydrofura n-2-yl)pyrimidine-2,4(1H,3H)-dione as a yellow solid (4.47 g, yield: 86.6%). LCMS(ESI): m/z=[M+1]⁺. ¹H NMR (500 MHz, DMSO-d6) δ 11.26 (s, 1H), 7.85 (d, *J* = 8.2 Hz, 1H), 5.61 (dd, *J* = 8.4, 3.3 Hz, 2H), 5.01 (t, *J* = 4.4 Hz, 1H), 4.27 - 4.16 (m, 2H), 3.59 (tdd, *J* = 11.9, 10.1, 8.1, 4.4 Hz, 2H), 2.05 (s, 3H), 0.75 (s, 9H), -0.00 (s, 3H), -0.12 (s, 3H).

Step 1f: Preparation of dimethyl((E)-2-((2R,3R,4R,5R)-3-((tert-butyldimethylsilyl)oxy)-5-(2,4-dioxo-3,4-dihydropyrimidin -1(2H)-yl)-4-(methylthio)tetrahydrofuran-2-yl)vinyl)phosphonate (Compound **1-7**): 1-((2R,3R,4R,5R)-4-((tert-butyldimethylsilyl)oxy)-5-(hydroxymethyl)-3-(methylthio)tetrahydrofura n-2-yl)pyrimidine-2,4(1H,3H)-dione (Compound **1-6**) (4.4 g, 11.3 mmol) was dissolved in anhydrous dichloromethane (120 mL), Dess-Martin periodinane (7.21 g, 17.0 mmol) was added at 0-5°C, and the mixture was stirred at room temperature for 3 h. The reaction was quenched with a saturated aqueous sodium thiosulfate solution, and a saturated aqueous sodium bicarbonate solution was added to adjust the pH to 7-8. After the layers were separated, the organic layer was dried over anhydrous sodium sulfate and concentrated in vacuo to give **1-6A** (crude). Tetramethyl methylenediphosphonate (6.54 g, 22.7 mmol) was dissolved in anhydrous tetrahydrofuran (100 mL), potassium tert-butoxide (2.54 g, 22.7 mmol) was added at 0-5°C and the mixture was stirred at room temperature for 15 min. A solution of 1-6A in anhydrous tetrahydrofuran (20 mL) was added, and the mixture was stirred at room temperature for 1 h. The reaction mixture was added to a saturated sodium chloride solution and extracted with dichloromethane. The organic layer was dried over anhydrous sodium sulfate and concentrated under reduced pressure. The residue was purified by silica gel column chromatography (eluent: dichloromethane/acetone = 5:1) to give dimethyl((E)-2-((2R,3R,4R,5R)-3-((tert-butyldimethylsilyl)oxy)-5-(2,4-dioxo-3,4-dihydropyrimidin -1(2H)-yl)-4-(methylthio)tetrahydrofuran-2-yl)vinyl)phosphonate as an off-white foamy solid (2.6 g, yield: 46.7%). LCMS (ESI): m/z=493[M+1]⁺.

Step 1g: Preparation of dimethyl((E)-2-((2R,3R,4R,5R)-5-(3-benzoyl-2,4-dioxo-3,4-dihydropyrimidin-1(2H)-yl)-3-((tert-bu tyldimethylsilyl)oxy)-4-(methylthio)tetrahydrofuran-2-yl)vinyl)phosphonate (Compound **1-8**): Benzoyl chloride (453 µL, 3.90 mmol) was added to a mixture of dimethyl((E)-2-((2R,3R,4R,5R)-3-((tert-butyldimethylsilyl)oxy)-5-(2,4-dioxo-3,4-dihydropyrimidin -1(2H)-yl)-4-(methylthio)tetrahydrofuran-2-yl)vinyl)phosphonate (Compound **1-7**) (1.6 g, 3.25 mmol) and N,N-diisopropylethylamine (545 mg, 4.23 mmol) in anhydrous dichloromethane (32 mL). The mixture was stirred at room temperature overnight. The reaction mixture was washed with a saturated aqueous sodium chloride solution, dried over anhydrous sodium sulfate and concentrated in vacuo. The residue was purified by silica gel column chromatography (eluent: petroleum ether/dichloromethane/methanol = 50:50:1 to dichloromethane/methanol = 50:1) to give dimethyl((E)-2-((2R,3R,4R,5R)-5-(3-benzoyl-2,4-dioxo-3,4-dihydropyrimidin-1(2H)-yl)-3-((tert-bu tyldimethylsilyl)oxy)-4-(methylthio)tetrahydrofuran-2-yl)vinyl)phosphonate as an off-white foamy solid (1.91 g, yield: 98.9%). LCMS (ESI): m/z=597[M+H]⁺.

Step 1h: Preparation of dimethyl((E)-2-((2R,3R,4R, 5R)-5-(3-benzoyl-2,4-dioxo-3,4-dihydropyrimidin-1(2H)-yl)-3-hydroxy-4-(methylthio)tetrahydrofu ran-2-yl)vinyl)phosphonate (Compound **1-9**): Triethylamine trihydrofluoride (2.67 mL, 15.9 mmol) was added to a mixture of dimethyl((E)-2-((2R,3R,4R,5R)-5-(3-benzoyl-2,4-dioxo-3,4-dihydropyrimidin-1(2H)-yl)-3-((tert-bu tyldimethylsilyl)oxy)-4-(methylthio)tetrahydrofuran-2-yl)vinyl)phosphonate (Compound **1-8**) (1.9 g, 3.19 mmol) and triethylamine (1.2 mL, 4.97 mmol) in tetrahydrofuran (40 mL). The mixture was stirred overnight at room temperature. The reaction mixture was concentrated under reduced pressure. The residue was purified by silica gel column chromatography (eluent: petroleum ether/dichloromethane/methanol = 50:50:1 to dichloromethane/methanol = 50:1) to give dimethyl((E)-2-((2R,3R,4R, 5R)-5-(3-benzoyl-2,4-dioxo-3,4-dihydropyrimidin-1(2H)-yl)-3-hydroxy-4-(methylthio)tetrahydrofu ran-2-yl)vinyl)phosphonate as an off-white foamy solid (1.2 g, yield: 76.1%). LCMS(ESI): m/z=[M+1]⁺. ¹H NMR (500 MHz, CDCl₃) δ 8.01 - 7.90 (m, 2H), 7.76 (d, *J* = 8.2 Hz, 1H), 7.67 (t, *J* = 7.4 Hz, 1H), 7.52 (t, *J* = 7.8 Hz, 2H), 6.83 (ddd, *J* = 22.1, 17.2, 4.7 Hz, 1H), 6.10 - 5.99 (m, 1H), 5.87 (d, *J* = 8.3 Hz, 1H), 5.72 (d, *J* = 2.1 Hz, 1H), 5.14 (dd, *J* = 5.2, 2.9 Hz, 1H), 4.38 (d, *J* = 2.1 Hz, 1H), 3.93 - 3.56 (m, 7H), 3.32 (dd, *J* = 5.4, 2.1 Hz, 1H), 2.16 (s, 3H).

### Step 1i: Preparation of (2R,3R,4R,5R)-5-(3-benzoyl-2,4-dioxo-3,4-dihydropyrimidin-1(2H)-yl)-2-((E)-2-(dimethoxyphosp horyl)vinyl)-4-(methylthio)tetrahydrofuran-3-yl(2-cyanoethyl)diisopropylphosphoramidite

(Compound **1**): Cyanoethyl-N,N-diisopropylchlorophosphoramidite (600 µL, 2.70 mmol) was added to a mixture of dimethyl((E)-2-((2R,3R,4R, 5R)-5-(3-benzoyl-2,4-dioxo-3,4-dihydropyrimidin-1(2H)-yl)-3-hydroxy-4-(methylthio)tetrahydrofu ran-2-yl)vinyl)phosphonate (Compound **1-9**) (1.25 g, 2.59 mmol) and N,N-diisopropylethylamine (1.34 g, 10.4 mmol) in anhydrous tetrahydrofuran (20 mL). The mixture was stirred at room temperature for 1 h. The mixture was concentrated in vacuo at 30°C. The residue was purified by silica gel column chromatography (eluent: petroleum ether/acetone = 3:1 + 2% triethylamine to petroleum ether/dichloromethane/acetone = 3:1:1 + 2% triethylamine) to give (2R,3R,4R,5R)-5-(3-benzoyl-2,4-dioxo-3,4-dihydropyrimidin-1(2H)-yl)-2-((E)-2-(dimethoxyphosp horyl)vinyl)-4-(methylthio)tetrahydrofuran-3-yl(2-cyanoethyl)diisopropylphosphoramidite as a white foamy solid (1.60 g, yield: 90%). LCMS(ESI): m/z=600[M-83+1]⁺ and m/z =547[M-135+1]⁺. ¹H NMR (500 MHz, CDCl₃) δ 7.94 (dd, *J* = 11.0, 7.9 Hz, 2H), 7.84 (d, *J* = 8.3 Hz, 1H), 7.66 (q, *J* = 7.1 Hz, 1H), 7.51 (q, *J* = 7.4 Hz, 2H), 6.87 (dddd, *J* = 21.9, 17.1, 9.3, 4.2 Hz, 1H), 6.15 - 5.99 (m, 2H), 5.88 (t, *J* = 8.8 Hz, 1H), 5.11 (dd, *J* = 17.9, 4.4 Hz, 1H), 4.54 (dd, *J* = 16.4, 9.6 Hz, 1H), 3.94 - 3.81 (m, 1H), 3.79 (d, *J* = 2.0 Hz, 6H), 3.75 - 3.69 (m, 1H), 3.60 (ddt, *J* = 23.5, 10.4, 6.7 Hz, 2H), 3.41 (dd, *J* = 56.0, 5.1 Hz, 1H), 2.55 (dt, *J* = 27.8, 5.7 Hz, 2H), 2.21 (d, *J* = 14.2 Hz, 3H), 1.23 - 1.11 (m, 12H).

### Embodiment 2: Preparation of (2R,3S,4R,5R)-5-(3-benzoyl-2,4-dioxo-3,4-dihydropyrimidin-1(2H)-yl)-2-((E)-2-(dimethoxypho sphoryl)vinyl)-4-propyltetrahydrofuran-3-yl(2-cyanoethyl)diisopropylphosphoramidite (Compound 2)

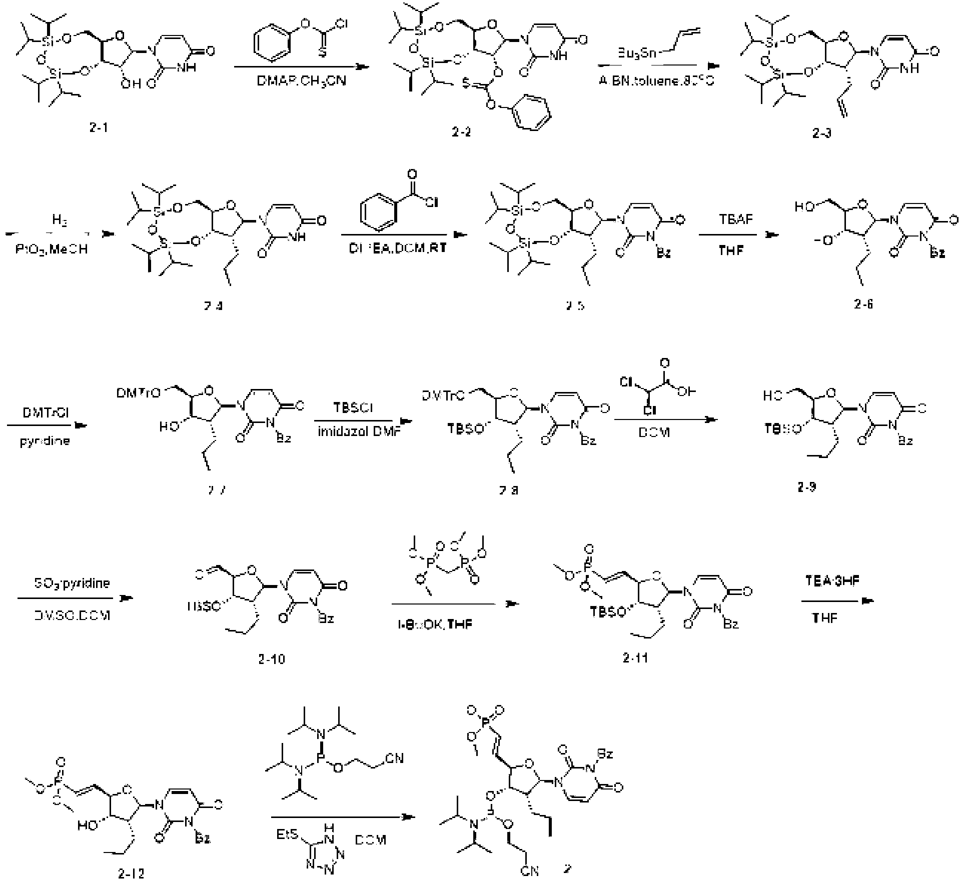

Step 2a: Preparation of *O*-((6aR,8R,9R,9aR)-8-(2,4-dioxo-3,4-dihydropyrimidin-1(2H)-yl)-2,2,4,4-tetraisopropyltetrahydro -6H-furo*[3,2-f*][1,3,5,2,4]trioxadisilocin-9-yl)O-phenyl thiocarbonate (Compound 2-2): Under a nitrogen atmosphere, at 10°C, phenyl chlorothionocarbonate (23.94 g, 138.70 mmol, 1.35 eq) was added dropwise to a mixture of 1-((6a*R*,8*R*,9*R*,9a*S*)-9-hydroxy-2,2,4,4-tetraisopropyltetrahydro-6H-furo[*3,2-f*][1,3,5,2,4]trioxadisil ocin-8-yl)pyrimidine-2,4(1H,3H)-dione (Compound 2-1) (50.0 g, 102.74 mmol, 1.0 eq) and 4-dimethylaminopyridine (25.1 g, 205.48 mmol, 2.0 eq) in dry acetonitrile (600 mL). The mixture was stirred at room temperature overnight. The mixture was filtered through celite. The filter cake was washed three times with ethyl acetate, and the filtrate was then washed with water and saturated brine. The organic phase was dried and concentrated under reduced pressure. The residue was purified by silica gel column chromatography (eluent: petroleum ether/ethyl acetate = 8/1 to 5/1) to give *O*-((6aR,8R,9R,9aR)-8-(2,4-dioxo-3,4-dihydropyrimidin-1(2*H*)-yl)-2,2,4,4-tetraisopropyltetrahydro -6H-furo[3,2-*f*][1,3,5,2,4]trioxadisilocin-9-yl)*O*-phenyl thiocarbonate as a white solid (59.67 g, yield: 93.2%). LCMS (ESI): m/z = 623[M+1]⁺. Step 2b: Preparation of 1-((6a*R*,8*R*,9*R*,9a*S*)-9-allyl-2,2,4,4-tetraisopropyltetrahydro-6H-furo[*3,2-f*][1,3,5,2,4]trioxadisilocin

-8-yl)pyrimidine-2,4(1H,3H)-dione (Compound 2-3): Under a nitrogen atmosphere, at room temperature, azobisisobutyronitrile mixture (3.95 g, 24.09 mmol, 0.5 eq, in 50 mL toluene) was added to a mixture of *O*-((6a*R*,8*R*,9*R*,9a*R*)-8-(2,4-dioxo-3,4-dihydropyrimidin-1(2*H*)-yl)-2,2,4,4-tetraisopropyltetrahydro-6H-furo[3,2-*f*][1,3,5,2,4]trioxadisilocin-9-yl)*O*-phenyl thiocarbonate (Compound 2-2) (30.0 g, 48.17 mmol, 1.0 eq) and allyltributylstannane (79.8 g, 240.85 mmol, 5.0 eq) in dry toluene (450 mL). The mixture was stirred at 80°C for 5.0 h. The solvent was removed under reduced pressure. The residue was dissolved in ethyl acetate and washed with water and saturated brine. The organic phase was dried and concentrated under reduced pressure. The residue was purified by silica gel column chromatography (eluent: petroleum ether/ethyl acetate = 4/1 to 1/1) to give 1-((6a*R*,8*R*,9*R*,9a*S*)-9-allyl-2,2,4,4-tetraisopropyltetrahydro-6*H*-furo[3,2-*f*][1,3,5,2,4]trioxadisilocin

-8-yl)pyrimidine-2,4(1*H*,3*H*)-dione as a white solid (17.4 g, yield: 70.4%). LCMS (ESI): m/z = 511[M+1]⁺

### Step 2c: Preparation of 1-((6aR,8R,9R,9aS)-2,2,4,4-tetraisopropyl-9-propyltetrahydro-6H-furo[3,2-f][1,3,5,2,4]trioxadisiloc in-8-yl)pyrimidine-2,4(1H,3H)-dione (Compound 2-4): At room temperature, platinum dioxide (357 mg, 1.57 mmol, 0.2 eq) was added to a solution of 1-((6aR,8R,9R,9aS)-9-allyl-2,2,4,4-tetraisopropyltetrahydro-6H-furo[3,2-f][1,3,5,2,4]trioxadisilocin

-8-yl)pyrimidine-2,4(1*H*,3H)-dione (Compound 2-3) (4.0 g, 7.84 mmol, 1.0 eq) in methanol (50 mL). The mixture was stirred under hydrogen at room temperature overnight. The mixture was filtered through celite. The filtrate was concentrated under reduced pressure to give 1-((6a*R*,8*R*,9*R*,9a*S*)-2,2,4,4-tetraisopropyl-9-propyltetrahydro-6*H*-furo[*3,2-f*][1,3,5,2,4]trioxadisiloc in-8-yl)pyrimidine-2,4(1H,3H)-dione as a white solid (4.1 g, crude). LCMS (ESI): m/z = 513[M+1]⁺ Step 2d: Preparation of 3-benzoyl-1-((6a*R*,8*R*,9*R*,9a*S*)-2,2,4,4-tetraisopropyl-9-propyltetrahydro-6*H*-furo[3,2-*f*][1,3,5,2,4]tri oxadisilocin-8-yl)pyrimidine-2,4(1*H*,3*H*)-dione (Compound 2-5): At room temperature, benzoyl chloride (1.69 g, 12.0mmol, 1.5 eq) was added to a mixture of 1-((6a*R*,8*R*,9*R*,9a*S*)-2,2,4,4-tetraisopropyl-9-propyltetrahydro-6*H*-furo[3,2-*f*][1,3,5,2,4]trioxadisiloc in-8-yl)pyrimidine-2,4(1*H*,3*H*)-dione (Compound 2-4) (4.1 g, 8.0 mmol, 1.0 eq) and diisopropylethylamine (2.27 g, 17.6 mmol, 2.2 eq) in dichloromethane (50 mL). The mixture was stirred at room temperature overnight. The mixture was diluted with ethyl acetate and washed with water and saturated brine. The organic phase was dried and concentrated under reduced pressure to give

3-benzoyl-1-((6a*R*,8*R*,9*R*,9a*S*)-2,2,4,4-tetraisopropyl-9-propyltetrahydro-6*H*-furo[3,2-*f*][1,3,5,2,4]tri oxadisilocin-8-yl)pyrimidine-2,4(1*H*,3*H*)-dione as a yellow oil (5.3 g, crude). LCMS (ESI): m/z = 617[M+1]⁺.

Step 2e: Preparation of 3-benzoyl-1-((2*R*,3*R*,4*S*,5*R*)-4-hydroxy-5-(hydroxymethyl)-3-propyltetrahydrofuran-2-yl)pyrimidin e-2,4(1H,3H)-dione (Compound 2-6): At room temperature, tetrabutylammonium fluoride (2.27 g, 7.2 mmol, 0.9 eq) was added to a solution of 3-benzoyl-1-((6a*R*,8*R*,9*R*,9a*S*)-2,2,4,4-tetraisopropyl-9-propyltetrahydro-6*H*-furo[3,2-*f*][1,3,5,2,4]tri oxadisilocin-8-yl)pyrimidine-2,4(1*H*,3*H*)-dione (Compound 2-5) (5.3 g, 8.0 mmol, 1.0 eq) in tetrahydrofuran (60 mL). The mixture was stirred at room temperature for 2 h. The mixture was diluted with ethyl acetate and washed with water and saturated brine. The organic phase was dried and concentrated under reduced pressure. The residue was purified by silica gel column chromatography (eluent: petroleum ether/ethyl acetate = 1/3 to 1/15) to obtain 3-benzoyl-1-((2*R*,3*R*,4*S*,5*R*)-4-hydroxy-5-(hydroxymethyl)-3-propyltetrahydrofuran-2-yl)pyrimidin e-2,4(1*H*,3H)-dione as a white solid (2.5 g, yield: 83.6%). LCMS (ESI): m/z = 375[M+1]⁺.

Step 2f: Preparation of 3-benzoyl-1-((2R,3R,4S,5R)-5-((bis(4-methoxyphenyl)(phenyl)methoxy)methyl)-4-hydroxy-3-prop yltetrahydrofuran-2-yl)pyrimidine-2,4(1H,3H)-dione (Compound 2-7): 4,4'-dimethoxytrityl chloride (2.73 g, 8.03 mmol, 1.2 eq) was added to a solution of 3-benzoyl-1-((2*R*,3*R*,4*S*,5*R*)-4-hydroxy-5-(hydroxymethyl)-3-propyltetrahydrofuran-2-yl)pyrimidin e-2,4(1*H*,3H)-dione (Compound 2-6) (2.5 g, 6.69 mmol, 1.0 eq) in pyridine (30 mL). The mixture was stirred at room temperature for 3 h. The mixture was concentrated under reduced pressure to remove pyridine. The residue was dissolved in ethyl acetate and washed with water and saturated brine. The organic phase was dried and concentrated under reduced pressure to give 3-benzoyl-1-((2*R*,3*R*,4*S*,5*R*)-5-((bis(4-methoxyphenyl)(phenyl)methoxy)methyl)-4-hydroxy-3-prop yltetrahydrofuran-2-yl)pyrimidine-2,4(1*H*,3H)-dione as a yellow solid (4.25 g, yield: 93.8%). LCMS (ESI): m/z = 677[M+1]⁺.

Step 2g: Preparation of 3-benzoyl-1-((2*R*,3*R*,4*S*,5*R*)-5-(bis(4-methoxyphenyl)(phenyl)methoxy)methyl)-4-((tert-butyldimet hylsilyl)oxy)-3-propyltetrahydrofuran-2-yl)pyrimidine-2,4(1*H*,3*H*)-dione (Compound 2-8): Under a nitrogen atmosphere, tert-butyldimethylsilyl chloride (2.21 g, 14.65 mmol, 2.5 eq) was added to a mixture of 3-benzoyl-1-((2*R*,3*R*,4*S*,5*R*)-5-((bis(4-methoxyphenyl)(phenyl)methoxy)methyl)-4-hydroxy-3-prop yltetrahydrofuran-2-yl)pyrimidine-2,4(1*H*,3*H*)-dione (Compound 2-7) (3.96 g, 5.86 mmol, 1.0 eq) and imidazole (2.0 g, 29.3 mmol, 5.0 eq) in dry dimethylformamide (13 mL). The mixture was stirred at 36°C overnight. The mixture was diluted with ethyl acetate and washed with water and saturated brine. The organic phase was dried and concentrated under reduced pressure. The residue was purified by silica gel column chromatography (eluent: petroleum ether/ethyl acetate = 8/1 to 4/1 + 2% triethylamine) to give 3-benzoyl-1-((2*R*,3*R*,4*S*,5*R*)-5-(bis(4-methoxyphenyl)(phenyl)methoxy)methyl)-4-((tert-butyldimet hylsilyl)oxy)-3-propyltetrahydrofuran-2-yl)pyrimidine-2,4(1*H*,3*H*)-dione as a yellow solid (3.31 g, yield: 68.8%). LCMS (ESI): m/z = 791[M+1]⁺.

Step 2h: Preparation of 3-benzoyl-1-((2*R*,3*R*,4*S*,5*R*)-4-((tert-butyldimethylsilyl)oxy)-5-(hydroxymethyl)-3-propyltetrahydro furan-2-yl)pyrimidine-2,4(1*H*,3*H*)-dione (Compound 2-9): Dichloroacetic acid (5 mL, 63.7 mmol, 15.0 eq) was added to a solution of 3-benzoyl-1-((2*R*,3*R*,4*S*,5*R*)-5-(bis(4-methoxyphenyl)(phenyl)methoxy)methyl)-4-((tert-butyldimet hylsilyl)oxy)-3-propyltetrahydrofuran-2-yl)pyrimidine-2,4(1*H*,3*H*)-dione (Compound 2-8) (3.31 g, 4.18 mmol, 1.0 eq) in dichloromethane (50 mL). The mixture was stirred at room temperature for 2 h. A saturated aqueous sodium carbonate solution was added for neutralization. The layers were separated. The organic phase was dried and concentrated under reduced pressure. The residue was purified by silica gel column chromatography (eluent: petroleum ether/ethyl acetate = 1/1 to 1/5) to give

3-benzoyl-1-((2*R*,3*R*,4*S*,5*R*)-4-((tert-butyldimethylsilyl)oxy)-5-(hydroxymethyl)-3-propyltetrahydro furan-2-yl)pyrimidine-2,4(1*H*,3*H*)-dione as a white solid (1.59 g, yield: 77.9%). LCMS (ESI): m/z = 489[M+1]⁺.

Step 2i: Preparation of (2*S*,3*S*,4*R*,5*R*)-5-(3-benzoyl-2,4-dioxo-3,4-dihydropyrimidin-1(2*H*)-yl)-3-((tert-butyldimethylsilyl)o xy)-4-propyltetrahydrofuran-2-carbaldehyde (Compound 2-10): Under a nitrogen atmosphere, pyridine sulfur trioxide complex (3.29 g, 21.0 mmol, 8.0 eq) was added to dry dimethyl sulfoxide (12 mL, 169.0 mmol, 64.0 eq). The mixture was stirred at room temperature for 1 h. Under a nitrogen atmosphere, at 10°C, this mixture was added to a mixture of 3-benzoyl-1-((2*R*,3*R*,4*S*,5*R*)-4-((tert-butyldimethylsilyl)oxy)-5-(hydroxymethyl)-3-propyltetrahydro furan-2-yl)pyrimidine-2,4(1*H*,3*H*)-dione (Compound 2-9) (1.26 g, 2.58 mmol, 1.0 eq) and diisopropylethylamine (3.8 mL, 21.9 mmol, 8.5 eq) in dichloromethane. The mixture was stirred at 10°C for 1 h. Ethyl acetate was added and the mixture was washed with water and saturated brine. The organic phase was dried and concentrated under reduced pressure to give (2*S*,3*S*,4*R*,5*R*)-5-(3-benzoyl-2,4-dioxo-3,4-dihydropyrimidin-1(2*H*)-yl)-3-((tert-butyldimethylsilyl)o xy)-4-propyltetrahydrofuran-2-carbaldehyde as a yellow oil (1.33 g, crude). LCMS (ESI): m/z = 487[M+1]⁺.

Step 2j: Preparation of dimethyl(*E*)-2-((2*R*,3*S*,4*R*,5*R*)-5-(3-benzoyl-2,4-dioxo-3,4-dihydropyrimidin-1(2*H*)-yl)-3-((tert-but yldimethylsilyl)oxy)-4-propyltetrahydrofuran-2-yl)vinyl)phosphonate (Compound 2-11): Under a nitrogen atmosphere, at room temperature, potassium tert-butoxide (578 mg, 5.16 mmol, 2.0 eq) was added to a solution of tetramethyl methylenediphosphonate (1.20 g, 5.16 mmol, 2.0 eq) in dry tetrahydrofuran (20 mL) and the mixture was stirred for 1 h. (2*S*,3*S*,4*R*,5*R*)-5-(3-benzoyl-2,4-dioxo-3,4-dihydropyrimidin-1(2*H*)-yl)-3-((tert-butyldimethylsilyl)o xy)-4-propyltetrahydrofuran-2-carbaldehyde (Compound 2-10) (1.33 g, 2.58 mmol, 1.0 eq) was dissolved in dry tetrahydrofuran (5 mL) and added to the above mixture. The mixture was stirred at room temperature for 1 h. The reaction was quenched with an aqueous ammonium chloride solution and extracted with ethyl acetate. The organic phase was dried and concentrated under reduced pressure. The residue was purified by silica gel column chromatography (eluent: dichloromethane/methanol = 50/1 to 20/1) to give dimethyl(*E*)-2-((2*R*,3*S*,4*R*,5*R*)-5-(3-benzoyl-2,4-dioxo-3,4-dihydropyrimidin-1(2*H*)-yl)-3-((tert-but yldimethylsilyl)oxy)-4-propyltetrahydrofuran-2-yl)vinyl)phosphonate as a white solid (1.08 g, yield: 70.5%). LCMS (ESI): m/z = 593[M+1]⁺.

Step 2k: Preparation of dimethyl((*E*)-2-((2*R*,3*S*,4*R*,5*R*)-5-(3-benzoyl-2,4-dioxo-3,4-dihydropyrimidin-1(2*H*)-yl)-3-hydroxy-4-propyltetrahydrofuran-2-yl)vinyl)phosphonate (Compound 2-12): Triethylamine (850 mg, 8.42 mmol, 4.0 eq) and triethylamine trihydrofluoride (2.38 g, 14.7 mmol, 7.0 eq) were added to a solution of

dimethyl(*E*)-2-((2*R*,3*S*,4*R*,5*R*)-5-(3-benzoyl-2,4-dioxo-3,4-dihydropyrimidin-1(2*H*)-yl)-3-((tert-but yldimethylsilyl)oxy)-4-propyltetrahydrofuran-2-yl)vinyl)phosphonate (Compound 2-11) (1.24 g, 2.10 mmol, 1.0 eq) in tetrahydrofuran (12.5 mL). The mixture was stirred at room temperature overnight. A saturated sodium carbonate solution was added for neutralization and ethyl acetate was added for extraction. The organic phase was washed with water and saturated brine, dried and concentrated under reduced pressure. The residue was purified by silica gel column chromatography (eluent: dichloromethane/methanol = 50/1 to 15/1) to give dimethyl((*E*)-2-((2*R*,3*S*,4*R*,5*R*)-5-(3-benzoyl-2,4-dioxo-3,4-dihydropyrimidin-1(2*H*)-yl)-3-hydroxy-4-propyltetrahydrofuran-2-yl)vinyl)phosphonate as a white solid (772 mg, yield: 80.6%). LCMS (ESI): m/z = 479[M+1]⁺.

Step 2l: Preparation of (2*R*,3*S*,4*R*,5*R*)-5-(3-benzoyl-2,4-dioxo-3,4-dihydropyrimidin-1(2*H*)-yl)-2-((E)-2-(dimethoxyphosph oryl)vinyl)-4-propyltetrahydrofuran-3-yl(2-cyanoethyl)diisopropylphosphoramidite (Compound 2): Under a nitrogen atmosphere, 5-(ethylthio)-1*H*-tetrazole (202 mg, 1.55 mmol, 1.5 eq) was added to a mixture of dimethyl((*E*)-2-((2*R*,3*S*,4*R*,5*R*)-5-(3-benzoyl-2,4-dioxo-3,4-dihydropyrimidin-1(2*H*)-yl)-3-hydroxy-4-propyltetrahydrofuran-2-yl)vinyl)phosphonate (Compound 2-12) (494 mg, 1.03 mmol, 1.0 eq) and 2-cyanoethyl-*N,N,N*',N'-tetraisopropylphosphorodiamidite (778 mg, 2.58 mmol, 2.5 eq) in dichloromethane (10 mL). The mixture was stirred at room temperature for 2 h. The mixture was diluted with ethyl acetate and washed with aqueous sodium bicarbonate solution and saturated brine. The organic phase was dried and concentrated under reduced pressure. The residue was purified by silica gel column chromatography (eluent: petroleum ether/dichloromethane/acetone = 3/1/1 + 2% triethylamine) to give (2*R*,3*S*,4*R*,5*R*)-5-(3-benzoyl-2,4-dioxo-3,4-dihydropyrimidin-1(2*H*)-yl)-2-((*E*)-2-(dimethoxyphosph oryl)vinyl)-4-propyltetrahydrofuran-3-yl(2-cyanoethyl)diisopropylphosphoramidite as a white solid (532 mg, yield: 75.4%) LCMS(ESI): m/z = 596 [M-100+18]⁺. ¹H NMR (500 MHz, CDCl₃) δ 7.93 (d, J = 7.6 Hz, 2H), 7.65 (t, J = 7.4 Hz, 1H), 7.50 (t, *J =* 7.8 Hz, 2H), 7.38 (*dd*, *J =* 10.8, 8.4 Hz, 1H), 7.00-6.81 (m, 1H), 6.05 (dt, *J* = 34.6, 12.7 Hz, 2H), 5.92 (dd, *J =* 8.2, 1.6 Hz, 1H), 4.75 (d, *J =* 24.6 Hz, 1H), 4.43-4.33 (m, 1H), 3.87 (td, *J=* 13.5, 6.4 Hz, 1H), 3.81 - 3.54 (m, 9H), 2.71 - 2.57 (m, 2H), 2.11 (dd, *J =* 25.5, 20.8 Hz, 1H), 1.68 (dd, *J =* 13.4, 7.7 Hz, 2H), 1.38-1.31 (m, 2H), 1.18 (t, *J =* 7.0 Hz, 12H), 0.92 (dd, *J =* 13.0, 6.9 Hz, 3H).

### Embodiment 3: Preparation of (2R,3S,4R,5R)-4-allyl-5-(3-benzoyl-2,4-dioxo-3,4-dihydropyrimidin-1(2H)-yl)-2-((E)-2-(dimeth oxyphosphoryl)vinyl)-tetrahydrofuran-3-yl(2-cyanoethyl)diisopropylphosphoramidite (Compound 3):

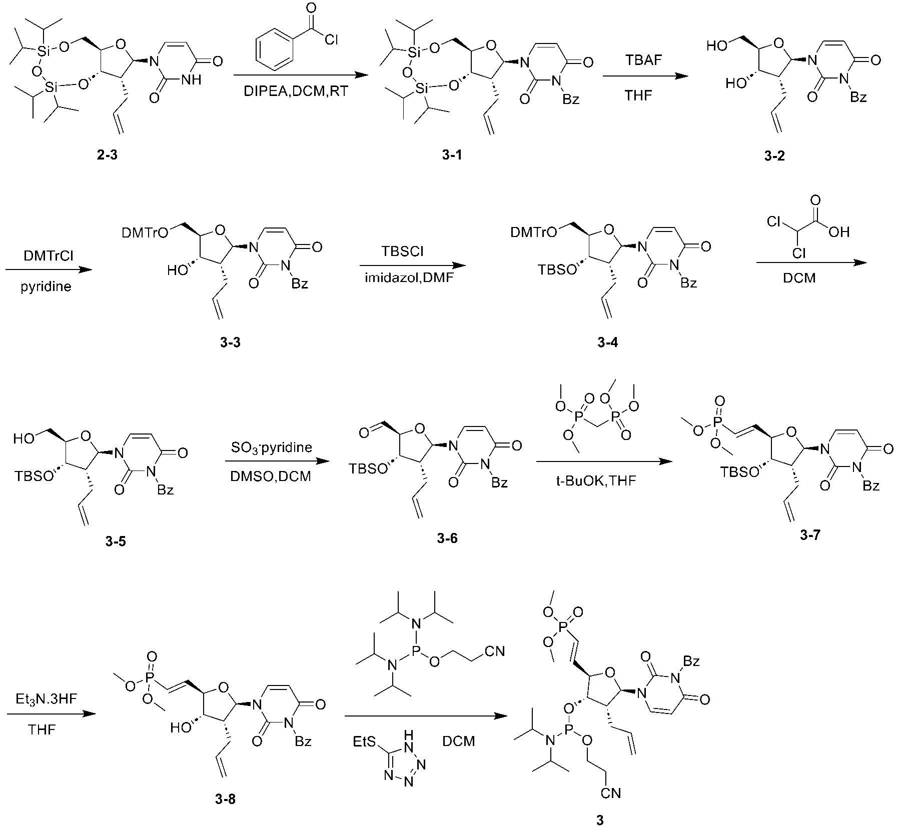

Step 3a: Preparation of 1-((6a*R*,8*R*,9*R*,9a*S*)-9-allyl-2,2,4,4-tetraisopropyltetrahydro-6*H*-furo[3,2-f][1,3,5,2,4]trioxadisilocin -8-yl) 3-benzoylpyrimidine-2,4(1*H*,3*H*)-dione (Compound 3-1): At room temperature, benzoyl chloride (3.02 g, 21.47mmol, 1.5 eq) was added to a mixture of 1-((6a*R*,8*R*,9*R*,9a*S*)-9-allyl-2,2,4,4-tetraisopropyltetrahydro-6H-furo[3,2-*f*][1,3,5,2,4]trioxadisilocin -8-yl)pyrimidine-2,4(1*H*,3*H*)-dione (Compound 2-3) (7.3 g, 14.31 mmol, 1.0 eq) and diisopropylethylamine (4.06 g, 31.48 mmol, 2.2 eq) in dichloromethane (73 mL). The mixture was stirred at room temperature overnight. The mixture was diluted with ethyl acetate and washed with water and saturated brine. The organic phase was dried and concentrated under reduced pressure to give

1-((6a*R*,8*R*,9*R*,9a*S*)-9-allyl-2,2,4,4-tetraisopropyltetrahydro-6H-furo[*3,2-f*][1,3,5,2,4]trioxadisilocin -8-yl) 3-benzoylpyrimidine-2,4(1*H*,3*H*)-dione as a yellow oil (9.94 g, crude). LCMS (ESI): m/z = 615[M+1]⁺.

Step 3b: Preparation of 1-((2*R*,3*R*,4*S*,5*R*)-3-allyl-4-hydroxy-5-(hydroxymethyl)-tetrahydrofuran-2-yl)-3-benzoylpyrimidine -2,4(1H,3H)-dione (Compound 3-2): At room temperature, tetrabutylammonium fluoride (3.61 g, 11.45 mmol, 0.8 eq) was added to a solution of 1-((6a*R*,8*R*,9*R*,9a*S*)-9-allyl-2,2,4,4-tetraisopropyltetrahydro-6*H*-furo[3,2-*f*][1,3,5,2,4]trioxadisilocin -8-yl) 3-benzoylpyrimidine-2,4(1*H*,3*H*)-dione (Compound 3-1) (9.94 g, 14.31 mmol, 1.0 eq) in tetrahydrofuran (90 mL). The mixture was stirred at room temperature for 2 h. The mixture was diluted with ethyl acetate and washed with water and saturated brine. The organic phase was dried and concentrated under reduced pressure. The residue was purified by silica gel column chromatography (eluent: petroleum ether/ethyl acetate = 1/3 to 1/15) to give 1-((2*R*,3*R*,4*S*,5*R*)-3-allyl-4-hydroxy-5-(hydroxymethyl)-tetrahydrofuran-2-yl)-3-benzoylpyrimidine -2,4(1*H*,3*H*)-dione as a white solid (4.42 g, yield: 83.0%). LCMS (ESI): m/z = 373[M+1]⁺.

Step 3c: Preparation of 1-((2*R*,3*R*,4*S*,5*R*)-3-allyl-5-((bis(4-methoxyphenyl)(phenyl)methoxy)methyl)-4-hydroxytetrahydrof uran-2-yl)-3-benzoylpyrimidine-2,4(1H,3H)-dione (Compound 3-3): At room temperature, 4,4'-dimethoxytrityl chloride (4.84 g, 14.27 mmol, 1.2 eq) was added to a solution of 1-((2*R*,3*R*,4*S*,5*R*)-3-allyl-4-hydroxy-5-(hydroxymethyl)-tetrahydrofuran-2-yl)-3-benzoylpyrimidine -2,4(1*H*,3H)-dione (Compound 3-1) (4.42 g, 11.89 mmol, 1.0 eq) in pyridine (50 mL). The mixture was stirred at room temperature for 3 h. The mixture was concentrated under reduced pressure to remove pyridine. The residue was dissolved in ethyl acetate and washed with water and saturated brine. The organic phase was dried and concentrated under reduced pressure to give 1-((2*R*,3*R*,4*S*,5*R*)-3-allyl-5-((bis(4-methoxyphenyl)(phenyl)methoxy)methyl)-4-hydroxytetrahydrof uran-2-yl)-3-benzoylpyrimidine-2,4(1*H*,3*H*)-dione as a yellow solid (7.4 g, yield: 92.2%). LCMS (ESI): m/z = 675[M+1]⁺.

Step 3d: Preparation of 1-((2*R*,3*R*,4*S*,5*R*)-3-allyl-5-(bis(4-methoxyphenyl)(phenyl)methoxy)methyl)-4-((tert-butyldimethyls ilyl)oxy)tetrahydrofuran-2-yl)-3-benzoylpyrimidine-2,4(1H,3H)-dione (Compound 3-4): Under a nitrogen atmosphere, tert-butyldimethylsilyl chloride (4.14 g, 27.45 mmol, 2.5 eq) was added to a mixture of 1-((2*R*,3*R*,4*S*,5*R*)-3-allyl-5-((bis(4-methoxyphenyl)(phenyl)methoxy)methyl)-4-hydroxytetrahydrof uran-2-yl)-3-benzoylpyrimidine-2,4(1*H*,3*H*)-dione (Compound 3-3) (7.4 g, 10.98 mmol, 1.0 eq) and imidazole (3.74 g, 54.9 mmol, 5.0 eq) in dry dimethylformamide (24 mL). The mixture was stirred at 36°C overnight. The mixture was diluted with ethyl acetate and washed with water and saturated brine. The organic phase was dried and concentrated under reduced pressure. The residue was purified by silica gel column chromatography (eluent: petroleum ether/ethyl acetate = 8/1 to 4/1 + 2% triethylamine) to give 1-((2*R*,3*R*,4*S*,5*R*)-3-allyl-5-(bis(4-methoxyphenyl)(phenyl)methoxy)methyl)-4-((tert-butyldimethyls ilyl)oxy)tetrahydrofuran-2-yl)-3-benzoylpyrimidine-2,4(1*H*,3*H*)-dione as a yellow solid (6.70 g, yield: 77.3%). LCMS (ESI): m/z = 789[M+1]⁺.

Step 3e: Preparation of 1-((2*R*,3*R*,4*S*,5*R*)-3-allyl-4-((tert-butyldimethylsilyl)oxy)-5-(hydroxymethyl)tetrahydrofuran-2-yl)-3 -benzoylpyrimidine-2,4(1*H*,3*H*)-dione (Compound 3-5): Dichloroacetic acid (10 mL, 127.5 mmol, 15.0 eq) was added to a solution of 1-((2*R*,3*R*,4*S*,5*R*)-3-allyl-5-(bis(4-methoxyphenyl)(phenyl)methoxy)methyl)-4-((tert-butyldimethyls ilyl)oxy)tetrahydrofuran-2-yl)-3-benzoylpyrimidine-2,4(1*H*,3*H*)-dione (Compound 3-4) (6.7 g, 8.50 mmol, 1.0 eq) in dichloromethane (100 mL). The mixture was stirred at room temperature for 2 h. A saturated aqueous sodium carbonate solution was added for neutralization. The layers were separated. The organic phase was dried and concentrated under reduced pressure. The residue was purified by silica gel column chromatography (eluent: petroleum ether/ethyl acetate = 1/1 to 1/5) to give 1-((2*R*,3*R*,4*S*,5*R*)-3-allyl-4-((tert-butyldimethylsilyl)oxy)-5-(hydroxymethyl)tetrahydrofuran-2-yl)-3 -benzoylpyrimidine-2,4(1*H*,3*H*)-dione as a white solid (3.29 g, yield: 79.4%). LCMS (ESI): m/z = 487[M+1]⁺.

Step 3f: Preparation of (2*S*,3*S*,4*R*,5*R*)-4-allyl-5-(3-benzoyl-2,4-dioxo-3,4-dihydropyrimidin-1(2*H*)-yl)-3-((tert-butyldimeth ylsilyl)oxy)tetrahydrofuran-2-carbaldehyde (Compound 3-6): Under a nitrogen atmosphere, pyridine sulfur trioxide complex (5.90 g, 37.1 mmol, 5.5 eq) was added to dry dimethyl sulfoxide (20 mL, 282 mmol, 42.0 eq). The mixture was stirred at room temperature for 1 h. Under a nitrogen atmosphere, at 10°C, this mixture was added to a mixture of 1-((2*R*,3*R*,4*S*,5*R*)-3-allyl-4-((tert-butyldimethylsilyl)oxy)-5-(hydroxymethyl)tetrahydrofuran-2-yl)-3 -benzoylpyrimidine-2,4(1*H*,3*H*)-dione (Compound 3-5) (3.29 g, 6.67 mmol, 1.0 eq) and diisopropylethylamine (10 mL, 57.46 mmol, 8.5 eq) in dichloromethane. The mixture was stirred at 10°C for 1 h. Ethyl acetate was added and the mixture was washed with water and saturated brine. The organic phase was dried and concentrated under reduced pressure to give (2*S*,3*S*,4*R*,5*R*)-4-allyl-5-(3-benzoyl-2,4-dioxo-3,4-dihydropyrimidin-1(2*H*)-yl)-3-((tert-butyldimeth ylsilyl)oxy)tetrahydrofuran-2-carbaldehyde as a yellow oil (3.56 g, crude). LCMS (ESI): m/z = 485[M+1]⁺.

Step 3g: Preparation of dimethyl(*E*)-2-((2*R*,3*S*,4*R*,5*R*)-4-allyl-5-(3-benzoyl-2,4-dioxo-3,4-dihydropyrimidin-1(2*H*)-yl)-3-((t ert-butyldimethylsilyl)oxy)tetrahydrofuran-2-yl)vinyl)phosphonate (Compound 3-7): Under a nitrogen atmosphere, at room temperature, potassium tert-butoxide (1.50 g, 13.52 mmol, 2.0 eq) was added to a solution of tetramethyl methylenediphosphonate (3.14 g, 13.52 mmol, 2.0 eq) in dry tetrahydrofuran (50 mL) and the mixture was stirred for 1 h.

(2*S*,3*S*,4*R*,5*R*)-4-allyl-5-(3-benzoyl-2,4-dioxo-3,4-dihydropyrimidin-1(2H)-yl)-3-((tert-butyldimeth ylsilyl)oxy)tetrahydrofuran-2-carbaldehyde (Compound 3-6) (3.56 g, 6.76 mmol, 1.0 eq) was dissolved in dry tetrahydrofuran (10 mL) and added to the above mixture. The mixture was stirred at room temperature for 1 h. The reaction was quenched with an aqueous ammonium chloride solution and extracted with ethyl acetate. The organic phase was dried and concentrated under reduced pressure. The residue was purified by silica gel column chromatography (eluent: dichloromethane/methanol = 50/1 to 20/1) to give dimethyl(*E*)-2-((2*R*,3*S*,4*R*,5*R*)-4-allyl-5-(3-benzoyl-2,4-dioxo-3,4-dihydropyrimidin-1(2*H*)-yl)-3-((t ert-butyldimethylsilyl)oxy)tetrahydrofuran-2-yl)vinyl)phosphonate as a white solid (3.06 g, yield: 77.6%). LCMS (ESI): m/z = 591[M+1]⁺.

Step 3h: Preparation of dimethyl((*E*)-2-((2*R*,3*S*,4*R*,5*R*)-4-allyl-5-(3-benzoyl-2,4-dioxo-3,4-dihydropyrimidin-1(2*H*)-yl)-3-h ydroxytetrahydrofuran-2-yl)vinyl)phosphonate (Compound 3-8): Triethylamine (1.92 g, 8.42 mmol, 6.0 eq) and triethylamine trihydrofluoride (3.83 g, 23.78 mmol, 7.5 eq) were added to a solution of dimethyl(*E*)-2-((2*R*,3*S*,4*R*,5*R*)-4-allyl-5-(3-benzoyl-2,4-dioxo-3,4-dihydropyrimidin-1(2*H*)-yl)-3-((t ert-butyldimethylsilyl)oxy)tetrahydrofuran-2-yl)vinyl)phosphonate (Compound 3-7) (1.87 g, 3.17 mmol, 1.0 eq) in tetrahydrofuran (13 mL). The mixture was stirred at room temperature overnight. A saturated sodium carbonate solution was added for neutralization and ethyl acetate was added for extraction. The organic phase was washed with water and saturated brine, dried and concentrated under reduced pressure. The residue was purified by silica gel column chromatography (eluent: dichloromethane/methanol = 50/1 to 15/1) to give dimethyl((*E*)-2-((2*R*,3*S*,4*R*,5*R*)-4-allyl-5-(3-benzoyl-2,4-dioxo-3,4-dihydropyrimidin-1(2*H*)-yl)-3-h ydroxytetrahydrofuran-2-yl)vinyl)phosphonate as a white solid (1.36 g, yield: 83.4%). LCMS (ESI): m/z = 477[M+1]⁺.

Step 3i: Preparation of (2*R*,3*S*,4*R*,5*R*)-4-allyl-5-(3-benzoyl-2,4-dioxo-3,4-dihydropyrimidin-1(2*H*)-yl)-2-((*E*)-2-(dimethoxy phosphoryl)vinyl)-tetrahydrofuran-3-yl(2-cyanoethyl)diisopropylphosphoramidite (Compound 3): Under a nitrogen atmosphere, 5-(ethylthio)-1*H*-tetrazole (283 mg, 2.18 mmol, 1.5 eq) was added to a mixture of dimethyl((E)-2-((2R,3S,4R,5R)-4-allyl-5-(3-benzoyl-2,4-dioxo-3,4-dihydropyrimidin-1(2H)-yl)-3-h ydroxytetrahydrofuran-2-yl)vinyl)phosphonate (Compound 3-8) (691 mg, 1.45 mmol, 1.0 eq) and 2-cyanoethyl-*N,N,N*',N'-tetraisopropylphosphorodiamidite (1.09 g, 3.63 mmol, 2.5 eq) in dichloromethane (14 mL). The mixture was stirred at room temperature for 2 h. The mixture was diluted with ethyl acetate and washed with aqueous sodium bicarbonate solution and saturated brine. The organic phase was dried and concentrated under reduced pressure. The residue was purified by silica gel column chromatography (eluent: petroleum ether/dichloromethane/acetone = 3/1/1 + 2% triethylamine) to give (2*R*,3*S*,4*R*,5*R*)-4-allyl-5-(3-benzoyl-2,4-dioxo-3,4-dihydropyrimidin-1(2*H*)-yl)-2-((*E*)-2-(dimethoxy phosphoryl)vinyl)-tetrahydrofuran-3-yl(2-cyanoethyl)diisopropylphosphoramidite as a white solid (632 mg, yield: 64.4%) LCMS(ESI): m/z = 594 [M-100+18]⁺. ¹H NMR (500 MHz, CDCl₃) δ 7.86 (d, J = 8.0 Hz, 2H), 7.58 (t, J = 7.4 Hz, 1H), 7.43 (t*, J* = 7.8 Hz, 2H), 7.29 (*dd, J* = 13.9, 8.3 Hz, 1H), 6.95-6.72 (m, 1H), 5.99 (dt*, J* = 34.0, 12.5 Hz, 2H), 5.84 (dd*, J* = 8.1, 5.1 Hz, 1H), 5.74-5.57 (m, 1H), 5.10-4.98 (m, 2H), 4.66 (d*, J* = 40.9 Hz, 1H), 4.40-4.28 (m, 1H), 3.86-3.76 (m, 1H), 3.76-3.46 (m, 9H), 2.67-2.52 (m, 2H), 2.50-2.31 (m, 1H), 2.18 (*ddt, J* = 21.4, 14.8, 8.1 Hz, 2H), 1.12 (t*, J* = 7.0 Hz, 12H).

### Embodiment 4: Preparation of (2R,3S,4R,5R)-5-(3-benzoyl-2,4-dioxo-3,4-dihydropyrimidin-1(2H)-yl)-2-((E)-2-(dimethoxyph osphoryl)vinyl)-4-(methoxymethyl)tetrahydrofuran-3-yl(2-cyanoethyl)diisopropylphosphora midite (Compound 4):

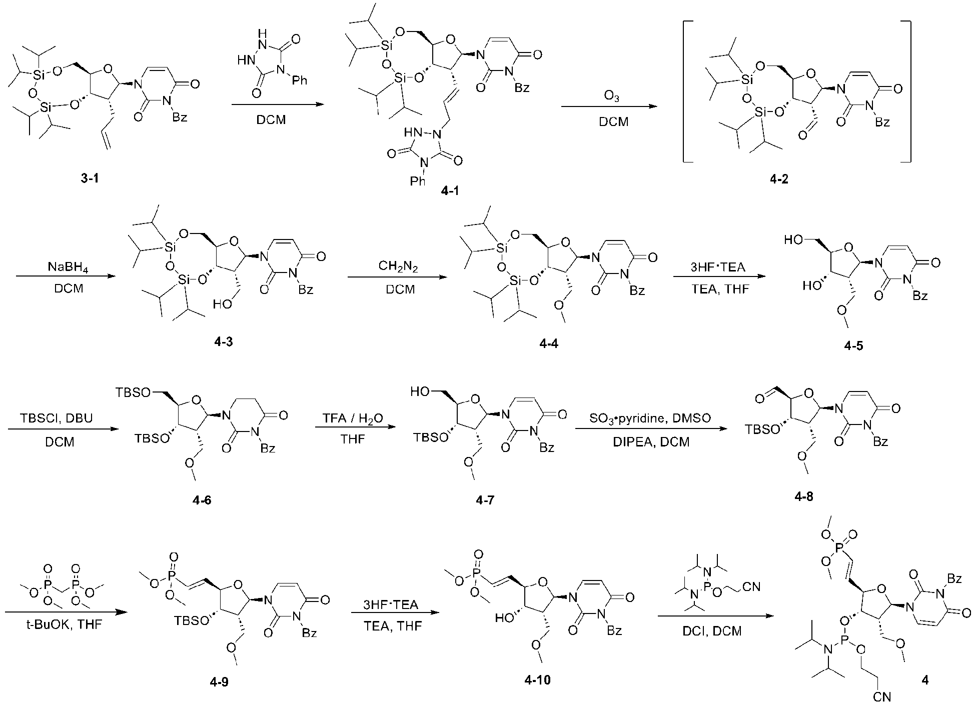

Step 4a: Preparation of 3-benzoyl-1-((6aR,8R,9R,9aS)-9-((E)-3-(3,5-dioxo-4-phenyl-1,2,4-triazolidin-1-yl)prop-1-en-1-yl)-2,2,4,4-tetraisopropyltetrahydro-6H-furo[3,2-f][1,3,5,2,4]trioxadisilocin-8-yl)pyrimidine-2,4(1H,3H )-dione (Compound 4-1): Under a nitrogen atmosphere, at room temperature and protected from light, 4-phenyl-1,2,4-triazolidine-3,5-dione (5.98 g, 34.152 mmol, 1.5 eq) was added to a solution of 1-((6aR,8R,9R,9aS)-9-allyl-2,2,4,4-tetraisopropyltetrahydro-6H-furo[3,2-f][1,3,5,2,4]trioxadisilocin -8-yl)-3-benzoylpyrimidine-2,4(1H,3H)-dione (Compound 3-1) (14.0 g, 22.768 mmol, 1.0 eq) in dichloromethane (200 mL). The mixture was stirred at room temperature and protected from light overnight. The mixture was diluted with water and extracted with ethyl acetate. The organic phase was washed with water and saturated brine, and concentrated in vacuo. The residue was purified by silica gel column chromatography (eluent: dichloromethane/methanol = 8/1 to 5/1) to give 3-benzoyl-1-((6aR,8R,9R,9aS)-9-((E)-3-(3,5-dioxo-4-phenyl-1,2,4-triazolidin-1-yl)prop-1-en-1-yl)-2,2,4,4-tetraisopropyltetrahydro-6H-furo[3,2-f][1,3,5,2,4]trioxadisilocin-8-yl)pyrimidine-2,4(1H,3H )-dione as yellow solid (15.57 g, yield: 86.6%). LCMS (ESI): m/z=790[M+1]⁺.

Step 4b: Preparation of 3-benzoyl-1-((6aR,8R,9R,9aS)-9-(hydroxymethyl)-2,2,4,4-tetraisopropyltetrahydro-6H-furo[3,2-f][ 1,3,5,2,4]trioxadisilocin-8-yl)pyrimidine-2,4(1H,3H)-dione (Compound 4-3): A solution of 3-benzoyl-1-((6aR,8R,9R,9aS)-9-((E)-3-(3,5-dioxo-4-phenyl-1,2,4-triazolidin-1-yl)prop-1-en-1-yl)-2,2,4,4-tetraisopropyltetrahydro-6H-furo[3,2-f][1,3,5,2,4]trioxadisilocin-8-yl)pyrimidine-2,4(1H,3H )-dione (Compound 4-1) (19.70 g, 24.937 mmol, 1.0 eq) in dichloromethane (250 mL) was stirred for 20 min at -70°C under an ozone atmosphere to give 6aR,8R benzoyl-2,4-dioxo-3,4-dihydropyrimidin-1(2H)-yl)-2,2,4,4-tetraisopropyltetrahydro-6H-furo[3,2-f][ 1,3,5,2,4]trioxadisilocin-9-carbonyl as an intermediate (Compound 4-2). The reaction mixture was diluted with methanol (250 mL). Sodium borohydride (3.79 g, 99.748 mmol, 4.0 eq) was added portionwise at -70°C. The mixture was stirred at room temperature for 10 min. The reaction was quenched with water and extracted with dichloromethane. The organic phase was washed with water and saturated brine, and concentrated in vacuo. The residue was purified by silica gel column chromatography (eluent: petroleum ether/ethyl acetate = 6/1 to 2/1) to give 3-benzoyl-1-((6aR,8R,9R,9aS)-9-(hydroxymethyl)-2,2,4,4-tetraisopropyltetrahydro-6H-furo[3,2-f][ 1,3,5,2,4]trioxadisilocin-8-yl)pyrimidine-2,4(1H,3H)-dione as a white solid (9.02 g, yield: 59.8%). LCMS (ESI): m/z=605[M+1]⁺.

Step 4c: Preparation of 3-benzoyl-1-((6aR,8R,9R,9aS)-2,2,4,4-tetraisopropyl-9-(methoxymethyl)tetrahydro-6H-furo[3,2-f][ 1,3,5,2,4]trioxadisilocin-8-yl)pyrimidine-2,4(1H,3H)-dione (Compound 4-4): In a 250 mL flask, 36 mL of 40% potassium hydroxide solution and 120 mL of dichloromethane were added. The mixture was cooled to 0°C and 12 g of crushed nitrosomethylurea was added portionwise while maintaining this temperature. After addition, the organic layer was quickly separated. The aqueous layer was extracted once with dichloromethane. The combined organic layer was dried over potassium hydroxide at 0°C for 2 h to obtain a diazomethane solution with a content of about 3.2 g (75.89 mmol, 9.0 eq). At -5°C, the prepared diazomethane solution (67.46 mmol, 8.0 eq) was added portionwise to a solution of 3-benzoyl-1-((6aR,8R,9R,9aS)-9-(hydroxymethyl)-2,2,4,4-tetraisopropyltetrahydro-6H-furo[3,2-f][ 1,3,5,2,4]trioxadisilocin-8-yl)pyrimidine-2,4(1H,3H)-dione (Compound 4-3) (5.10 g, 8.432 mmol, 1.0 eq) and boron trifluoride-diethyl etherate (0.3 mL, 0.843 mmol, 0.1 eq) in dry dichloromethane (30 mL). The mixture was stirred at 0°C for 0.5 h. The reaction mixture was sequentially washed with a 10% sodium bicarbonate solution and saturated brine. The organic phase was dried and concentrated in vacuo. The residue was purified by silica gel column chromatography (eluent: petroleum ether/ethyl acetate = 10/1 to 4/1) to give 3-benzoyl-1-((6aR,8R,9R,9aS)-2,2,4,4-tetraisopropyl-9-(methoxymethyl)tetrahydro-6H-furo[3,2-f][ 1,3,5,2,4]trioxadisilocin-8-yl)pyrimidine-2,4(1H,3H)-dione as a white solid (3.80 g, yield: 72.8%). LCMS (ESI): m/z=619[M+1]⁺.

Step 4d: Preparation of 3-benzoyl-1-((2R,3R,4S,5R)-4-hydroxy-5-(hydroxymethyl)-3-(methoxymethyl)tetrahydrofuran-2-y l)pyrimidine-2,4(1H,3H)-dione (Compound 4-5): Triethylamine (2.48 g, 24.56 mmol, 4.0 eq) and triethylamine trihydrofluoride (6.92 g, 42.973 mmol, 7.0 eq) were added to a solution of 3-benzoyl-1-((6aR,8R,9R,9aS)-2,2,4,4-tetraisopropyl-9-(methoxymethyl)tetrahydro-6H-furo[3,2-f][ 1,3,5,2,4]trioxadisilocin-8-yl)pyrimidine-2,4(1H,3H)-dione (Compound 4-4) (3.80 g, 6.139 mmol, 1.0 eq) in tetrahydrofuran (40 mL). The mixture was stirred at room temperature overnight. A saturated sodium carbonate solution was added for neutralization and ethyl acetate was added for extraction. The organic phase was washed with water and saturated brine, dried and concentrated in vacuo. The residue was purified by silica gel column chromatography (eluent: dichloromethane/methanol = 80/1 to 10/1) to give 3-benzoyl-1-((2R,3R,4S,5R)-4-hydroxy-5-(hydroxymethyl)-3-(methoxymethyl)tetrahydrofuran-2-y l)pyrimidine-2,4(1H,3H)-dione as a white solid (1.62 g, yield: 97%). LCMS (ESI): m/z = 377[M+1]⁺. Step 4e: Preparation of 3-benzoyl-1-((2R,3R,4S,5R)-4-((tert-butyldimethylsilyl)oxy)-5-(((tert-butyldimethylsilyl)oxy)meth yl)-3-(methoxymethyl)tetrahydrofuran-2-yl)pyrimidine-2,4(1H,3H)-dione (Compound 4-6): Under a nitrogen atmosphere, at 0°C, tert-butyldimethylsilyl chloride (1.88 g, 12.471 mmol, 3.0 eq) was added to a mixture of 3-benzoyl-1-((2R,3R,4S,5R)-4-hydroxy-5-(hydroxymethyl)-3-(methoxymethyl)tetrahydrofuran-2-y l)pyrimidine-2,4(1H,3H)-dione (Compound 4-5) (1.7 g, 4.517 mmol, 1.0 eq) and 1,8-diazacyclo[5,4,0]undecene-7 (2.75 g, 18.068 mmol, 4.0 eq) in dichloromethane (16 mL). The mixture was stirred at room temperature for 7 h. The mixture was diluted with dichloromethane and washed with water and saturated brine. The organic phase was concentrated in vacuo. The residue was purified by silica gel column chromatography (eluent: petroleum ether/ethyl acetate = 20/1 to 10/1) to give 3-benzoyl-1-((2R,3R,4S,5R)-4-((tert-butyldimethylsilyl)oxy)-5-((((tert-butyldimethylsilyl)oxy)meth yl)-3-(methoxymethyl)tetrahydrofuran-2-yl)pyrimidine-2,4(1H,3H)-dione as a white solid (1.90 g, yield: 74.5%). LCMS (ESI): m/z=605[M+1]⁺.

Step 4f: Preparation of 3-benzoyl-1-((2R,3R,4S,5R)-4-((tert-butyldimethylsilyl)oxy)-5-(hydroxymethyl)-3-(methoxymethy l)tetrahydrofuran-2-yl)pyrimidine-2,4(1H,3H)-dione (Compound 4-7): Under a nitrogen atmosphere, at 0°C, trifluoroacetic acid/water (5 mL/5 mL) was added to a solution of 3-benzoyl-1-((2R,3R,4S,5R)-4-((tert-butyldimethylsilyl)oxy)-5-(((tert-butyldimethylsilyl)oxymethy l)-3-(methoxymethyl)tetrahydrofuran-2-yl)pyrimidine-2,4(1H,3H)-dione (Compound 4-6) (1.90 g, 3.069 mmol, 1.0 eq) in tetrahydrofuran (20 mL). The mixture was stirred at 0°C for 4 h. The reaction was quenched with a saturated aqueous sodium bicarbonate solution, and extracted with ethyl acetate. The organic phase was washed with saturated brine, and concentrated in vacuo. The residue was purified by silica gel column chromatography (eluent: petroleum ether/ethyl acetate = 3/1 to 1/2) to give

3-benzoyl-1-((2R,3R,4S,5R)-4-(((tert-butyldimethylsilyl)oxy)-5-(hydroxymethyl)-3-(methoxymeth yl)tetrahydrofuran-2-yl)pyrimidine-2,4(1H,3H)-dione as a white solid (1.3 g, yield: 86.6%). LCMS (ESI): m/z=491[M+1]⁺.

Step 4g: Preparation of (2S,3S,4R,5R)-5-(3-benzoyl-2,4-dioxo-3,4-dihydropyrimidin-1(2H)-yl)-3-((tert-butyldimethylsilyl) oxy)-4-(methoxymethyl)tetrahydrofuran-2-carbaldehyde (Compound 4-8): Under a nitrogen atmosphere, pyridine sulfur trioxide (1.17 g, 7.338 mmol, 4.0 eq) was added to dry dimethyl sulfoxide (10 mL). The mixture was stirred at room temperature for 15 min. At 0°C, the mixture was added to a mixture of 3-benzoyl-1-((2R,3R,4S,5R)-4-(((tert-butyldimethylsilyl)oxy)-5-(hydroxymethyl)-3-(methoxymeth

yl)tetrahydrofuran-2-yl)pyrimidine-2,4(1H,3H)-dione (Compound 4-7) (900 mg, 1.834 mmol, 1.0 eq) and N,N-diisopropylethylamine (1.89 g, 14.672 mmol, 8.0 eq) in dry dichloromethane (15 mL). The mixture was stirred at 0°C for 1.0 h. Ethyl acetate was added and the mixture was washed with water and saturated brine. The organic phase was dried and concentrated in vacuo to give (2S,3S,4R,5R)-5-(3-benzoyl-2,4-dioxo-3,4-dihydropyrimidin-1(2H)-yl)-3-(tert-butyldimethylsilyl)o xy)-4-(methoxymethyl)tetrahydrofuran-2-carbaldehyde as a light yellow solid (920 mg, crude). LCMS (ESI): m/z=489[M+1]⁺.

Step 4h: Preparation of dimethyl(E)-2-((2R,3S,4R,5R)-5-(3-benzoyl-2,4-dihydropyrimidin-1(2H)-yl)-3-((tert-butyldimethyl silyl)oxy)-4-(methoxymethyl)tetrahydrofuran-2-yl)vinyl)phosphonate (Compound 4-9): Under a nitrogen atmosphere, at room temperature, potassium tert-butoxide (411 mg, 3.668 mmol, 2.0 eq) was added to a solution of tetramethyl methylenediphosphonate (936 mg, 4.035 mmol, 2.2 eq) in dry tetrahydrofuran (20 mL). The mixture was stirred at room temperature for 15 min. (2S,3S,4R,5R)-5-(3-benzoyl-2,4-dioxo-3,4-dihydropyrimidin-1(2H)-yl)-3-((tert-butyldimethylsilyl) oxy)-4-(methoxymethyl)tetrahydrofuran-2-carbaldehyde (Compound 4-8) (896 mg, 1.834 mmol, 1.0 eq) was added. The mixture was stirred at room temperature for 15 min. The reaction was quenched with an aqueous ammonium chloride solution and extracted with ethyl acetate. The organic phase was dried and concentrated in vacuo. The residue was purified by silica gel column chromatography (eluent: dichloromethane/methanol = 300/1 to 100/1) to give dimethyl((E)-2-((2R,3S,4R,5R)-5-(3-benzoyl-2,4-dioxo-3,4-dihydropyrimidin-1(2H)-yl)-3-(((tert-b utyldimethylsilyl)oxy)-4-(methoxymethyl)tetrahydrofuran-2-yl)vinyl)phosphonate as a white solid (903 mg, yield: 82.84%). LCMS (ESI): m/z = 595[M+1]⁺.

Step 4i: Preparation of dimethyl((E)-2-((2R,3S,4R,5R)-5-(3-benzoyl-2,4-dioxo-3,4-dihydropyrimidin-1(2H)-yl)-3-hydroxy -4-(methoxymethyl)tetrahydrofuran-2-yl)vinyl)phosphonate (Compound 4-10): Under a nitrogen atmosphere, triethylamine (613 mg, 6.072 mmol, 4.0 eq) and triethylamine trihydrofluoride (1.71 g, 10.629 mmol, 7.0 eq) were added to a solution of dimethyl(E)-2-(((2R,3S,4R,5R)-5-(3-benzoyl-2, 4-dihydropyrimidin-1(2H)-yl)-3-((tert-butyldimethylsilyl)oxy)-4-(methoxymethyl)tetrahydrofuran-2-yl)vinyl)phosphonate (Compound 4-9) (903 mg, 1.518 mmol, 1.0 eq) in dry tetrahydrofuran (10 mL). The mixture was stirred at room temperature overnight. The reaction mixture was diluted with water and extracted with ethyl acetate. The organic phase was washed with water and saturated brine, dried and concentrated in vacuo. The residue was purified by silica gel column chromatography (eluent: dichloromethane/methanol = 100/1 to 30/1) to give dimethyl((E)-2-((2R,3S,4R,5R)-5-(3-benzoyl-2,4-dioxo-3,4-dihydropyrimidin-1(2H)-yl)-3-hydroxy -4-(methoxymethyl)tetrahydrofuran-2-yl)vinyl)phosphonate as a white solid (608 mg, yield: 83%). LCMS (ESI): m/z = 481[M+1]⁺.

Step 4j: Preparation of (2R,3S,4R,5R)-5-(3-benzoyl-2,4-dioxo-3,4-dihydropyrimidin-1(2H)-yl)-2-((E)-2-(dimethoxyphosp horyl)vinyl)-4-(methoxymethyl)tetrahydrofuran-3-yl(2-cyanoethyl)diisopropylphosphoramidite (Compound 4): Under a nitrogen atmosphere, tetrazole (133 mg, 1.898 mmol, 1.5 eq) was added to a mixture of dimethyl((E)-2-((2R,3S,4R,5R)-5-(3-benzoyl-2,4-dioxo-3,4-dihydropyrimidin-1(2H)-yl)-3-hydroxy -4-(methoxymethyl)tetrahydrofuran-2-yl)vinyl)phosphonate (Compound 4-10) (608 mg, 1.265 mmol, 1.0 eq) and bis(diisopropylamino)(2-cyanoethoxy)phosphine (1.14 g, 3.796 mmol, 3.0 eq) in dichloromethane (15 mL). The mixture was stirred at room temperature for 2 h, diluted with ethyl acetate, and then washed with an aqueous sodium bicarbonate solution and saturated brine. The organic phase was dried and concentrated in vacuo. The residue was purified by silica gel column chromatography (eluent: dichloromethane/petroleum ether/acetone = 2/5/1 to 2/2/1 + 2% triethylamine) to give (2R,3S,4R,5R)-5-(3-benzoyl-2,4-dioxo-3,4-dihydropyrimidin-1(2H)-yl)-2-((E)-2-(dimethoxyphosp horyl)vinyl)-4-(methoxymethyl)tetrahydrofuran-3-yl(2-cyanoethyl)diisopropylphosphoramidite as a white solid (816 mg, yield: 94%). LCMS (ESI): m/z = 599[M-100+18]⁺. ¹H NMR (500 MHz, CDCl₃) δ 7.87 (d, *J =* 7.4 Hz, 2H), 7.58 (t, *J =* 7.4 Hz, 1H), 7.43 (t, *J =* 7.7 Hz, 2H), 7.33 (t, *J=* 7.7 Hz, 1H), 6.93-6.72 (m, 1H), 6.11 (t, *J=* 7.4 Hz, 1H), 6.03-5.88 (m, 1H), 5.82 (t, *J =* 8.1 Hz, 1H), 4.63 (d, *J=* 44.5 Hz, 1H), 4.42-4.32 (m, 1H), 3.86-3.75 (m, 1H), 3.72-3.60 (m, 7H), 3.60-3.51 (m, 3H), 3.22 (d, *J=* 15.4 Hz, 3H), 2.61-2.36 (m, 4H), 1.12 (t, *J=* 5.1 Hz, 12H).

### Embodiment 5: Preparation of (2R,3S,4R,5R)-5-(3-benzoyl-2,4-dioxo-3,4-dihydropyrimidin-1(2H)-yl)-4-(cyanomethyl)-2-((E) -2-(dimethoxyphosphoryl)vinyl)tetrahydrofuran-3-yl(2-cyanoethyl)diisopropylphosphoramidi te (Compound 5)

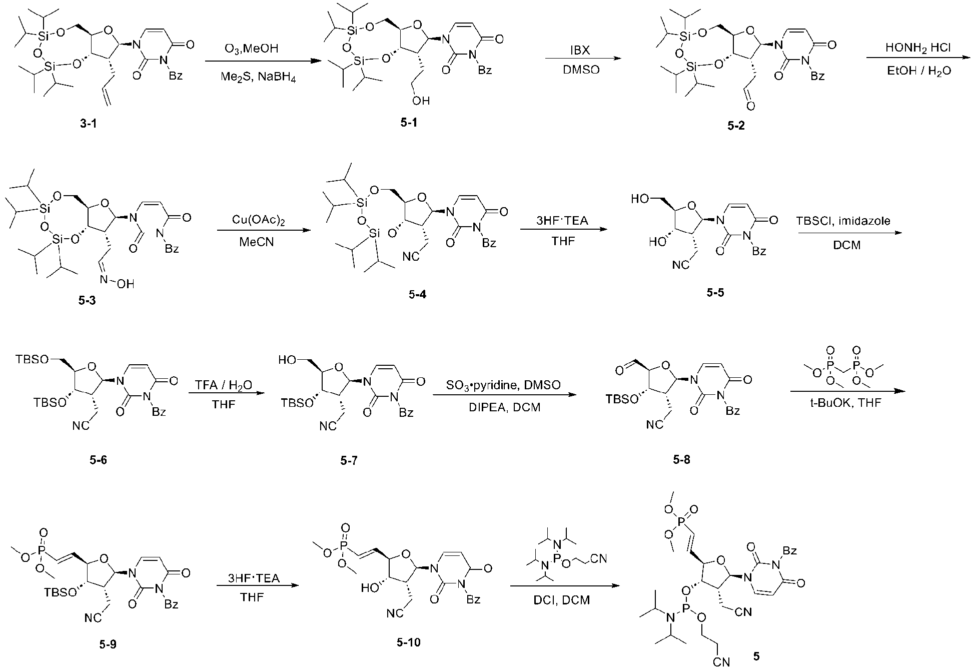

Step 5a: Preparation of 3-benzoyl-1-((6aR,8R,9R,9aS)-9-(2-hydroxyethyl)-2,2,4,4-tetraisopropyltetrahydro-6H-furo[3,2-f][ 1,3,5,2,4]trioxadisilocin-8-yl)pyrimidine-2,4(1H,3H)-dione (Compound 5-1): At -70°C, a solution of 1-((6aR,8R,9R, 9aS)-9-allyl-2,2,4,4-tetraisopropyltetrahydro-6H-furo[3,2-f][1,3,5,2,4]trioxadisilocin-8-yl)-3-benzo ylpyrimidine-2,4(1H,3H)-dione (Compound 3-1) (20.0 g, 32.57 mmol, 1.0 eq) in methanol (280 mL) was purged with ozone for 25 min. Thin-layer chromatography indicated the absence of starting material. Dimethyl sulfide (40 mL) was added dropwise and the mixture was warmed to 10°C. The reaction mixture was stirred for 30 min. Then, the mixture was cooled to -70°C. Sodium borohydride (2.35 g, 61.88 mmol, 1.9 eq) was added portionwise and the mixture was warmed to room temperature. The reaction mixture was stirred for 30 min. An ammonium chloride solution was added and then the mixture was concentrated under reduced pressure to remove methanol. The residue was extracted with dichloromethane washed with water and saturated brine. The organic phase was concentrated under reduced pressure. The residue was purified by silica gel column chromatography (eluent: petroleum ether/ethyl acetate = 8/1 to 2/1) to give 3-benzoyl-1-((6aR,8R,9R,9aS)-9-(2-hydroxyethyl)-2,2,4,4-tetraisopropyltetrahydro-6H-furo[3,2-f][ 1,3,5,2,4]trioxadisilocin-8-yl)pyrimidine-2,4(1H,3H)-dione as a white solid (9.2 g, yield: 45.7%). LCMS (ESI): m/z = 619[M+1]⁺.

Step 5b: Preparation of 2-((6aR,8R,9R,9aS)-8-(3-benzoyl-2,4-dioxo-3,4-dihydropyrimidin-1(2H)-yl)-2,2,4,4-tetraisopropylt etrahydro-6H-furo[3,2-f][1,3,5,2,4]trioxadisilocin-9-yl)acetaldehyde (Compound 5-2): At room temperature, a mixture of 3-benzoyl-1-((6aR,8R,9R,9aS)-9(2-hydroxyethyl)-2,2,4,4-tetraisopropyltetrahydro-6H-furo[3,2-f][1 ,3,5,2,4]trioxadisilocin-8-yl)pyrimidine-2,4(1H,3H)-dione (Compound 5-1) (2.5 g, 4.04 mmol, 1.0 eq) and 2-iodoxybenzoic acid (1.36 g, 4.85 mmol, 1.2 eq) in dry dimethyl sulfoxide (25 mL) was stirred for 1.5 h. The reaction was quenched with a saturated aqueous sodium bisulfite solution, and the pH was adjusted to 7 with a saturated aqueous sodium bicarbonate solution. The mixture was extracted with ethyl acetate. The organic phase was washed with saturated brine, dried over anhydrous sodium sulfate, and concentrated in vacuo to give 2-((6aR,8R,9R,9aS)-8-(3-benzoyl-2,4-dioxo-3,4-dihydropyrimidin-1(2H)-yl)-2,4,4-tetraisopropyltet rahydro-6H-furo[3,2-f][1,3,5,2,4]trioxadisilocin-9-yl)acetaldehyde as a pale yellow solid, which was directly used in the next step. (2.48 g, yield: 99.5%). LCMS (ESI): m/z = 617[M+1]⁺.

Step 5c: Preparation of (Z)-2-((6aR,8R,9R,9aS)-8-(3-benzoyl-2,4-dioxo-3,4-dihydropyrimidin-1(2H)-yl)-2,4,4-tetraisoprop yltetrahydro-6H-furo[3,2-f][1,3,5,2,4]trioxadisilocin-9-yl)acetaldehyde oxime (Compound 5-3): At room temperature, a mixture of 2-(6aR,8R,9R,9aS)-8-(3-benzoyl-2,4-dioxo-3,4-dihydropyrimidin-1(2H)-yl)-2,2,4,4-tetraisopropylt etrahydro-6H-furo[3,2-f][1,3,5,2,4]trioxadisilocin-9-yl)acetaldehyde (Compound 5-2) (2.48 g, 4.019 mmol, 1.0 eq) and hydroxylamine hydrochloride (1.12 g, 16.078 mmol, 4.0 eq) in ethanol/water (25 mL/25 mL) was stirred for 15 min. The reaction mixture was diluted with water and extracted with ethyl acetate. The organic phase was washed with saturated brine, dried over anhydrous sodium sulfate, and concentrated in vacuo to give (Z)-2-((6aR,8R,9R,9aS)-8-(3-benzoyl-2,4-dioxo-3,4-dihydropyrimidin-1(2H)-yl)-2,2,4,4-tetraisopr opyltetrahydro-6H-furo[3,2-f][1,3,5,2,4]trioxadisilocin-9-yl)acetaldehyde oxime as a light yellow solid (2.40 g, yield: 94.5%), which was used directly in the next step. LCMS (ESI): m/z=632[M+1]⁺. Step 5d: Preparation of 2-(6aR,8R,9R,9aS)-8-(3-benzoyl-2,4-dioxo-3,4-dihydropyrimidin-1(2H)-yl)-2,2,4,4-tetraisopropylt etrahydro-6H-furo[3,2-f][1,3,5,2,4]trioxadisilocin-9-yl)acetonitrile (Compound 5-4): Under a nitrogen atmosphere, a mixture of (Z)-2-((6aR,8R,9R,9aS)-8-(3-benzoyl-2,4-dioxo-3,4-dihydropyrimidin-1(2H)-yl)-2,2,4,4-tetraisopr opyltetrahydro-6H-furo[3,2-f][1,3,5,2,4]trioxadisilocin-9-yl)acetaldehyde oxime (Compound 5-3) (2.40 g, 3.798 mmol, 1.0 eq) and copper acetate (1.52 g, 7.596 mmol, 2.0 eq) in acetonitrile (30 mL) was stirred at 60°C for 5 h. The mixture was cooled to room temperature, diluted with water, and extracted with ethyl acetate. The organic phase was washed with saturated brine, and concentrated in vacuo. The residue was purified by silica gel column chromatography (eluent: petroleum ether/ethyl acetate = 10/1 to 5/1) to give 2-((6aR,8R,9R,9aS)-8-(3-benzoyl-2,4-dioxo-3,4-dihydropyrimidin-1(2H)-yl)-2,2,4,4-tetraisopropylt etrahydro-6H-furo[3,2-f][1,3,5,2,4]trioxadisilocin-9-yl)acetonitrile as a light yellow solid (2.0 g, yield: 85.8%). LCMS (ESI): m/z = 614[M+1]⁺.

Step 5e: Preparation of 2-(2R,3R,4S,5R)-2-(3-benzoyl-2,4-dioxo-3,4-dihydro-pyrimidin-1(2H)-yl)-4-hydroxy-5-(hydroxym ethyl)tetrahydrofuran-3-yl)acetonitrile (Compound 5-5): Under a nitrogen atmosphere, at 0°C, triethylamine trihydrofluoride (4.62 g, 28.67 mmol, 10.0 eq) was added dropwise to a solution of 2-(6aR,8R,9R,9aS)-8-(3-benzoyl-2,4-dioxo-3,4-dihydropyrimidin-1(2H)-yl)-2,2,4,4-tetraisopropylt etrahydro-6H-furo[3,2-f][1,3,5,2,4]trioxadisilocin-9-yl)acetonitrile (Compound 5-4) (1.76 g, 2.867 mmol, 1.0 eq) in dry tetrahydrofuran (60 mL). The mixture was stirred at room temperature overnight. The reaction mixture was diluted with water and extracted with ethyl acetate. The organic phase was washed with saturated brine, and concentrated in vacuo. The residue was purified by silica gel column chromatography (eluent: dichloromethane/methanol = 200/1 to 20/1) to give 2-((2R,3R,4S,5R)-2-(3-benzoyl-2,4-dioxo-3,4-dihydropyrimidin-1(2H)-yl)-4-hydroxy-5-(hydroxym ethyl)tetrahydrofuran-3-yl)acetonitrile as a white solid (1.0 g, yield: 84.7%). LCMS (ESI): m/z=372[M+1]⁺.

Step 5f: Preparation of 2-(2R,3R,4S,5R)-2-(3-benzoyl-2,4-dioxo-3,4-dihydro-pyrimidin-1(2H)-yl)-4-((tert-butyldimethylsil yl)oxy)-5-((tert-butyldimethylsilyl)oxymethyl)tetrahydrofuran-3-yl)acetonitrile (Compound 5-6): Under a nitrogen atmosphere, at 0°C, tert-butyldimethylsilyl chloride (2.44 g, 16.157mmol, 6.0 eq) was added to a solution of 2-((2R,3R,4S,5R)-2-(3-benzoyl)-2,4-dioxo-3,4-dihydropyrimidin-1(2H)-yl)-4-hydroxy-5-(hydroxy methyl)tetrahydrofuran-3-yl)acetonitrile (Compound 5-5) (1.0 g, 2.693 mmol, 1.0 eq) and imidazole (1.46 g, 21.544 mmol, 8.0 eq) in dry dichloromethane (10 mL). The mixture was stirred at room temperature overnight. The mixture was diluted with dichloromethane and washed with water and saturated brine. The organic phase was concentrated in vacuo. The residue was purified by silica gel column chromatography (eluent: petroleum ether/ethyl acetate = 30/1 to 5/1 + 2% dichloromethane) to give 2-((2R,3R,4S,5R)-2-(3-benzoyl-2,4-dioxo-3,4-dihydropyrimidin-1(2H)-yl)-4-((tert-butyldimethylsil yl)oxy)-5-(((tert-butyldimethylsilyl)oxymethyl)tetrahydrofuran-3-yl)acetonitrile as white solid (1.12 g, yield: 69.4%). LCMS (ESI): m/z=600[M+1]⁺.

Step 5g: Preparation of 2-((2R,3R,4S,5R)-2-(3-benzoyl-2,4-dioxo-3,4-dihydropyrimidin-1(2H)-yl)-4-((tert-butyldimethylsil yl)oxy)-5-(hydroxymethyl)tetrahydrofuran-3-yl)acetonitrile (Compound 5-7): Under a nitrogen atmosphere, at 0°C, trifluoroacetic acid/water (4 mL/4 mL) was added to a solution of 2-((2R,3R,4S,5R)-2-(3-benzoyl-2,4-dioxo-3,4-dihydropyrimidin-1(2H)-yl)-4-((tert-butyldimethylsil yl)oxy)-5-(((tert-butyldimethylsilyl)oxymethyl)tetrahydrofuran-3-yl)acetonitrile (Compound 5-6) (1.12 g, 1.867 mmol, 1.0 eq) in tetrahydrofuran (16 mL). The mixture was stirred at 0°C for 4 h. The reaction was quenched with a saturated aqueous sodium bicarbonate solution, and extracted with ethyl acetate. The organic phase was washed with saturated brine, and concentrated in vacuo. The residue was purified by silica gel column chromatography (eluent: petroleum ether/ethyl acetate = 3/1 to 1/1) to give 2-((2R,3R,4S,5R)-2-(3-benzoyl-2,4-dioxo-3,4-dihydropyrimidin-1(2H)-yl)-4-((tert-butyldimethylsil yl)oxy)-5-(hydroxymethyl)tetrahydrofuran-3-yl)acetonitrile as a white solid (760 mg, yield: 84%). LCMS (ESI): m/z=486[M+1]⁺.

Step 5h: Preparation of 2-((2R,3R,4S,5S)-2-(3-benzoyl-2,4-dioxo-3,4-dihydropyrimidin-1(2H)-yl)-4-((tert-butyldimethylsil yl)oxy)-5-formyltetrahydrofuran-3-yl)acetonitrile (Compound 5-8): Under a nitrogen atmosphere, pyridine sulfur trioxide (995 mg, 6.260 mmol, 6.0 eq) was added to dry dimethyl sulfoxide (10 mL).

The mixture was stirred at room temperature for 15 min. Under a nitrogen atmosphere, at 0°C, the mixture was added to a mixture of 2-((2R,3R,4S,5R)-2-(3-benzoyl-2,4-dihydropyrimidin-1(2H)-yl)-4-((tert-butyldimethylsilyl)oxy)-5-(hydroxymethyl)tetrahydrofuran-3-yl)acetonitrile (Compound 5-7) (760 mg, 1.565 mmol, 1.0 eq) and N,N-diisopropylethylamine (1.62 g, 12.52 mmol, 8.0 eq) in dry dichloromethane (15 mL). The mixture was stirred at 0°C for 1.0 h. Ethyl acetate was added and the mixture was washed with water and saturated brine. The organic phase was dried and concentrated in vacuo to give 2-((2R,3R,4S,5S)-2-(3-benzoyl-2,4-dioxo-3,4-dihydropyrimidin-1(2H)-yl)-4-(((tert-butyldimethylsi lyl)oxy)-5-formyltetrahydrofuran-3-yl)acetonitrile as a light yellow solid (716 mg, crude). LCMS (ESI): m/z=484[M+1]⁺.

Step 5i: Preparation of dimethyl((E)-2-((2R,3S,4R,5R)-5-(3-benzoyl-2,4-dioxo-3,4-dihydropyrimidin-1(2H)-yl)-3-((tert-bu tyldimethylsilyl)oxy)-4-(cyanomethyl)tetrahydrofuran-2-yl)vinyl)phosphonate (Compound 5-9): Under a nitrogen atmosphere, at room temperature, potassium tert-butoxide (332 mg, 2.96 mmol, 2.0 eq) was added to a solution of tetramethyl methylenediphosphonate (756 mg, 3.257 mmol, 2.2 eq) in dry tetrahydrofuran (20 mL). The mixture was stirred at room temperature for 15 min. 2-((2R,3R,4S,5S)-2-(3-benzoyl-2,4-dioxo-3,4-dihydropyrimidin-1(2H)-yl)-4-((tert-butyldimethylsil yl)oxy)-5-formyltetrahydrofuran-3-yl)acetonitrile (Compound 5-8) (716 mg, 1.480 mmol, 1.0 eq) was dissolved in dry tetrahydrofuran (5 mL) and added to the above mixture. The mixture was stirred at room temperature for 15 min. The reaction was quenched with an aqueous ammonium chloride solution and extracted with ethyl acetate. The organic phase was dried and concentrated in vacuo. The residue was purified by silica gel column chromatography (eluent: dichloromethane/methanol = 300/1 to 100/1) to give dimethyl((E)-2-((2R,3S,4R,5R)-5-(3-benzoyl-2,4-dioxo-3,4-dihydropyrimidin-1(2H)-yl)-3-(((tert-b utyldimethylsilyl)oxy)-4-(cyanomethyl)tetrahydrofuran-2-yl)vinyl)phosphonate as a white solid (578 mg, yield: 66.3%). LCMS (ESI): m/z=590[M+1]⁺.

Step 5j: Preparation of dimethyl((E)-2-((2R,3S,4R,5R)-5-(3-benzoyl-2,4-dioxo-3,4-dihydropyrimidin-1(2H)-yl)-4-(cyanom ethyl)-3-hydroxytetrahydrofuran-2-yl)vinyl)phosphonate (Compound 5-10): Under a nitrogen atmosphere, at 0°C, triethylamine trihydrofluoride (1.57 g, 9.802 mmol, 10.0 eq) was added dropwise to a solution of dimethyl((E)-2-((2R,3S,4R,5R)-5-(3-benzoyl-2,4-dioxo-3,4-dihydropyrimidin-1(2H)-yl)-3-((tert-bu tyldimethylsilyl)oxy)-4-(cyanomethyl)tetrahydrofuran-2-yl)vinyl)phosphonate (Compound 5-9) (578 mg, 0.980 mmol, 1.0 eq) in dry tetrahydrofuran (25 mL). The mixture was stirred at room temperature overnight. The reaction mixture was diluted with water and extracted with ethyl acetate. The organic phase was washed with water and saturated brine, dried and concentrated in vacuo. The residue was purified by silica gel column chromatography (eluent: dichloromethane/methanol = 200/1 to 30/1) to give dimethyl((E)-2-(((2R,3S,4R,5R)-5-(3-benzoyl-2,4-dioxo-3,4-dihydropyrimidin-1(2H)-yl)-4-(cyano methyl)-3-hydroxytetrahydrofuran-2-yl)vinyl)phosphonate as a white solid (387 mg, yield: 83%). LCMS (ESI): m/z=476[M+1]⁺.

Step 5k: Preparation of (2R,3S,4R,5R)-5-(3-benzoyl-2,4-dioxo-3,4-dihydropyrimidin-1(2H)-yl)-4-(cyanomethyl)-2-((E)-2-( dimethoxyphosphoryl)vinyl)tetrahydrofuran-3-yl(2-cyanoethyl)diisopropylphosphoramidite (Compound 5): Under a nitrogen atmosphere, 4,5-dicyanoimidazole (178 mg, 1.499 mmol, 1.5 eq) was added to a solution of dimethyl((E)-2-(((2R,3S,4R,5R)-5-(3-benzoyl-2,4-dioxo-3,4-dihydropyrimidin-1(2H)-yl)-4-(cyano

methyl)-3-hydroxytetrahydrofuran-2-yl)vinyl)phosphonate (Compound 5-10) (475 mg, 0.999 mmol, 1.0 eq) in dry dichloromethane (3 mL). The mixture was stirred at room temperature for 15 min. 3-(bis(diisopropylamino)phosphino)oxy)propanenitrile (903 mg, 2.997 mmol, 3.0 eq) was added to the above solution. The reaction mixture was stirred at room temperature for 1.0 h. The mixture was diluted with ethyl acetate and washed with aqueous sodium bicarbonate solution and saturated brine. The organic phase was dried and concentrated in vacuo. The residue was purified by silica gel column chromatography (eluent: dichloromethane/petroleum ether/acetone = 2/6/1 to 2/0.5/1 + 2% triethylamine) to give (2R,3S,4R,5R)-5-(3-benzoyl-2,4-dioxo-3,4-dihydropyrimidin-1(2H)-yl)-4-(cyanomethyl)-2-((E)-2-( dimethoxyphosphoryl)vinyl)tetrahydrofuran-3-yl(2-cyanoethyl)diisopropylphosphoramidite as a white solid (488 mg, yield: 72.3%). LCMS (ESI): m/z=593[M-100+18]⁺. ¹H NMR (500 MHz, CDCl₃) δ 7.91 (d, *J* = 8.0 Hz, 2H), 7.59 (t, *J* = 7.4 Hz, 1H), 7.43 (t, *J* = 7.7 Hz, 2H), 7.34 (t, *J* = 7.7 Hz, 1H), 6.82 (dddd, *J* = 41.4, 22.0, 17.2, 4.6 Hz, 1H), 6.06 - 5.93 (m, 2H), 5.88 (t, *J* = 8.5 Hz, 1H), 4.71 (d, *J* = 51.3 Hz, 1H), 4.49 - 4.35 (m, 1H), 3.87 - 3.77 (m, 1H), 3.72 - 3.51 (m, 8H), 2.82 - 2.40 (m, 6H), 1.18 - 1.05 (m, 12H).

### Embodiment 6: Preparation of (2R,3S,4R,5R)-5-(3-benzoyl-2,4-dioxo-3,4-dihydropyrimidin-1(2H)-yl)-2-(E)-2-(dimethoxypho sphoryl)vinyl)-4-(fluoromethyl)tetrahydrofuran-3-yl(2-cyanoethyl)diisopropylphosphoramidit e (Compound 6):

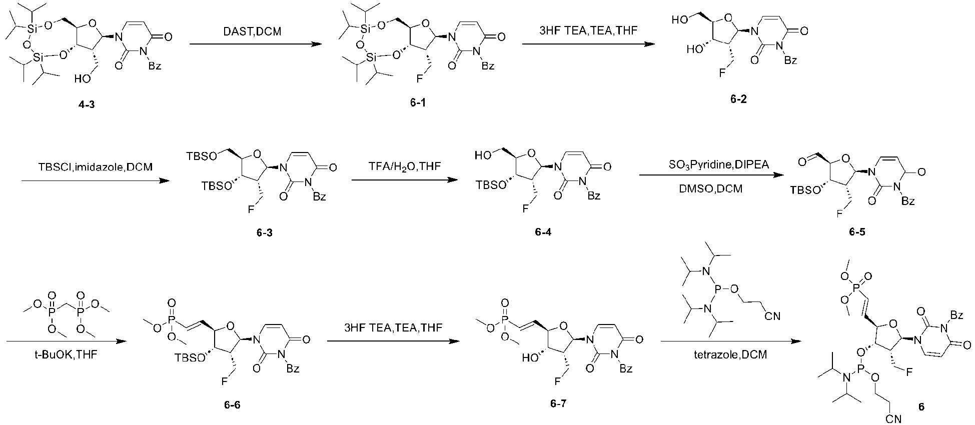

Step 6a: Preparation of 3-benzoyl-1-((6aR,8R,9R,9aS)-9-(fluoromethyl)-2,2,4,4-tetraisopropyltetrahydro-6H-furo[3,2-f][1, 3,5,2,4]trioxadisilocin-8-yl)pyrimidine-2,4(1H,3H)-dione (Compound 6-1): Under a nitrogen atmosphere, at 0°C, diethylaminosulfur trifluoride (1.6 g, 9.9 mmol, 3.0 eq) was added dropwise to a solution of 3-benzoyl-1-((6aR,8R,9R,9aS)-9-(hydroxymethyl)-2,2,4,4-tetraisopropyltetrahydro-6H-furo[3,2-f][ 1,3,5,2,4]trioxadisilocin-8-yl)pyrimidine-2,4(1H,3H)-dione (Compound 4-3) (2 g, 3.3 mmol, 1.0 eq) in dry dichloromethane (80 mL). The mixture was stirred at 0°C for 30 min. The mixture was diluted with dichloromethane and washed with water and saturated brine. The organic phase was dried and concentrated in vacuo. The residue was purified by silica gel column chromatography (eluent: petroleum ether/ethyl acetate = 10/1 to 2/1) to give 3-benzoyl-1-((6aR,8R,9R,9aS)-9-(fluoromethyl)-2,2,4,4-tetraisopropyltetrahydro-6H-furo[3,2-f][1, 3,5,2,4]trioxadisilocin-8-yl)pyrimidine-2,4(1H,3H)-dione as a white solid (1.09 g, yield: 54.5%). LCMS (ESI): m/z = 607[M+1]⁺.

Step 6b: Preparation of 3-benzoyl-1-((2R,3R,4S,5R)-3-(fluoromethyl)-4-hydroxy-5-(hydroxymethyl)tetrahydrofuran-2-yl)p yrimidine-2,4(1H,3H)-dione (Compound 6-2): Under a nitrogen atmosphere, at 0°C, Triethylamine trihydrofluoride (2.0 g, 12.6 mmol, 7.0 eq) was added to a mixture of 3-benzoyl-1-((6aR,8R,9R,9aS)-9-(fluoromethyl)-2,2,4,4-tetraisopropyltetrahydro-6H-furo[3,2-f][1, 3,5,2,4]trioxadisilocin-8-yl)pyrimidine-2,4(1H,3H)-dione (Compound 6-1) (1.09 g, 1.8 mmol, 1.0 eq) and triethylamine (725 mg, 7.2 mmol, 4.0 eq) in dry tetrahydrofuran (10 mL). The mixture was stirred at room temperature overnight. A saturated sodium bicarbonate solution was added to adjust the pH to 6-7. The reaction mixture was extracted with ethyl acetate and washed with water and saturated brine. The organic phase was dried and concentrated in vacuo. The residue was purified by silica gel column chromatography (eluent: dichloromethane/methanol = 50/1 to 20/1) to give 3-benzoyl-1-((2R,3R,4S,5R)-3-(fluoromethyl)-4-hydroxy-5-(hydroxymethyl)tetrahydrofuran-2-yl)p yrimidine-2,4(1H,3H)-dione as a white solid (585.9 mg, yield: 89.6%). LCMS (ESI): m/z = 365[M+1]⁺.

Step 6c: Preparation of 3-benzoyl-1-((2R,3R,4S,5R)-4-((tert-butyldimethylsilyl)oxy)-5-((tert-butyldimethylsilyl)oxy)methy l)-3-(fluoromethyl)tetrahydrofuran-2-yl)pyrimidine-2,4(1H,3H)-dione (Compound 6-3): Under a nitrogen atmosphere, at room temperature, tert-butyldimethylsilyl chloride (1.9 g, 12.5mmol, 4.0 eq) was added to a mixture of 3-benzoyl-1-((2R,3R,4S,5R)-3-(fluoromethyl)-4-hydroxy-5-(hydroxymethyl)tetrahydrofuran-2-yl)p yrimidine-2,4(1H,3H)-dione (Compound 6-2) (1.14 g, 3.1 mmol, 1.0 eq) and imidazole (1.3 g, 18.8 mmol, 6.0 eq) in dichloromethane (10 mL). The mixture was stirred at room temperature overnight. The mixture was diluted with dichloromethane and washed with water and saturated brine. The organic phase was dried and concentrated in vacuo. The residue was purified by silica gel column chromatography (eluent: petroleum ether/ethyl acetate = 10/1 to 2/1) to give 3-benzoyl-1-((2R,3R,4S,5R)-4-((tert-butyldimethylsilyl)oxy)-5-((tert-butyldimethylsilyl)oxy)methy l)-3-(fluoromethyl)tetrahydrofuran-2-yl)pyrimidine-2,4(1H,3H)-dione as a white solid (1.1 g, yield: 61%). LCMS (ESI): m/z = 593[M+1]⁺.

Step 6d: Preparation of 3-benzoyl-1-((2R,3R,4S,5R)-4-((tert-butyldimethylsilyl)oxy)-3-(fluoromethyl)-5-(hydroxymethyl)t etrahydrofuran-2-yl)pyrimidine-2,4(1H,3H)-dione (Compound 6-4): Under a nitrogen atmosphere, at 0°C, trifluoroacetic acid/water = 1/1 (5 mL/5 mL) was added to a solution of 3-benzoyl-1-((2R,3R,4S,5R)-4-((tert-butyldimethylsilyl)oxy)-5-((tert-butyldimethylsilyl)oxy)methy l)-3-(fluoromethyl)tetrahydrofuran-2-yl)pyrimidine-2,4(1H,3H)-dione (Compound 6-3) (1.48 g, 2.49 mmol, 1.0 eq) in tetrahydrofuran (50 mL). The mixture was stirred at 0°C for 3 h. A saturated sodium carbonate solution was added to the ice bath to adjust the pH to 8. The reaction mixture was extracted with ethyl acetate and washed with water and saturated brine. The organic phase was dried and concentrated in vacuo. The residue was purified by silica gel column chromatography (eluent: petroleum ether/ethyl acetate = 5/1 to 2/1) to give 3-benzoyl-1-((2R,3R,4S,5R)-4-((tert-butyldimethylsilyl)oxy)-3-(fluoromethyl)-5-(hydroxymethyl)t etrahydrofuran-2-yl)pyrimidine-2,4(1H,3H)-dione as white solid (1.07 g, yield: 89.9%). LCMS (ESI): m/z = 479[M+1]⁺.

Step 6e: Preparation of (2S,3S,4R,5R)-5-(3-benzoyl-2,4-dioxo-3,4-dihydropyrimidin-1(2H)-yl)-3-((tert-butyldimethylsilyl) oxy)-4-(fluoromethyl)tetrahydrofuran-2-carbaldehyde (Compound 6-5): Under a nitrogen atmosphere, pyridine sulfur trioxide complex (1.4 g, 8.9 mmol, 4.0 eq) was added to dry dimethyl sulfoxide (10 mL). The mixture was stirred at room temperature for 30 min. Under a nitrogen atmosphere, at 0°C, the mixture was added to a mixture of 3-benzoyl-1-((2R,3R,4S,5R)-4-((tert-butyldimethylsilyl)oxy)-3-(fluoromethyl)-5-(hydroxymethyl)t etrahydrofuran-2-yl)pyrimidine-2,4(1H,3H)-dione (Compound 6-4) (1.07 g, 2.3 mmol, 1.0 eq) and diisopropylethylamine (2.3 g, 17.9 mmol, 8.0 eq) in dichloromethane. The mixture was stirred at 0°C for 1 h. Ethyl acetate was added and the mixture was washed with water and saturated brine. The organic phase was dried and concentrated under reduced pressure to give (2S,3S,4R,5R)-5-(3-benzoyl-2,4-dioxo-3,4-dihydropyrimidin-1(2H)-yl)-3-((tert-butyldimethylsilyl) oxy)-4-(fluoromethyl)tetrahydrofuran-2-carbaldehyde as a yellow oil (1.07 g, crude). LCMS (ESI): m/z = 477[M+1]⁺.

Step 6f: Preparation of dimethyl(E)-2-((2R,3S,4R,5R)-5-(3-benzoyl-2,4-dioxo-3,4-dihydropyrimidin-1(2H)-yl)-3-((tert-but yldimethylsilyl)oxy)-4-(fluoromethyl)tetrahydrofuran-2-yl)vinyl)phosphonate (Compound 6-6): Under a nitrogen atmosphere, at room temperature, potassium tert-butoxide (502 mg, 4.4 mmol, 2.0 eq) was added to a solution of tetramethyl methylenediphosphonate (1.14 g, 4.9 mmol, 2.2 eq) in dry tetrahydrofuran (10 mL) and the mixture was stirred for 30 min. (2S,3S,4R,5R)-5-(3-benzoyl-2,4-dioxo-3,4-dihydropyrimidin-1(2H)-yl)-3-((tert-butyldimethylsilyl) oxy)-4-(fluoromethyl)tetrahydrofuran-2-carbaldehyde (1.07 g, 2.2 mmol, 1.0 eq) was dissolved in dry tetrahydrofuran (10 mL) and added to the above mixture. The mixture was stirred at room temperature for 30 min. The reaction was quenched with an aqueous ammonium chloride solution and extracted with ethyl acetate. The organic phase was dried and concentrated under reduced pressure. The residue was purified by silica gel column chromatography (eluent: dichloromethane/methanol = 50/1 to 20/1) to give dimethyl(E)-2-((2R,3S,4R,5R)-5-(3-benzoyl-2,4-dioxo-3,4-dihydropyrimidin-1(2H)-yl)-3-((tert-but yldimethylsilyl)oxy)-4-(fluoromethyl)tetrahydrofuran-2-yl)vinyl)phosphonate as a white solid (687.5 mg, yield: 52.8%). LCMS (ESI): m/z = 583[M+1]⁺.

Step 6g: Preparation of dimethyl(E)-2-((2R,3S,4R,5R)-5-(3-benzoyl-2,4-dioxo-3,4-dihydropyrimidin-1(2H)-yl)-4-(fluorom ethyl)-3-hydroxytetrahydrofuran-2-yl)vinyl)phosphonate (Compound 6-7): Triethylamine (402.6 mg, 3.98 mmol, 4.0 eq) and triethylamine trihydrofluoride (1.1 g, 6.9 mmol, 7.0 eq) were added to a solution of dimethyl(E)-2-((2R,3S,4R,5R)-5-(3-benzoyl-2,4-dioxo-3,4-dihydropyrimidin-1(2H)-yl)-3-((tert-but yldimethylsilyl)oxy)-4-(fluoromethyl)tetrahydrofuran-2-yl)vinyl)phosphonate (Compound 6-6) (581 mg, 0.99 mmol, 1.0 eq) in tetrahydrofuran (5 mL). The mixture was stirred at room temperature overnight. A saturated sodium carbonate solution was added for neutralization and ethyl acetate was added for extraction. The organic phase was washed with water and saturated brine, dried and concentrated under reduced pressure. The residue was purified by silica gel column chromatography (eluent: dichloromethane/methanol = 50/1 to 15/1) to give dimethyl(E)-2-((2R,3S,4R,5R)-5-(3-benzoyl-2,4-dioxo-3,4-dihydropyrimidin-1(2H)-yl)-4-(fluorom ethyl)-3-hydroxytetrahydrofuran-2-yl)vinyl)phosphonate as a white solid (370 mg, yield: 79.3%). LCMS (ESI): m/z = 469[M+1]⁺.

Step 6h: Preparation of (2R,3S,4R,5R)-5-(3-benzoyl-2,4-dioxo-3,4-dihydropyrimidin-1(2H)-yl)-2-(E)-2-(dimethoxyphosph oryl)vinyl)-4-(fluoromethyl)tetrahydrofuran-3-yl(2-cyanoethyl)diisopropylphosphoramidite (Compound 6): Under a nitrogen atmosphere, tetrazole (83 mg, 1.2 mmol, 1.5 eq) was added to a mixture of dimethyl(E)-2-((2R,3S,4R,5R)-5-(3-benzoyl-2,4-dioxo-3,4-dihydropyrimidin-1(2H)-yl)-4-(fluorom ethyl)-3-hydroxytetrahydrofuran-2-yl)vinyl)phosphonate (Compound 6-7) (370 mg, 0.79 mmol, 1.0 eq) and 2-cyanoethyl-N,N,N',N'-tetraisopropylphosphorodiamidite (714.9 mg, 2.4 mmol, 3.0 eq) in dichloromethane (10 mL). The mixture was stirred at room temperature for 1 h. The mixture was diluted with ethyl acetate and washed with aqueous sodium bicarbonate solution and saturated brine. The organic phase was dried and concentrated under reduced pressure. The residue was purified by silica gel column chromatography (eluent: petroleum ether/dichloromethane/acetone = 5/2/1 to 2/2/1 + 2% triethylamine) to give (2R,3S,4R,5R)-5-(3-benzoyl-2,4-dioxo-3,4-dihydropyrimidin-1(2H)-yl)-2-(E)-2-(dimethoxyphosph oryl)vinyl)-4-(fluoromethyl)tetrahydrofuran-3-yl(2-cyanoethyl)diisopropylphosphoramidite as a white solid (470 mg, yield: 89%). LCMS(ESI): m/z =586[M-100+18]⁺. ¹H NMR (500 MHz, CDCl₃) δ 7.87 (t, *J* = 7.3 Hz, 2H), 7.58 (t, *J* = 7.3 Hz, 1H), 7.42 (dd, *J* = 16.3, 8.6 Hz, 2H), 7.39 - 7.33 (m, 1H), 6.84 (dddd, *J* = 27.0, 22.0, 17.2, 4.5 Hz, 1H), 6.12 (dd, *J* = 15.1, 6.2 Hz, 1H), 5.97 (dd, *J* = 37.9, 18.8 Hz, 1H), 5.84 (t, *J* = 8.2 Hz, 1H), 4.83 - 4.69 (m, 1H), 4.68 - 4.61 (m, 1H), 4.57 - 4.41 (m, 2H), 3.81 (tt, *J* = 16.3, 8.0 Hz, 1H), 3.73 - 3.49 (m, 8H), 2.76 - 2.48 (m, 4H), 1.16 - 1.04 (m, 12H).

### Embodiment 7: Preparation of (2R,3S,4R,5R)-5-(3-benzoyl-2,4-dioxo-3,4-dihydropyrimidin-1(2H)-yl)-2-((E)-2-(dimethoxyph osphoryl)vinyl)-4-(2-methoxyethyl)tetrahydrofuran-3-yl(2-cyanoethyl)diisopropylphosphora midite (Compound 7)

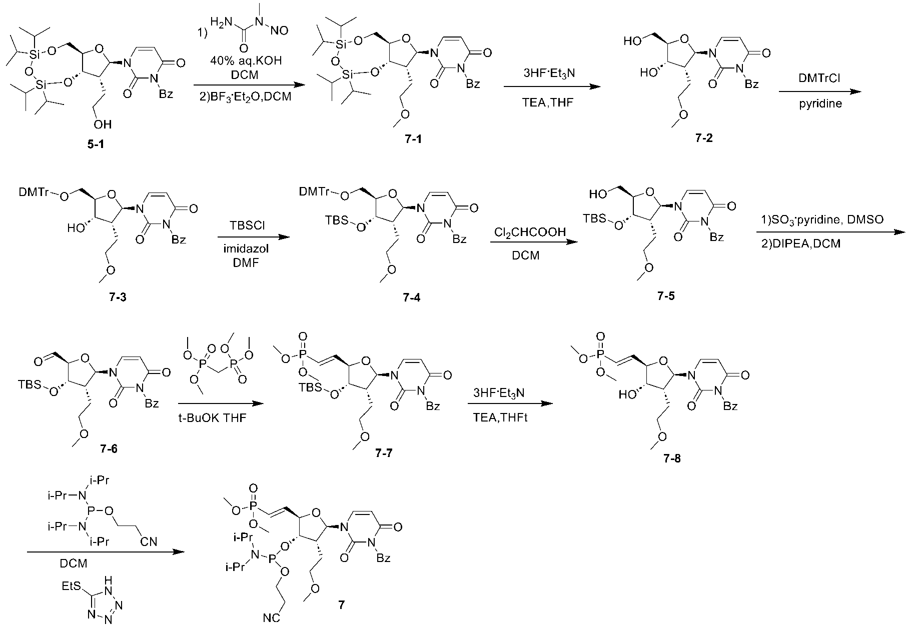

Step 7a: Preparation of 3-benzoyl-1-((6aR,8R,9R,9aS)-2,2,4,4-tetraisopropyl-9-(2-methoxyethyl)tetrahydro-6H-furo[3,2-f][ 1,3,5,2,4]trioxadisilocin-8-yl)pyrimidine-2,4(1H,3H)-dione (Compound 7-1): At -5°C, 1-methyl-1-nitrosourea (8.24 g, 79.9 mmol, 12.35 eq) was added portionwise to a mixture of aqueous potassium hydroxide solution (24 mL) and dichloromethane (80 mL). The mixture was stirred for 30 min. The layers were separated. The organic phase was dried over potassium hydroxide below 0°C for 2 h. At 0°C, the organic phase was added portionwise to a mixture of 3-benzoyl-1-((6aR,8R,9R,9aS)-9-(2-hydroxyethyl)-2,2,4,4-tetraisopropyltetrahydro-6H-furo[3,2-f][ 1,3,5,2,4]trioxadisilocin-8-yl)pyrimidine-2,4(1H,3H)-dione (Compound 5-1) (4.0 g, 6.47 mmol, 1.0 eq) and boron trifluoride diethyl etherate (197.8 mg, 0.65 mmol, 0.1 eq) in dichloromethane (24 mL). The mixture was stirred at 0°C for 1 h and the reaction was quenched with a saturated sodium bicarbonate solution. The organic phase was washed with water and saturated brine, and concentrated under reduced pressure. The residue was purified by silica gel column chromatography (eluent: petroleum ether/ethyl acetate = 8/1 to 2/1) to give 3-benzoyl-1-((6aR,8R,9R,9aS)-2,2,4,4-tetraisopropyl-9-(2-methoxyethyl)tetrahydro-6H-furo[3,2-f][ 1,3,5,2,4]trioxadisilocin-8-yl)pyrimidine-2,4(1H,3H)-dione as white solid (2.86 g, yield: 69.9%). LCMS (ESI): m/z = 633[M+1]⁺.

Step 7b: Preparation of 3-benzoyl-1-((2R,3R,4S,5R)-4-hydroxy-5-hydroxymethyl-3-(2-methoxyethyl)tetrahydrofuran-2-yl) pyrimidine-2,4(1H,3H)-dione (Compound 7-2): Under a nitrogen atmosphere, triethylamine (2.78 g, 27.54 mmol, 6.0 eq) and triethylamine trihydrofluoride (5.54 g, 34.41 mmol, 7.5 eq) were added to a solution of 3-benzoyl-1-((6aR,8R,9R,9aS)-2,2,4,4-tetraisopropyl-9-(2-methoxyethyl)tetrahydro-6H-furo[3,2-f][ 1,3,5,2,4]trioxadisilocin-8-yl)pyrimidine-2,4(1H,3H)-dione (compound 7-1) (2.9 g, 4.59 mmol, 1.0 eq) in tetrahydrofuran (60 mL). The mixture was stirred at room temperature overnight. A saturated sodium carbonate solution was added for neutralization and ethyl acetate was added for extraction. The organic phase was washed with water and saturated brine, dried and concentrated under reduced pressure. The residue was purified by silica gel column chromatography (eluent: dichloromethane/methanol = 50/1 to 25/1) to give 3-benzoyl-1-((2R,3R,4S,5R)-4-hydroxy-5-hydroxymethyl-3-(2-methoxyethyl)tetrahydrofuran-2-yl) pyrimidine-2,4(1H,3H)-dione as a white solid (1.78 g, yield: 99.4%). LCMS (ESI): m/z = 391[M+1]⁺. Step 7c: Preparation of 3-benzoyl-1-((2R,3R,4S,5R)-5-(((4-methoxyphenyl)(phenyl)methoxy)methyl)-4-hydroxy-3-(2-met hoxyethyl)tetrahydrofuran-2-yl)pyrimidine-2,4(1H,3H)-dione (Compound 7-3): Under a nitrogen atmosphere, 4,4'-dimethoxytrityl chloride (2.08 g, 6.14 mmol, 1.2 eq) was added to a solution of 3-benzoyl-1-((2R,3R,4S,5R)-4-hydroxy-5-hydroxymethyl-3-(2-methoxyethyl)tetrahydrofuran-2-yl) pyrimidine-2,4(1H,3H)-dione (Compound 7-2) (2.0 g, 5.12 mmol, 1.0 eq) in pyridine (20 mL). The mixture was stirred at room temperature for 3 h. The mixture was concentrated under reduced pressure to remove pyridine. The residue was dissolved in ethyl acetate and washed with water and saturated brine. The organic phase was dried and concentrated under reduced pressure. The residue was purified by silica gel column chromatography (eluent: petroleum ether/ethyl acetate = 5/1 to 1/1) to give 3-benzoyl-1-((2R,3R,4S,5R)-5-(((4-methoxyphenyl)(phenyl)methoxy)methyl)-4-hydroxy-3-(2-met hoxyethyl)tetrahydrofuran-2-yl)pyrimidine-2,4(1H,3H)-dione as a yellow solid (3.14 g, yield: 88.4%). LCMS (ESI): m/z = 693[M+1]⁺.

Step 7d: Preparation of 3-benzoyl-1-((2R,3R,4S,5R)-5-((bis(4-methoxyphenyl)(phenyl)methoxy)methyl)-4-((tert-butyldime thylsilyl)oxy)-3-(2-methoxyethyl)tetrahydrofuran-2-yl)pyrimidine-2,4(1H,3H)-dione (Compound 7-4): Under a nitrogen atmosphere, tert-butyldimethylsilyl chloride (1.0 g, 6.68 mmol, 2.5 eq) was added to a mixture of 3-benzoyl-1-((2R,3R,4S,5R)-5-(((4-methoxyphenyl)(phenyl)methoxy)methyl)-4-hydroxy-3-(2-met hoxyethyl)tetrahydrofuran-2-yl)pyrimidine-2,4(1H,3H)-dione (Compound 7-3) (1.85 g, 2.67 mmol, 1.0 eq) and imidazole (909 mg, 13.36 mmol, 5.0 eq) in dry dimethylformamide (5.6 mL). The mixture was stirred at 36°C overnight. The mixture was diluted with ethyl acetate and washed with water and saturated brine. The organic phase was dried and concentrated under reduced pressure. The residue was purified by silica gel column chromatography (eluent: petroleum ether/ethyl acetate = 8/1 to 4/1 + 2% triethylamine) to give 3-benzoyl-1-((2R,3R,4S,5R)-5-((bis(4-methoxyphenyl)(phenyl)methoxy)methyl)-4-((tert-butyldime thylsilyl)oxy)-3-(2-methoxyethyl)tetrahydrofuran-2-yl)pyrimidine-2,4(1H,3H)-dione as a yellow solid (1.33 g, yield: 61.8%). LCMS (ESI): m/z = 807[M+1]⁺.

Step 7e: Preparation of 3-benzoyl-1-((2R,3R,4S,5R)-4-((tert-butyldimethylsilyl)oxy)-5-(hydroxymethyl)-3-(2-methoxyethy l)tetrahydrofuran-2-yl)pyrimidine-2,4(1H,3H)-dione (Compound 7-5): Dichloroacetic acid (1.5 mL) was added to a solution of 3-benzoyl-1-((2R,3R,4S,5R)-5-((bis(4-methoxyphenyl)(phenyl)methoxy)methyl)-4-((tert-butyldime thylsilyl)oxy)-3-(2-methoxyethyl)tetrahydrofuran-2-yl)pyrimidine-2,4(1H,3H)-dione (Compound 7-4) (1.34 g, 1.66 mmol, 1.0 eq) in dichloromethane (15 mL). The mixture was stirred at room temperature for 2 h. A saturated sodium bicarbonate solution was added for neutralization. The layers were separated. The organic phase was dried and concentrated under reduced pressure. The residue was purified by silica gel column chromatography (eluent: petroleum ether/ethyl acetate = 1/1 to 1/5) to give 3-benzoyl-1-((2R,3R,4S,5R)-4-((tert-butyldimethylsilyl)oxy)-5-(hydroxymethyl)-3-(2-methoxyethy l)tetrahydrofuran-2-yl)pyrimidine-2,4(1H,3H)-dione as a white solid (680 mg, yield: 81.1%). LCMS (ESI): m/z = 505[M+1]⁺.

Step 7f: Preparation of (2S,3S,4R,5R)-5-(3-benzoyl-2,4-dioxo-3,4-dihydropyrimidin-1(2H)-yl)-3-((tert-butyldimethylsilyl) oxy)-4-(2-methoxyethyl)tetrahydrofuran-2-carbaldehyde (Compound 7-6): Under a nitrogen atmosphere, pyridine sulfur trioxide complex (2.61 g, 16.4 mmol, 8.0 eq) was added to dry dimethyl sulfoxide (8.6 mL, 121.0 mmol, 59.0 eq). The mixture was stirred at room temperature for 1 h. Under a nitrogen atmosphere, at 0°C, this mixture was added to a mixture of 3-benzoyl-1-((2R,3R,4S,5R)-4-((tert-butyldimethylsilyl)oxy)-5-(hydroxymethyl)-3-(2-methoxyethy l)tetrahydrofuran-2-yl)pyrimidine-2,4(1H,3H)-dione (Compound 7-5) (1.03 g, 2.05 mmol, 1.0 eq) and diisopropylethylamine (2.38 g, 18.43 mmol, 9.0 eq) in dichloromethane. The mixture was stirred at 0°C for 1 h. Ethyl acetate was added and the mixture was washed with water and saturated brine. The organic phase was dried and concentrated under reduced pressure to give (2S,3S,4R,5R)-5-(3-benzoyl-2,4-dioxo-3,4-dihydropyrimidin-1(2H)-yl)-3-((tert-butyldimethylsilyl) oxy)-4-(2-methoxyethyl)tetrahydrofuran-2-carbaldehyde as a yellow oil (1.09 g, crude). LCMS (ESI): m/z = 503[M+1]⁺.

Step 7g: Preparation of dimethyl((E)-2-((2R,3S,4R,5R)-5-(3-benzoyl-2,4-dioxo-3,4-dihydropyrimidin-1(2H)-yl)-3-((tert-bu tyldimethylsilyl)oxy)-4-(2-methoxyethyl)tetrahydrofuran-2-yl)vinyl)phosphonate (Compound 7-7): Under a nitrogen atmosphere, at room temperature, potassium tert-butoxide (486 mg, 4.34 mmol, 2.0 eq) was added to a solution of tetramethyl methylenediphosphonate (1.0 g, 4.34 mmol, 2.0 eq) in dry tetrahydrofuran (16 mL) and the mixture was stirred for 1 h. (2S,3S,4R,5R)-5-(3-benzoyl-2,4-dioxo-3,4-dihydropyrimidin-1(2H)-yl)-3-((tert-butyldimethylsilyl) oxy)-4-(2-methoxyethyl)tetrahydrofuran-2-carbaldehyde (Compound 7-6) (1.09 g, 2.17 mmol, 1.0 eq) was dissolved in dry tetrahydrofuran (5 mL) and added to the above mixture. The mixture was stirred at room temperature for 1 h. The reaction was quenched with an aqueous ammonium chloride solution and extracted with ethyl acetate. The organic phase was dried and concentrated under reduced pressure. The residue was purified by silica gel column chromatography (eluent: dichloromethane/methanol = 80/1 to 60/1) to give dimethyl((E)-2-((2R,3S,4R,5R)-5-(3-benzoyl-2,4-dioxo-3,4-dihydropyrimidin-1(2H)-yl)-3-((tert-bu tyldimethylsilyl)oxy)-4-(2-methoxyethyl)tetrahydrofuran-2-yl)vinyl)phosphonate as a white solid (975 mg, yield: 73.8%). LCMS (ESI): m/z = 609[M+1]⁺.

Step 7h: Preparation of dimethyl((E)-2-((2R,3S,4R,5R)-5-(3-benzoyl-2,4-dioxo-3,4-dihydropyrimidin-1(2H)-yl)-3-hydroxy -4-(2-methoxyethyl)tetrahydrofuran-2-yl)vinyl)phosphonate (Compound 7-8): Under a nitrogen atmosphere, triethylamine (971 mg, 9.61 mmol, 6.0 eq) and triethylamine trihydrofluoride (1.94 g, 12.0 mmol, 7.5 eq) were added to a solution of dimethyl((E)-2-((2R,3S,4R,5R)-5-(3-benzoyl-2,4-dioxo-3,4-dihydropyrimidin-1(2H)-yl)-3-((tert-bu tyldimethylsilyl)oxy)-4-(2-methoxyethyl)tetrahydrofuran-2-yl)vinyl)phosphonate (Compound 7-7) (975 mg, 1.60 mmol, 1.0 eq) in tetrahydrofuran (40 mL). The mixture was stirred at room temperature overnight. A saturated sodium carbonate solution was added for neutralization and ethyl acetate was added for extraction. The organic phase was washed with water and saturated brine, dried and concentrated under reduced pressure. The residue was purified by silica gel column chromatography (eluent: dichloromethane/methanol = 50/1 to 15/1) to give dimethyl((E)-2-((2R,3S,4R,5R)-5-(3-benzoyl-2,4-dioxo-3,4-dihydropyrimidin-1(2H)-yl)-3-hydroxy -4-(2-methoxyethyl)tetrahydrofuran-2-yl)vinyl)phosphonate as a white solid (636 mg, yield: 80.3%). LCMS (ESI): m/z = 495[M+1]⁺.

Step 7i: Preparation of (2R,3S,4R,5R)-5-(3-benzoyl-2,4-dioxo-3,4-dihydropyrimidin-1(2H)-yl)-2-((E)-2-(dimethoxyphosp horyl)vinyl)-4-(2-methoxyethyl)tetrahydrofuran-3-yl(2-cyanoethyl)diisopropylphosphoramidite (Compound 7): Under a nitrogen atmosphere, 5-(ethylthio)-1H-tetrazole (219 mg, 1.68 mmol, 1.5 eq) was added to a mixture of dimethyl((E)-2-((2R,3S,4R,5R)-5-(3-benzoyl-2,4-dioxo-3,4-dihydropyrimidin-1(2H)-yl)-3-hydroxy -4-(2-methoxyethyl)tetrahydrofuran-2-yl)vinyl)phosphonate (Compound 7-8) (554 mg, 1.12 mmol, 1.0 eq) and 2-cyanoethyl-N,N,N',N'-tetraisopropylphosphorodiamidite (1.01 g, 3.36 mmol, 3.0 eq) in dichloromethane (13 mL). The mixture was stirred at room temperature for 2 h. The mixture was diluted with ethyl acetate and washed with aqueous sodium bicarbonate solution and saturated brine. The organic phase was dried and concentrated under reduced pressure. The residue was purified by silica gel column chromatography (eluent: petroleum ether/dichloromethane/acetone = 3/1 /1 + 2% triethylamine) to give (2R,3S,4R,5R)-5-(3-benzoyl-2,4-dioxo-3,4-dihydropyrimidin-1(2H)-yl)-2-((E)-2-(dimethoxyphosp horyl)vinyl)-4-(2-methoxyethyl)tetrahydrofuran-3-yl(2-cyanoethyl)diisopropylphosphoramidite as a white solid (659 mg, yield: 84.7%). LCMS (ESI): m/z = 612[M-100+18]⁺. ¹H NMR (500 MHz, CDCl₃) δ 7.88 (d, *J* = 8.1 Hz, 2H), 7.58 (t, *J* = 7.4 Hz, 1H), 7.43 (t, *J* = 7.7 Hz, 2H), 7.33 (dd, *J* = 10.7, 8.4 Hz, 1H), 6.93 - 6.74 (m, 1H), 6.13 - 6.04 (m, 1H), 5.96 (dd, *J* = 34.8, 17.8 Hz, 1H), 5.84 (dd, *J* = 8.2, 3.5 Hz, 1H), 4.67 (d, *J* = 33.2 Hz, 1H), 4.39 - 4.26 (m, 1H), 3.80 (td, *J* = 13.5, 6.8 Hz, 1H), 3.73 - 3.47 (m, 9H), 3.41 - 3.26 (m, 2H), 3.22 (d, *J* = 5.8 Hz, 3H), 2.58 (dt, *J* = 12.4, 6.6 Hz, 2H), 2.34 (ddd, *J* = 21.6, 14.0, 6.8 Hz, 1H), 1.99 - 1.76 (m, 1H), 1.64 (dt, *J* = 20.8, 6.7 Hz, 1H), 1.16 - 1.03 (m, 12H).

### Embodiment 8: Preparation of (2R,3R,4R,5R)-5-(3-benzoyl-2,4-dioxo-3,4-dihydropyrimidin-1(2H)-yl)-2-((E)-2-(dimethoxyph osphoryl)vinyl)-4-(ethylthio)tetrahydrofuran-3-yl(2-cyanoethyl)diisopropylphosphoramidite (Compound 8)

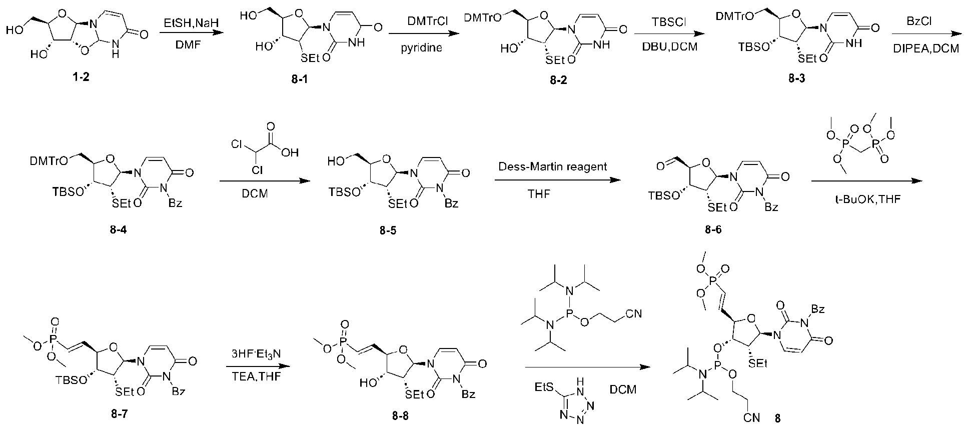

Step 8a: Preparation of 1-((2R,4R,5R)-3-(ethylthio)-4-hydroxy-5-(hydroxymethyl)tetrahydrofuran-2-yl)pyrimidine-2,4(1H, 3H)-dione (Compound 8-1): Under a nitrogen atmosphere, at 0°C, sodium hydride (13.3 g, 331.6 mmol, 5.0 eq) was added to a solution of ethanethiol (2.1 g, 331.6 mmol, 1.0 eq) in dimethylformamide (150 mL). The mixture was stirred at room temperature for 1 h. A solution of (2R,3R,3aR,9aR)-3-hydroxy-2-(hydroxymethyl)-2,3,3a,4a,5,9a-hexahydro-6H-furo[2',3':4,5]oxazol o[3,2-a]pyrimidin-6-one (Compound 1-2) (15 g, 66.3 mmol, 1.0 eq) in N,N-dimethylformamide (10 mL) was added. The mixture was stirred at room temperature for 1 h. The pH was adjusted to 6 with 6M hydrochloric acid solution. The mixture was filtered. The filtrate was concentrated under reduced pressure. The residue was purified by silica gel column chromatography (eluent: dichloromethane/methanol = 15/1 to 8/1) to give 1-((2R,4R,5R)-3-(ethylthio)-4-hydroxy-5-(hydroxymethyl)tetrahydrofuran-2-yl)pyrimidine-2,4(1H, 3H)-dione as a yellow solid (21 g, crude). LCMS (ESI): m/z = 289[M+1]⁺.

Step 8b: Preparation of 1-((2R,3R,4R,5R)-5-((bis(4-methoxyphenyl)(phenyl)methoxy)methyl)-3-(ethylthio)-4-hydroxytetra hydrofuran-2-yl)pyrimidine-2,4(1H,3H)-dione (Compound 8-2): At room temperature, 4,4'-dimethoxytrityl chloride (26.92 g, 79.6 mmol, 1.2 eq) was added to a solution of 1-((2R,4R,5R)-3-(ethylthio)-4-hydroxy-5-(hydroxymethyl)tetrahydrofuran-2-yl)pyrimidine-2,4(1H, 3H)-dione (Compound 8-1) (21 g, 66.3 mmol, 1.0 eq) in pyridine (200 mL). The mixture was stirred at room temperature overnight. The mixture was concentrated under reduced pressure to remove pyridine. The residue was dissolved in ethyl acetate and washed with water and saturated brine. The organic phase was dried and concentrated under reduced pressure. The residue was purified by silica gel column chromatography (eluent: petroleum ether/dichloromethane/ethyl acetate = 1/1/1 to 1/1/3) to give 1-((2R,3R,4R,5R)-5-((bis(4-methoxyphenyl)(phenyl)methoxy)methyl)-3-(ethylthio)-4-hydroxytetra hydrofuran-2-yl)pyrimidine-2,4(1H,3H)-dione as a yellow solid (3.47 g, yield: 8.87%). LCMS (ESI): m/z = 591[M+1]⁺.

Step 8c: Preparation of 1-((2R,3R,4R,5R)-5-((bis(4-methoxyphenyl)(phenyl)methoxy)methyl)-4-((tert-butyldimethylsilyl)o xy)-3-(ethylthio)tetrahydrofuran-2-yl)pyrimidine-2,4(1H,3H)-dione (Compound 8-3): Under a nitrogen atmosphere, tert-butyldimethylsilyl chloride (1.78 g, 11.76 mmol, 2.0 eq) was added to a mixture of 1-((2R,3R,4R,5R)-5-((bis(4-methoxyphenyl)(phenyl)methoxy)methyl)-3-(ethylthio)-4-hydroxytetra hydrofuran-2-yl)pyrimidine-2,4(1H,3H)-dione (Compound 8-2) (3.47 g, 5.88 mmol, 1.0 eq) and 1,8-diazabicyclo[5.4.0]undec-7-ene (1.89 g, 12.35 mmol, 2.1 eq) in dry dichloromethane (35 mL). The mixture was stirred at room temperature overnight. The mixture was diluted with dichloromethane and washed with water and saturated brine. The organic phase was dried and concentrated under reduced pressure. The residue was purified by silica gel column chromatography (eluent: petroleum ether/ethyl acetate = 8/1 to 4/1 + 2% triethylamine) to give 1-((2R,3R,4R,5R)-5-((bis(4-methoxyphenyl)(phenyl)methoxy)methyl)-4-((tert-butyldimethylsilyl)o xy)-3-(ethylthio)tetrahydrofuran-2-yl)pyrimidine-2,4(1H,3H)-dione as a white solid (3.49 g, yield: 84.2%). LCMS (ESI): m/z = 705[M+1]⁺.

Step 8d: Preparation of 3-benzoyl-1-((2R,3R,4R,5R)-5-((bis(4-methoxyphenyl)(phenyl)methoxy)methyl)-4-((tert-butyldim ethylsilyl)oxy)-3-(ethylthio)tetrahydrofuran-2-yl)pyrimidine-2,4(1H,3H)-dione (Compound 8-4): At room temperature, benzoyl chloride (1.05 g, 7.44mmol, 1.5 eq) was added to a mixture of 1-((2R,3R,4R,5R)-5-((bis(4-methoxyphenyl)(phenyl)methoxy)methyl)-4-((tert-butyldimethylsilyl)o xy)-3-(ethylthio)tetrahydrofuran-2-yl)pyrimidine-2,4(1H,3H)-dione (Compound 8-3) (3.49 g, 4.96 mmol, 1.0 eq) and diisopropylethylamine (1.41 g, 10.91 mmol, 2.2 eq) in dichloromethane (35 mL). The mixture was stirred at room temperature overnight. The mixture was diluted with ethyl acetate and washed with water and saturated brine. The organic phase was dried and concentrated under reduced pressure. The residue was purified by silica gel column chromatography (eluent: petroleum ether/ethyl acetate = 10/1 to 6/1 + 2% triethylamine) to give 3-benzoyl-1-((2R,3R,4R,5R)-5-((bis(4-methoxyphenyl)(phenyl)methoxy)methyl)-4-((tert-butyldim ethylsilyl)oxy)-3-(ethylthio)tetrahydrofuran-2-yl)pyrimidine-2,4(1H,3H)-dione as a white solid (3.97 g, yield: 99.2%). LCMS (ESI): m/z = 809[M+1]⁺.

Step 8e: Preparation of 3-benzoyl-1-((2R,3R,4R,5R)-4-((tert-butyldimethylsilyl)oxy)-3-(ethylthio)-5-(hydroxymethyl)tetra hydrofuran-2-yl)pyrimidine-2,4(1H,3H)-dione (Compound 8-5): Dichloroacetic acid (4.0 mL) was added to a solution of 3-benzoyl-1-((2R,3R,4R,5R)-5-((bis(4-methoxyphenyl)(phenyl)methoxy)methyl)-4-((tert-butyldim ethylsilyl)oxy)-3-(ethylthio)tetrahydrofuran-2-yl)pyrimidine-2,4(1H,3H)-dione (Compound 8-4)

(3.97 g, 4.91 mmol, 1.0 eq) in dichloromethane (40 mL). The mixture was stirred at room temperature for 2 h. A saturated sodium bicarbonate solution was added for neutralization. The layers were separated. The organic phase was dried and concentrated under reduced pressure. The residue was purified by silica gel column chromatography (eluent: petroleum ether/ethyl acetate = 10/1 to 2/1) to give 3-benzoyl-1-((2R,3R,4R,5R)-4-((tert-butyldimethylsilyl)oxy)-3-(ethylthio)-5-(hydroxymethyl)tetra hydrofuran-2-yl)pyrimidine-2,4(1H,3H)-dione as a white solid (1.9 g, yield: 76.3%). LCMS (ESI): m/z = 507[M+1]⁺.

Step 8f: Preparation of (2S,3R,4R,5R)-5-(3-benzoyl-2,4-dioxo-3,4-dihydropyrimidin-1(2H)-yl)-3-((tert-butyldimethylsilyl) oxy)-4-(ethylthio)tetrahydrofuran-2-carbaldehyde (Compound 8-6): Under a nitrogen atmosphere, at 0°C, Dess-Martin periodinane (2.15 g, 5.07 mmol, 1.5 eq) was added to a solution of 3-benzoyl-1-((2R,3R,4R,5R)-4-((tert-butyldimethylsilyl)oxy)-3-(ethylthio)-5-(hydroxymethyl)tetra hydrofuran-2-yl)pyrimidine-2,4(1H,3H)-dione (Compound 8-5) (1.71 g, 3.38 mmol, 1.0 eq) in tetrahydrofuran (40 mL). The mixture was stirred at 0°C for 1.5 h. A saturated sodium thiosulfate solution and dichloromethane were added. The organic phase was washed with water and saturated aqueous sodium bicarbonate solution. The organic phase was dried over anhydrous sodium sulfate and concentrated under reduced pressure to give (2S,3R,4R,5R)-5-(3-benzoyl-2,4-dioxo-3,4-dihydropyrimidin-1(2H)-yl)-3-((tert-butyldimethylsilyl) oxy)-4-(ethylthio)tetrahydrofuran-2-carbaldehyde as a yellow oil (1.9 g, crude). LCMS (ESI): m/z = 505[M+1]⁺.

Step 8g: Preparation of dimethyl((E)-2-((2R,3R,4R,5R)-5-(3-benzoyl-2,4-dioxo-3,4-dihydropyrimidin-1(2H)-yl)-3-((tert-bu tyldimethylsilyl)oxy)-4-(ethylthio)tetrahydrofuran-2-yl)vinyl)phosphonate (Compound 8-7): Under a nitrogen atmosphere, at room temperature, potassium tert-butoxide (757 mg, 6.67 mmol, 2.0 eq) was added to a solution of tetramethyl methylenediphosphonate (1.57 g, 6.67 mmol, 2.0 eq) in dry tetrahydrofuran (40 mL). The mixture was stirred at room temperature for 1 h. A solution of (2S,3R,4R,5R)-5-(3-benzoyl-2,4-dioxo-3,4-dihydropyrimidin-1(2H)-yl)-3-((tert-butyldimethylsilyl) oxy)-4-(ethylthio)tetrahydrofuran-2-carbaldehyde (Compound 8-6) (1.9 g, 3.38 mmol, 1.0 eq) in dry tetrahydrofuran (5 mL) was added. The mixture was stirred at room temperature for 1 h. The reaction was quenched with an aqueous ammonium chloride solution and extracted with ethyl acetate. The organic phase was dried and concentrated under reduced pressure. The residue was purified by silica gel column chromatography (eluent: dichloromethane/methanol = 80/1 to 50/1) to give dimethyl((E)-2-((2R,3R,4R,5R)-5-(3-benzoyl-2,4-dioxo-3,4-dihydropyrimidin-1(2H)-yl)-3-((tert-bu tyldimethylsilyl)oxy)-4-(ethylthio)tetrahydrofuran-2-yl)vinyl)phosphonate as a yellow solid (1.77 g, yield: 85.5%). LCMS (ESI): m/z = 611[M+1]⁺.

Step 8h: Preparation of dimethyl(E)-2-((2R,3R,4R,5R)-5-(3-benzoyl-2,4-dioxo-3,4-dihydropyrimidin-1(2H)-yl)-4-(ethylthi o)-3-hydroxytetrahydrofuran-2-yl)vinyl)phosphonate (Compound 8-8): Under a nitrogen atmosphere, triethylamine (1.78 g, 17.58 mmol, 6.0 eq) and triethylamine trihydrofluoride (3.54 g, 21.98 mmol, 7.5 eq) were added to a solution of dimethyl((E)-2-((2R,3R,4R,5R)-5-(3-benzoyl-2,4-dioxo-3,4-dihydropyrimidin-1(2H)-yl)-3-((tert-bu tyldimethylsilyl)oxy)-4-(ethylthio)tetrahydrofuran-2-yl)vinyl)phosphonate (Compound 8-7) (1.77 g, 2.93 mmol, 1.0 eq) in tetrahydrofuran (30 mL). The mixture was stirred at room temperature overnight. A saturated sodium carbonate solution was added for neutralization and ethyl acetate was added for extraction. The organic phase was washed with water and saturated brine, dried and concentrated under reduced pressure. The residue was purified by silica gel column chromatography (eluent: dichloromethane/methanol = 50/1 to 15/1) to give dimethyl((E)-2-((2R,3R,4R,5R)-5-(3-benzoyl-2,4-dioxo-3,4-dihydropyrimidin-1(2H)-yl)-4-(ethylthi o)-3-hydroxytetrahydrofuran-2-yl)vinyl)phosphonate (1.05 g, yield: 72.4%). LCMS (ESI): m/z = 497[M+1]⁺.

Step 8i: Preparation of (2R,3R,4R,5R)-5-(3-benzoyl-2,4-dioxo-3,4-dihydropyrimidin-1(2H)-yl)-2-((E)-2-(dimethoxyphosp horyl)vinyl)-4-(ethylthio)tetrahydrofuran-3-yl(2-cyanoethyl)diisopropylphosphoramidite (Compound 8): Under a nitrogen atmosphere, 5-(ethylthio)-1H-tetrazole (154 mg, 1.19 mmol, 1.5 eq) was added to a mixture of dimethyl(E)-2-((2R,3R,4R,5R)-5-(3-benzoyl-2,4-dioxo-3,4-dihydropyrimidin-1(2H)-yl)-4-(ethylthi o)-3-hydroxytetrahydrofuran-2-yl)vinyl)phosphonate (Compound 8-8) (886 mg, 1.79 mmol, 1.0 eq) and 2-cyanoethyl-N,N,N',N'-tetraisopropylphosphorodiamidite (1.61 g, 5.35 mmol, 3.0 eq) in dichloromethane (15 mL). The mixture was stirred at room temperature for 2 h. The mixture was diluted with ethyl acetate and washed with aqueous sodium bicarbonate solution and saturated brine. The organic phase was dried and concentrated under reduced pressure. The residue was purified by silica gel column chromatography (eluent: petroleum ether/dichloromethane/acetone = 3/1 /1 + 2% triethylamine) to give (2R,3R,4R,5R)-5-(3-benzoyl-2,4-dioxo-3,4-dihydropyrimidin-1(2H)-y1)-2-((E)-2-(dimethoxyphosp horyl)vinyl)-4-(ethylthio)tetrahydrofuran-3-yl(2-cyanoethyl)diisopropylphosphoramidite as a white solid (1.2 g, yield: 96.0%). LCMS(ESI): m/z = 561[M-100+18]⁺. ¹H NMR (500 MHz, CDCl₃) δ 7.87 (dd, *J=* 9.8, 8.6 Hz, 2H), 7.76 (dd, *J* = 8.3*,* 2.1 Hz, 1H), 7.59 (dd, *J=* 13.6, 7.3 Hz, 1H), 7.43 (q, *J=* 7.4 Hz, 2H), 6.87-6.73 (m, 1H), 6.05-5.89 (m, 2H), 5.81 (dd, *J* = 9.8*,* 8.5 Hz, 1H), 5.05 (dd, *J* = 22.7, 3.5 Hz, 1H), 4.47 (t, *J* = 10.5 Hz, 1H), 3.82-3.61 (m, 8H), 3.51 (*dddd, J* = 65.9, 55.9, 32.2, 5.9 Hz, 3H), 2.62-2.43 (m, 4H), 1.20 (d, *J=* 8.3 Hz, 3H), 1.12-1.03 (m, 12H).

### Embodiment 9: Preparation of (R)-2-((R)-1-benzoyl-5-methyl-2,6-dioxo-1,2,3,6-tetrahydropyridin-3-yl)-3-(bis(4-methoxyphe nyl)(phenyl)methoxy)propyl(2-cyanoethyl)diisopropylphosphoramidite (Compound 9)

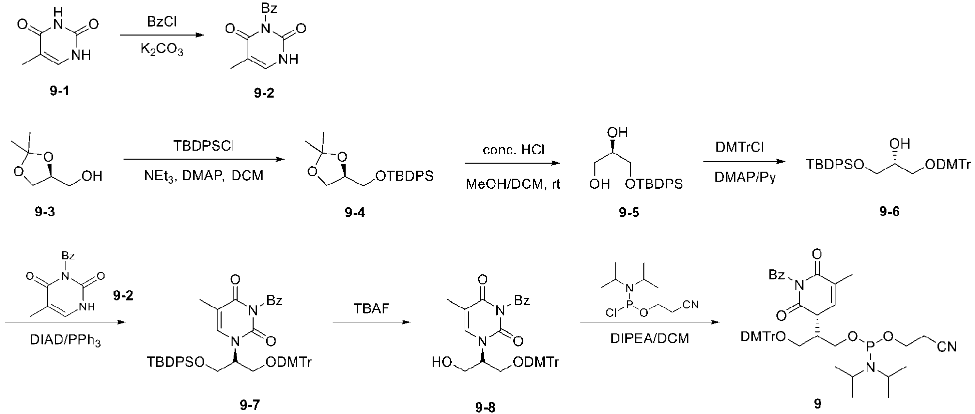

Step 9a: Preparation of 3-benzoyl-5-methylpyrimidine-2,4(1H,3H)-dione (Compound 9-2): 5-methylpyrimidine-2,4(1H,3H)-dione (10 g, 79.4 mmol) was dissolved in anhydrous pyridine and anhydrous toluene, and benzoyl chloride was added. The mixture was stirred overnight at room temperature and concentrated under reduced pressure. The residue was dissolved in 1,4-dioxane (320 mL). Aqueous potassium carbonate solution (180 mL) was added. The mixture was stirred overnight at room temperature. The mixture was extracted with dichloromethane. The organic layer was dried over anhydrous sodium sulfate and concentrated under reduced pressure. The residue was slurried with dichloromethane. The solid was filtered and dried in vacuo to give 3-benzoyl-5-methylpyrimidine-2,4(1H,3H)-dione (Compound 9-2). LCMS (ESI): m/z=231[M+1]⁺. ¹H NMR (500 MHz, DMSO-*d*₆), δ 11.33 (s, 1H), 7.95 (d, *J* = 9.5 Hz, 2H), 7.77 (t, *J* = 7.5 Hz, 1H), 7.64 - 7.58 (m, 2H), 7.52 (s, 1H), 1.82 (s, 3H).

Step 9b: Preparation of (R)-tert-butyl((2,2-dimethyl-1,3-dioxolan-4-yl)methoxy)diphenylsilane (Compound 9-4): Under a nitrogen atmosphere, tert-butylchlorodiphenylsilane was added to a mixture of (S)-(2,2-dimethyl-1,3-dioxolan-4-yl)methanol (5.0 g, 38 mmol), 4-dimethylaminopyridine (370 mg, 3.0 mmol) and triethylamine in dichloromethane. The mixture was stirred overnight at room temperature. The mixture was successively washed with sodium bicarbonate solution and water, dried over anhydrous sodium sulfate, and concentrated in vacuo. The residue was purified by silica gel column chromatography to give (R)-tert-butyl((2,2-dimethyl-1,3-dioxolan-4-yl)methoxy)diphenylsilane (Compound 9-4).

Step 9c: Preparation of (R)-3-((tert-butyldiphenylsilyl)oxy)propane-1,2-diol (Compound 9-5): A solution of concentrated hydrochloric acid in methanol was added to a solution of (R)-tert-butyl((2,2-dimethyl-1,3-dioxolan-4-yl)methoxy)diphenylsilane (Compound 9-4) (12 g, 32 mmol) in dichloromethane. The mixture was stirred at room temperature for 3 h. The mixture was concentrated under reduced pressure. The residue was purified by silica gel column chromatography to give (R)-3-((tert-butyldiphenylsilyl)oxy)propane-1,2-diol (Compound 9-5). ¹H NMR (500 MHz, DMSO-*d*₆), δ 7.70-7.62 (m, 4H), 7.50-7.39 (m, 6H), 4.61 (d, *J* = 4.5 Hz, 1H), 4.43 (t, *J* = 5.6 Hz, 1H), 3.66-3.54 (m, 3H), 3.49 (dt, *J* = 10.2, 4.8 Hz, 1H), 3.39 (dt, *J* = 10.6, 4.9 Hz, 1H), 1.00 (s, 9H). Step 9d: Preparation of (R)-1-(bis(4-methoxyphenyl)(phenyl)methoxy)-3-((tert-butyldiphenylsilyl)oxy)propan-2-ol (Compound 9-6): 4,4'-dimethoxytrityl chloride (3.7 g, 10.9 mmol) was added to a mixture of (R)-3-((tert-butyldiphenylsilyl)oxy)propane-1,2-diol (Compound 9-5) (3.0 g, 9.1 mmol) and 4-dimethylaminopyridine (77 mg, 0.63 mmol) in anhydrous pyridine (40 mL). After the mixture was stirred at room temperature for 3 h, 10 mL of methanol was added and the mixture was stirred for 0.5 h. The mixture was concentrated under reduced pressure. The residue was purified by silica gel column chromatography to give (R)-1-(bis(4-methoxyphenyl)(phenyl)methoxy)-3-((tert-butyldiphenylsilyl)oxy)propan-2-ol (Compound 9-6). ¹H NMR (500 MHz, DMSO-*d*₆), δ 7.65 - 7.49 (m, 4H), 7.49 - 7.34 (m, 8H), 7.31 - 7.24 (m, 6H), 7.21 (dd, *J* = 5.8, 3.8 Hz, 1H), 6.88 - 6.81 (m, 4H), 3.81 (d, *J* = 5.4 Hz, 1H), 3.72 (s, 7H), 3.63 (d, *J* = 5.6 Hz, 2H), 3.13 (dd, *J* = 9.1, 4.8 Hz, 1H), 3.00 (dd, *J* = 9.1, 5.7 Hz, 1H), 0.88 (s, 9H).

Step 9e: Preparation of (R)-3-benzoyl-1-(1-(bis(4-methoxyphenyl)(phenyl)methoxy)-3-((tert-butyldiphenylsilyl)oxy)propa n-2-yl)-5-methylpyrimidine-2,4(1H,3H)-dione (Compound 9-7): Under a nitrogen atmosphere, at 0-5°C, diisopropyl azodicarboxylate was added to a mixture of 3-benzoyl-5-methylpyrimidine-2,4(1H,3H)-dione (Compound 9-2) (800 mg, 3.47 mmol), (R)-1-(bis(4-methoxyphenyl)(phenyl)methoxy)-3-((tert-butyldiphenylsilyl)oxy)propan-2-ol

(Compound 9-6) (1.98 g, 3.13 mmol) and triphenylphosphine (911 mg, 3.47 mmol) in anhydrous tetrahydrofuran. The mixture was stirred at room temperature overnight. The mixture was concentrated under reduced pressure. The residue was purified by silica gel column chromatography to give (R)-3-benzoyl-1-(1-(bis(4-methoxyphenyl)(phenyl)methoxy)-3-((tert-butyldiphenylsilyl)oxy)propa n-2-yl)-5-methylpyrimidine-2,4(1H,3H)-dione (Compound 9-7). ¹H NMR (500 MHz, Chloroform-*d*), δ 7.94 - 7.83 (m, 3H), 7.62 (dt, *J* = 8.0, 1.5 Hz, 6H), 7.53 (s, 2H), 7.49 - 7.39 (m, 11H), 7.38 - 7.32 (m, 3H), 7.29 (dd, *J* = 8.5, 2.7 Hz, 4H), 7.18 (d, *J* = 2.1 Hz, 5H), 6.85 - 6.82 (m, 5H), 4.98 (h, *J* = 5.4, 4.6 Hz, 2H), 3.89 (d, *J* = 4.8 Hz, 1H), 3.80 (s, 7H), 3.78 - 3.71 (m, 1H), 3.55 - 3.44 (m, 1H), 1.97 - 1.93 (m, 3H), 1.09 (s, 9H).

Step 9f: Preparation of (S)-3-benzoyl-1-(1-(bis(4-methoxyphenyl)(phenyl)methoxy)-3-hydroxypropyl-2-yl)-5-methylpyrim idine-2,4(1H,3H)-dione (Compound 9-8): Tetrabutylammonium fluoride was added to a solution of (R)-3-benzoyl-1-(1-(bis(4-methoxyphenyl)(phenyl)methoxy)-3-((tert-butyldiphenylsilyl)oxy)propa n-2-yl)-5-methylpyrimidine-2,4(1H,3H)-dione (Compound 9-7) (1.4 g, 1.63 mmol) in tetrahydrofuran (14 mL). The mixture was stirred at room temperature for 0.5 h. Water was added and the mixture was extracted with dichloromethane. The organic layer was dried over anhydrous sodium sulfate and concentrated under reduced pressure. The residue was purified by silica gel column chromatography to give (S)-3-benzoyl-1-(1-(bis(4-methoxyphenyl)(phenyl)methoxy)-3-hydroxypropyl-2-yl)-5-methylpyrim idine-2,4(1H,3H)-dione as a white foamy solid (Compound 9-8). LCMS (ESI): m/z=605[M-1]⁻ .¹H NMR (500 MHz, Chloroform-*d*), δ 7.93 - 7.87 (m, 2H), 7.62 (t, *J* = 7.5 Hz, 1H), 7.43 (t, *J* = 7.7 Hz, 2H), 7.37 (d, *J* = 7.3 Hz, 2H), 7.34 (s, 1H), 7.32 - 7.27 (m, 4H), 7.26 (s, 4H), 6.84 (d, *J* = 8.4 Hz, 4H), 4.73 - 4.65 (m, 1H), 4.04 - 3.87 (m, 2H), 3.80 (s, 6H), 3.53 (ddd, *J* = 36.4, 10.4, 5.6 Hz, 2H), 1.92 (s, 3H).

Step 9g: Preparation of (R)-2-((R)-1-benzoyl-5-methyl-2,6-dioxo-1,2,3,6-tetrahydropyridin-3-yl)-3-(bis(4-methoxyphenyl)( phenyl)methoxy)propyl(2-cyanoethyl)diisopropylphosphoramidite (Compound 9): Under a nitrogen atmosphere, 2-cyanoethyl N,N-diisopropylchlorophosphoramidite was added to a mixture of (S)-3-benzoyl-1-(1-(bis(4-methoxyphenyl)(phenyl)methoxy)-3-hydroxypropyl-2-yl)-5-methylpyrim idine-2,4(1H,3H)-dione (Compound 9-8) (0.55 g, 0.91 mmol) and N,N-diisopropylethylamine (585 mg, 4.54 mmol) in tetrahydrofuran. The mixture was stirred at room temperature for 1 h. The mixture was concentrated under reduced pressure. The residue was purified by silica gel column chromatography to give (R)-2-((R)-1-benzoyl-5-methyl-2,6-dioxo-1,2,3,6-tetrahydropyridin-3-yl)-3-(bis(4-methoxyphenyl)( phenyl)methoxy)propyl(2-cyanoethyl)diisopropylphosphoramidite as a white foamy solid (Compound 9). LCMS (ESI): m/z=669[M-137]⁻ and m/z=722[M-84]⁻. ¹H NMR (500 MHz, Chloroform-*d*), δ 7.94 - 7.86 (m, 2H), 7.62 (t, *J* = 7.4 Hz, 1H), 7.48 - 7.41 (m, 2H), 7.38 (dd, *J* = 7.5, 3.5 Hz, 2H), 7.34 - 7.27 (m, 4H), 7.23 (d, *J* = 7.1 Hz, 1H), 7.16 (d, *J* = 5.7 Hz, 1H), 6.83 (dt, *J* = 8.8, 2.0 Hz, 4H), 5.00 - 4.87 (m, 1H), 3.98 - 3.82 (m, 2H), 3.79 (s, 6H), 3.77 - 3.65 (m, 2H), 3.58 - 3.44 (m, 4H), 2.54 (dt, *J* = 10.0, 6.2 Hz, 2H), 1.89 (d, *J=* 5.1 Hz, 3H), 1.20 - 1.13 (m, 6H), 1.09 (d, *J* = 6.8 Hz, 6H).

### Embodiment 10: Preparation of (R)-2-(6-benzamido-9H-purin-9-yl)-3-(bis(4-methoxyphenyl)(phenyl)methoxy)propyl(2-cyano ethyl)diisopropylphosphoramidite (Compound 10)

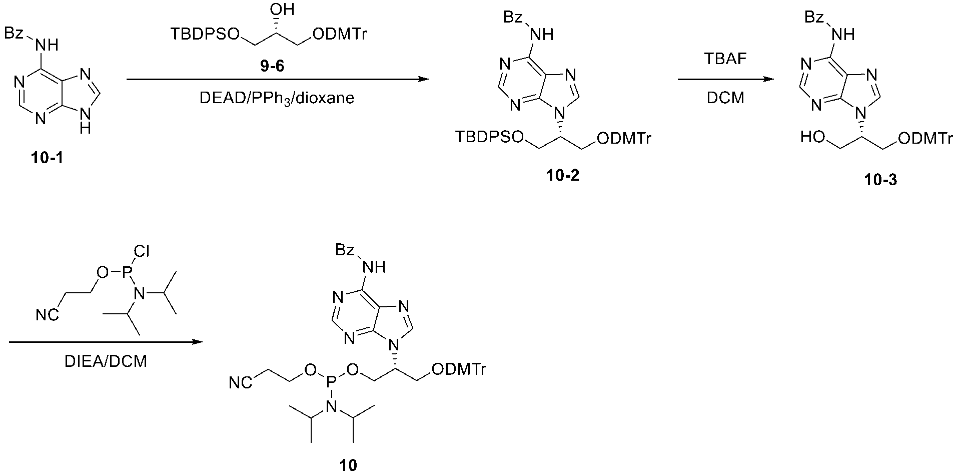

Diethyl azodicarboxylate was added to a mixture of N-(9H-purin-6-yl)benzamide (Compound 10-1) (2.08 g, 1.0 eq), (R)-1-(bis(4-methoxyphenyl)(phenyl)methoxy)-3-((tert-butyldiphenylsilyl)oxy)propan-2-ol (Compound 9-6) (5.5 g, 1.0 eq) and triphenylphosphine (4.6 g, 2.0 eq) in dioxane. The mixture was sonicated for 30 min. The mixture was concentrated under reduced pressure. The residue was purified by silica gel column chromatography to give (R)-N-(9-(1-(bis(4-methoxyphenyl)(phenyl)methoxy)-3-((tert-butyldiphenylsilyl)oxy)propan-2-yl)-9H-purin-6-yl)benzamide (Compound 10-2). LCMS (ESI): m/z=855 [M+1]⁺. Tetrabutylammonium fluoride (12 mL, 4.0 eq, 1 mol/L tetrahydrofuran solution) was added to a solution of (R)-N-(9-(1-(bis(4-methoxyphenyl)(phenyl)methoxy)-3-((tert-butyldiphenylsilyl)oxy)propan-2-yl)-9H-purin-6-yl)benzamide (Compound 10-2) (2.6 g, 1.0 eq) in dichloromethane (210 mL). The mixture was stirred at room temperature for 1 h. The mixture was concentrated under reduced pressure, dissolved in dichloromethane, washed with a saturated aqueous sodium bicarbonate solution, dried over anhydrous sodium sulfate, and concentrated in vacuo. The residue was purified by silica gel column chromatography to give (S)-N-(9-(1-(bis(4-methoxyphenyl)(phenyl)methoxy)-3-hydroxypropyl-2-yl)-9H-purin-6-yl)benza mide as an off-white foamy solid (Compound 10-3). LCMS (ESI): m/z=616[M+1]⁺.¹H NMR (500 MHz, Chloroform-*d*) δ, 9.12 (s, 1H), 8.68 (s, 1H), 8.13 (s, 1H), 8.07 - 8.00 (m, 2H), 7.62 (t, *J* = 7.4 Hz, 1H), 7.53 (t, *J* = 7.6 Hz, 2H), 7.23 - 7.07 (m, 9H), 6.72 (dd, *J* = 11.5, 8.6 Hz, 4H), 4.72 - 4.57 (m, 2H), 4.22 - 4.07 (m, 2H), 3.81 - 3.65 (m, 8H).

Under a nitrogen atmosphere, 2-cyanoethyl N,N-diisopropylchlorophosphoramidite was added to a mixture of (S)-N-(9-(1-(bis(4-methoxyphenyl)(phenyl)methoxy)-3-hydroxypropyl-2-yl)-9H-purin-6-yl)benza mide (Compound 10-3) (1.3 g, 1.0 eq) and N,N-diisopropylethylamine (1.36 g, 5.0 eq) in dichloromethane (13 mL). The mixture was stirred at room temperature for 1 h. The mixture was concentrated under reduced pressure. The residue was purified by silica gel column chromatography to give (R)-2-(6-benzamido-9H-purin-9-yl)-3-(bis(4-methoxyphenyl)(phenyl)methoxy)propyl(2-cyanoethyl )diisopropylphosphoramidite as an off-white foamy solid (Compound 10). LCMS (ESI): m/z=733[M-83]⁻. ¹H NMR (500 MHz, Chloroform-*d*) δ, 9.06 (s, 1H), 8.75 (d, *J* = 4.0 Hz, 1H), 8.09 (dt, *J* = 43.0, 5.5 Hz, 3H), 7.76 - 7.49 (m, 3H), 7.26 (s, 3H), 7.23 - 7.13 (m, 6H), 6.76 (dd, *J* = 8.3, 4.2 Hz, 4H), 5.17 - 4.96 (m, 1H), 4.25 (ddt, *J* = 23.2, 12.2, 6.1 Hz, 1H), 4.08 (ddd, *J* = 38.7, 10.9, 5.6 Hz, 1H), 3.77 (d, *J* = 4.6 Hz, 6H), 3.74 - 3.70 (m, 1H), 3.65 - 3.56 (m, 2H), 3.46 (dq, *J* = 11.7, 6.0 Hz, 2H), 2.61 (d, *J* = 17.1 Hz, 1H), 2.50 (dq, *J* = 17.4, 5.9 Hz, 2H), 1.26 (d, *J* = 4.5 Hz, 5H), 1.16 - 1.07 (m, 7H).

### Embodiment 11: Preparation of (S)-3-(bis(4-methoxyphenyl)(phenyl)methoxy)-2-(4-isobutyramido-2-oxopyrimidin-1(2H)-yl)p ropyl(2-cyanoethyl)diisopropylphosphoramidite (Compound 11)

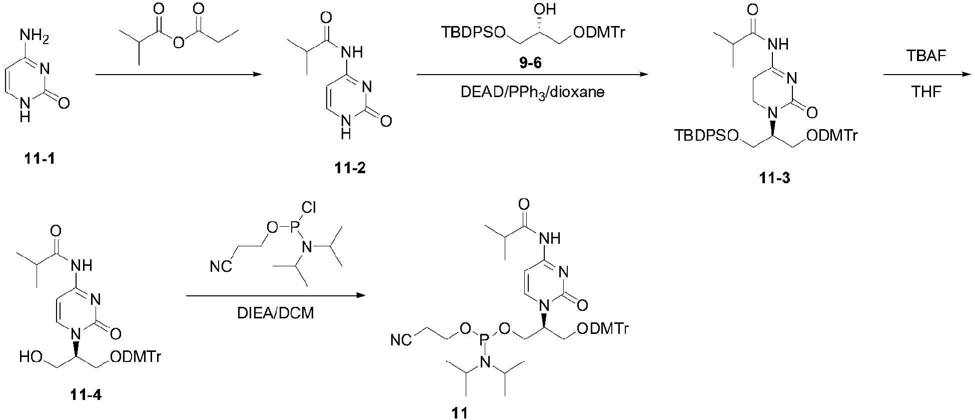

Isobutyric anhydride (6.0 mL, 2.0 eq) was added to a solution of cytosine (Compound **11-1**) (2.0 g, 1.0 eq) in pyridine (100 mL). The mixture was heated to 115°C and stirred for 2 h. After cooling to room temperature, the mixture was concentrated under reduced pressure. Methanol was added and the mixture was stirred at room temperature for 15 min. The solid was filtered and dried in vacuo to give N-(2-oxo-1,2-dihydropyrimidin-4-yl)isobutyramide as an off-white solid (Compound 11-2). LCMS (ESI): m/z=182[M+1]⁺. ¹H NMR (500 MHz, DMSO-*d*₆) δ, 11.45 (s, 1H), 10.69 (s, 1H), 7.79 (d, *J* = 7.0 Hz, 1H), 7.13 (d, *J* = 7.1 Hz, 1H), 2.71 (hept, *J* = 6.8 Hz, 1H), 1.06 (d, *J* = 6.9 Hz, 6H). Under a nitrogen atmosphere and ice bath conditions, diisopropyl azodicarboxylate was added to a mixture of N-(2-oxo-1,2-dihydropyrimidin-4-yl)isobutyramide (Compound 11-2) (2.0 g, 1.0 eq), (R)-1-(bis(4-methoxyphenyl)(phenyl)methoxy)-3-((tert-butyldiphenylsilyl)oxy)propan-2-ol (Compound 9-6) (6.9 g, 1.0 eq) and triphenylphosphine (3.74 g, 1.3 eq) in dioxane. The mixture was stirred at room temperature overnight. The mixture was concentrated under reduced pressure. The residue was purified by silica gel column chromatography to give (S)-N-(1-(1-(bis(4-methoxyphenyl)(phenyl)methoxy)-3-((tert-butyldiphenylsilyl)oxy)propan-2-yl)-2-oxo-1,2-dihydropyrimidin-4-yl)isobutyramide as an off-white foamy solid (Compound 11-3). LCMS (ESI): m/z=794[M-83]⁻.

¹H NMR (500 MHz, Chloroform-*d*) δ 8.34 (d, *J* = 5.5 Hz, 1H), 7.81 - 7.71 (m, 2H), 7.65 - 7.57 (m, 4H), 7.45 - 7.36 (m, 4H), 7.31 - 7.29 (m, 5H), 7.25 - 7.14 (m, 5H), 6.86 - 6.81 (m, 2H), 6.80 - 6.72 (m, 4H), 5.43 (t, *J* = 5.2 Hz, 1H), 3.99 (qd, *J* = 10.6, 5.4 Hz, 2H), 3.76 (d, *J* = 2.4 Hz, 6H), 3.53 - 3.41 (m, 2H), 2.55 (dq, *J* = 13.9, 6.9 Hz, 1H), 1.27 - 1.24 (m, 6H), 0.94 (s, 9H). Tetrabutylammonium fluoride was added to a solution of (S)-N-(1-(1-(bis(4-methoxyphenyl)(phenyl)methoxy)-3-((tert-butyldiphenylsilyl)oxy)propan-2-yl)-2-oxo-1,2-dihydropyrimidin-4-yl)isobutyramide (Compound 11-3) (3.6 g, 1.0 eq) in tetrahydrofuran (215 mL). The mixture was stirred at room temperature for 1 h. The mixture was concentrated under reduced pressure. The residue was dissolved in dichloromethane, washed with saturated aqueous sodium bicarbonate solution, dried over anhydrous sodium sulfate, and concentrated under reduced pressure. The residue was purified by silica gel column chromatography to give (R)-N-(1-(1-(bis(4-methoxyphenyl)(phenyl)methoxy)-3-hydroxypropyl-2-yl)-2-oxo-1,2-dihydropyr imidin-4-yl)isobutyramide as an off-white foamy solid (Compound 11-4). LCMS (ESI): m/z=556[M-1]⁻.

¹H NMR (500 MHz, Chloroform-*d*) δ 8.42 - 8.31 (m, 1H), 8.14 (s, 1H), 7.92 - 7.80 (m, 1H), 7.47 - 7.38 (m, 1H), 7.32 - 7.25 (m, 6H), 7.17 (d, *J* = 8.8 Hz, 3H), 6.91 - 6.71 (m, 4H), 5.31 - 5.13 (m, 1H), 4.01 - 3.87 (m, 3H), 3.79 (d, *J* = 11.4 Hz, 6H), 3.41 (ddd, *J* = 30.1, 9.8, 5.3 Hz, 1H), 2.59 - 2.54 (m, 1H), 1.25 (d, *J* = 7.0 Hz, 6H).

Under a nitrogen atmosphere, 2-cyanoethyl N,N-diisopropylchlorophosphoramidite was added to a mixture of (R)-N-(1-(1-(bis(4-methoxyphenyl)(phenyl)methoxy)-3-hydroxypropyl-2-yl)-2-oxo-1,2-dihydropyr imidin-4-yl)isobutyramide (Compound 11-4) (1.6 g, 1.0 eq) and N,N-diisopropylethylamine (1.82 g, 5.0 eq) in tetrahydrofuran. The mixture was stirred at room temperature for 1 h. The mixture was concentrated under reduced pressure. The residue was purified by silica gel column chromatography to give

(S)-3-(bis(4-methoxyphenyl)(phenyl)methoxy)-2-(4-isobutyramido-2-oxopyrimidin-1(2H)-yl)propy l(2-cyanoethyl)diisopropylphosphoramidite as a white foamy solid (Compound 11). LCMS (ESI): m/z=620[M-137]⁻.

¹H NMR (500 MHz, Chloroform-*d*) δ 8.36 (d, *J* = 5.8 Hz, 1H), 7.95 (d, *J* = 5.6 Hz, 1H), 7.78 (d, *J* = 5.8 Hz, 1H), 7.40 (dd, *J* = 8.1, 3.9 Hz, 2H), 7.29 (dt, *J* = 9.1, 2.9 Hz, 4H), 7.22 (d, *J* = 5.1 Hz, 3H), 7.17 (dt, *J* = 8.1, 4.2 Hz, 1H), 6.78 (dt, *J* = 9.0, 4.6 Hz, 4H), 5.49 (h, *J* = 5.5 Hz, 1H), 4.08 - 3.97 (m, 1H), 3.88 (dq, *J* = 13.0, 6.9 Hz, 1H), 3.76 (d, *J* = 6.7 Hz, 6H), 3.71 (p, *J* = 6.3 Hz, 2H), 3.52 (dq, *J* = 10.7, 6.8 Hz, 2H), 3.40 (dq, *J* = 11.3, 6.2, 5.4 Hz, 2H), 2.54 (tq, *J* = 13.1, 6.8 Hz, 3H), 1.25 (d, *J* = 6.9 Hz, 6H), 1.16 - 1.07 (m, 12H).

### Embodiment 12: Preparation of 9-(2S)-1-(bis(4-methoxyphenyl)(phenyl)methoxy)-3-(((2-cyanoethoxy)(diisopropylamino)phos phono)oxy)propan-2-yl)-2-isobutyrylamino-9H-purin-6-yl diphenylcarbamate (Compound 12)

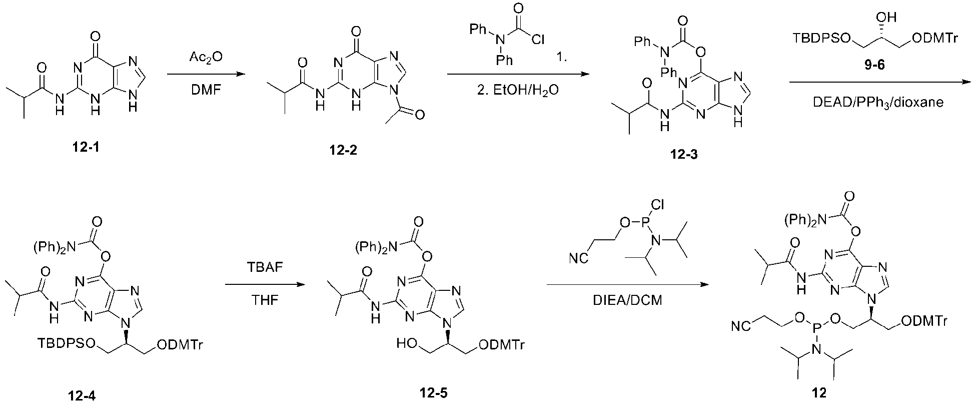

### Acetic anhydride (2.22 mL, 2.6 eq) was added to a solution of

N-(6-oxo-6,9-dihydro-3H-purin-2-yl)isobutyramide (Compound 12-1) (2.0 g, 1.0 eq) in N,N-dimethylformamide (10 mL). The mixture was heated to 100°C and stirred for 2 h. After cooling to room temperature, the solid was filtered, washed with a small amount of ethanol, and dried in vacuo to give N-(9-acetyl-6-oxo-6,9-dihydro-3H-purin-2-yl)isobutyramide as an off-white solid (Compound **12-2).**

¹H NMR (500 MHz, DMSO-*d*₆) δ, 12.26 (s, 1H), 11.66 (s, 1H), 8.44 (s, 1H), 2.83 (s, 4H), 1.14 (d, *J* = 6.8 Hz, 6H).

Diphenylcarbamoyl chloride (1.2 eq) was added to a mixture of

N-(9-acetyl-6-oxo-6,9-dihydro-3H-purin-2-yl)isobutyramide (Compound 12-2) (1.0 g, 1.0 eq) and N,N-diisopropylethylamine (0.98 g, 2.0 eq) in pyridine. The mixture was stirred at room temperature overnight. Ethanol (10 mL) was added to the mixture. The mixture was stirred at room temperature for 10 min, and concentrated under reduced pressure to remove the solvent. Ethanol and water were added. The mixture was heated to 100°C and stirred for 2 h. After cooling to room temperature, the solid was filtered and dried in vacuo to give 2-isobutyramide-9H-purin-6-yl diphenylcarbamate as an off-white solid (Compound **12-3).** LCMS (ESI): m/z=417[M+1]⁺.

¹H NMR (500 MHz, DMSO-*d*₆) δ 13.51 (s, 1H), 10.53 (s, 1H), 8.40 (s, 1H), 7.46 (dt, *J =* 15.3, 7.7 Hz, 8H), 7.32 (t, *J =* 7.3 Hz, 2H), 2.80 (p, *J =* 6.9 Hz, 1H), 1.09 (d, *J =* 6.8 Hz, 6H).

Under a nitrogen atmosphere, diisopropyl azodicarboxylate was added to a mixture of 2-isobutyramide-9H-purin-6-yl diphenylcarbamate (Compound **12-3)** (1.45 g, 1.1 eq), (R)-1-(bis(4-methoxyphenyl)(phenyl)methoxy)-3-((tert-butyldiphenylsilyl)oxy)propan-2-ol (Compound 9-6) (2.0 g, 1.0 eq) and triphenylphosphine (1.66 g, 2.0 eq) in dioxane. The mixture was heated to 65°C and stirred overnight. The mixture was filtered. The filtrate was concentrated under reduced pressure. The residue was purified by silica gel column chromatography to give (S)-9-(1-(bis(4-methoxyphenyl)(phenyl)methoxy)-3-((tert-butyldiphenylsilyl)oxy)propan-2-yl)-2-is obutyramido-9H-purin-6-yl diphenylcarbamate as an off-white foamy solid (Compound 12-4).

¹H NMR (500 MHz, Chloroform-*d*) δ 7.95 (s, 1H), 7.76 (s, 1H), 7.72 - 7.63 (m, 2H), 7.55 (td, *J* = 7.3, 1.5 Hz, 1H), 7.51 - 7.40 (m, 9H), 7.39 - 7.32 (m, 6H), 7.22 (d, *J* = 2.8 Hz, 2H), 7.20 - 7.14 (m, 3H), 7.10 (dd, *J* = 10.3, 8.7 Hz, 4H), 6.76 - 6.66 (m, 4H), 4.78 (q, *J* = 5.6 Hz, 1H), 4.13 - 4.03 (m, 2H), 3.73 (d, *J* = 4.0 Hz, 6H), 3.71 - 3.67 (m, 1H), 3.56 (dd, *J* = 9.9, 4.5 Hz, 1H), 3.07 (s, 1H), 1.19 (dd, *J* = 6.8, 1.8 Hz, 6H), 0.91 (s, 9H).

Tetrabutylammonium fluoride was added to a solution of (S)-9-(1-(bis(4-methoxyphenyl)(phenyl)methoxy)-3-((tert-butyldiphenylsilyl)oxy)propan-2-yl)-2-is obutyramido-9H-purin-6-yl diphenylcarbamate (Compound 12-4) (2.36 g, 1.0 eq) in tetrahydrofuran. The mixture was stirred at room temperature for 20 min. Dichloromethane was added. The mixture was washed with a saturated aqueous sodium bicarbonate solution, dried over anhydrous sodium sulfate, and concentrated under reduced pressure. The residue was purified by silica gel column chromatography to give (R)-9-(1-(bis(4-methoxyphenyl)(phenyl)methoxy)-3-hydroxypropyl-2-yl)-2-isobutyramido-9H-puri n-6-yl diphenylcarbamate as an off-white foamy solid (Compound **12-5**). LCMS (ESI): m/z=793[M+1]⁺.

¹H NMR (500 MHz, Chloroform-*d*) δ 8.01 (d, *J* = 7.0 Hz, 2H), 7.45 (d, *J* = 7.8 Hz, 4H), 7.37 (d, *J* = 7.7 Hz, 4H), 7.26 - 7.19 (m, 4H), 7.13 (dt, *J* = 23.9, 8.2 Hz, 5H), 7.05 (d, *J* = 8.5 Hz, 2H), 6.67 (dd, *J* = 16.7, 8.5 Hz, 4H), 5.78 (s, 1H), 4.38 (dd, *J* = 9.0, 4.4 Hz, 1H), 4.17 - 4.10 (m, 2H), 3.82 - 3.72 (m, 2H), 3.70 (d, *J* = 8.6 Hz, 6H), 2.56 (p, *J* = 6.9 Hz, 1H), 1.28 - 1.23 (m, 6H).

Under a nitrogen atmosphere, 2-cyanoethyl N,N-diisopropylchlorophosphoramidite was added to a solution of (R)-9-(1-(bis(4-methoxyphenyl)(phenyl)methoxy)-3-hydroxypropyl-2-yl)-2-isobutyramido-9H-puri n-6-yl diphenylcarbamate (Compound 12-5) (1.42 g, 1.0 eq) and N,N-diisopropylethylamine (1.16 g, 5.0 eq) in dichloromethane. The mixture was stirred at room temperature for 1 h. The mixture was concentrated under reduced pressure. The residue was purified by silica gel column chromatography to give 9-(2S)-1-(bis(4-methoxyphenyl)(phenyl)methoxy)-3-(((2-cyanoethoxy)(diisopropylamino)phospho no)oxy)propan-2-yl)-2-isobutyrylamino-9H-purin-6-yl diphenylcarbamate as an off-white foamy solid (Compound **12**). LCMS (ESI): m/z=661[M-331]⁻.

¹H NMR (500 MHz, Chloroform-*d*) δ 8.06 (d, *J* = 8.6 Hz, 1H), 7.87 (d, *J* = 7.5 Hz, 1H), 7.44 (d, *J* = 7.9 Hz, 4H), 7.35 (t, *J* = 7.7 Hz, 4H), 7.27 (s, 2H), 7.25 - 7.19 (m, 4H), 7.13 (td, *J* = 8.9, 6.3 Hz, 5H), 6.73 (ddd, *J* = 8.4, 5.4, 2.4 Hz, 4H), 4.91 (t, *J* = 5.9 Hz, 1H), 4.26 - 4.15 (m, 1H), 4.05 - 3.85 (m, 1H), 3.74 (d, *J* = 2.6 Hz, 6H), 3.61 (s, 2H), 3.60 - 3.51 (m, 2H), 3.49 - 3.38 (m, 2H), 3.07 (s, 1H), 2.51 (t, *J* = 6.3 Hz, 1H), 2.45 (td, *J* = 6.2, 3.4 Hz, 1H), 1.26 - 1.23 (m, 6H), 1.10 (dd, *J* = 6.8, 4.6 Hz, 6H), 1.03 (d, *J* = 6.8 Hz, 3H), 0.98 (d, *J* = 6.8 Hz, 3H).

### Embodiment 13 Synthesis of siRNA

Oligonucleotide synthesis at a scale of 0.2-1 µmol was performed on a 12-channel nucleic acid synthesizer (Beijing Tsingke Biotech Co., Ltd.) using a solid-phase oligonucleotide synthesis protocol. The siRNA sequence containing the GalNAc ligand needed to be synthesized on a CPG pre-packed column conjugated with GalNAc. An ammonolysis reagent was added to the synthesized oligonucleotide and the mixture was incubated at 45-80°C to separate the oligonucleotide from the solid support, releasing it into solution. The crude oligonucleotide was then precipitated with ethanol. After high-speed centrifugation, the supernatant was discarded. This was repeated twice to give the crude oligonucleotide and the precipitate was resuspended in DEPC-treated water. The crude oligonucleotide was purified by ion-pairing HPLC, and the collected product was dried to powder in a vacuum centrifugal dryer. The purified product was dissolved in DEPC-treated water and analyzed by TOF LC-MS. The concentration of the oligonucleotide was determined. The volumes required for equimolar amounts of the sense and antisense strands were calculated. The equimolar amounts of the sense and antisense strands were mixed well and annealed (heated at 95°C for 5 min, then naturally cooled to room temperature) to prepare a duplex.

The sequences of the modified siRNA duplexes synthesized by this method are shown in Tables 1-4. All duplexes contain a GalNAc ligand for liver-targeted delivery.

**Table 1 shows the modified siRNA sequences targeting TMPRSS6. The corresponding unmodified sequences are as follows:**

| Sense Strand Sequence 5'-3' | Antisense Strand Sequence 5'-3' |
|---|---|
| UGGGAACUUACUACAACUCCA (SEQ ID NO:1) | UGGAGUUGUAGUAAGUUCCCAGG (SEQ ID NO:2) |
| UGCUACUCUGGUAUUUCCUAA (SEQ ID NO:3) | UUAGGAAAUACCAGAGUAGCACC (SEQ ID NO:4) |

**Table 2 shows the modified siRNA sequences targeting CIDEB. The corresponding unmodified sequences are as follows:**

| Sense Strand Sequence 5'-3' | Antisense Strand Sequence 5'-3' |
|---|---|
| CCAGAGGAGGAUGGAACUGCA (SEQ ID NO:5) | UGCAGUUCCAUCCUCCUCUGG (SEQ ID NO:6) |

**Table 3 shows the modified siRNA sequences targeting ANGPTL3. The corresponding unmodified sequences are as follows:**

| Sense Strand Sequence 5'-3' | Antisense Strand Sequence 5'-3' |
|---|---|
| GCCACUUGAACUCAACUCAAA (SEQ ID NO:7) | UUUGAGUUGAGUUCAAGUGGC (SEQ ID NO:8) |
| CGCAAAACTUCAAUGAAAA (SEQ ID NO:9) | UUUUCAUUGAAGUUUUGUGUU (SEQ ID NO:10) |

**Table 4 shows the modified siRNA sequences targeting PCSK9. The corresponding unmodified sequences are as follows:**

| Sense Strand Sequence 5'-3' | Antisense Strand Sequence 5'-3' |
|---|---|
| CUACAGCCAACUUUUCUAGAA (SEQ ID NO:11) | UUCUAGAAAAGUUGGCUGUAG (SEQ ID NO:12) |
| CUUAUUCUGGGUUUUGUAGCA (SEQ ID NO:13) | UGCUACAAAACCCAGAAUAAG (SEQ ID NO:14) |

**Table 1 Modified siRNA Sequences Targeting TMPRSS6**

| Duplex Name | Modified Sense Strand Name | Modified Sense Strand Sequence 5'-3' | Modified Antisense Strand Name | Modified Antisense Strand Sequence 5'-3' |
|---|---|---|---|---|
| 2051. 10-7 | 2051SM7 | mUmGmGmGmAmAfC mUfUfAfCmUmAmCm AmAmCmUmCmCmA | 2051AM10 | mUfGmGmAmGisoGNA-T mUmGmUfAmGdTmAfA mGfUmUmCmCmCmAm GmG |
| 2051. 20-7 | 2051SM7 | mUmGmGmGmAmAfC mUfUfAfCmUmAmCm AmAmCmUmCmCmA | 2051AM20 | VPUfGmGmAmGisoGNA- TmUmGmUfAmGdTmAfA mGfUmUmCmCmCmAm GmG |
| 2051. 36-7 | 2051SM7 | mUmGmGmGmAmAfC mUfUfAfCmUmAmCm AmAmCmUmCmCmA | 2051AM36 | VPU-SfGmGmAmGisoGN A-TmUmGmUfAmGdTmA fAmGfUmUmCmCmCmA mGmG |
| 2051. 39-7 | 2051SM7 | mUmGmGmGmAmAfC mUfUfAfCmUmAmCm AmAmCmUmCmCmA | 2051AM39 | VPU-6fGmGmAmGisoGN A-TmUmGmUfAmGdTmA fAmGfUmUmCmCmCmA mGmG |
| 2051. 40-7 | 2051SM7 | mUmGmGmGmAmAfC mUfUfAfCmUmAmCm AmAmCmUmCmCmA | 2051AM40 | VPU-7fGmGmAmGisoGN A-TmUmGmUfAmGdTmA fAmGfUmUmCmCmCmA mGmG |
| 2090. 1-7 | 2090SM7 | mUmGmCmUmAmCfU mCfUfGfGmUmAmUm UmUmCmCmUmAmA | 2090AM1 | mUfUmAmGmGmAmAm AmUfAmCdCmAfGmAfG mUmAmGmCmAmCmC |
| 2090. 16-7 | 2090SM7 | mUmGmCmUmAmCfU mCfUfGfGmUmAmUm UmUmCmCmUmAmA | 2090AM16 | VPUfUmAmGmGmAmAm AmUfAmCdCmAfGmAfG mUmAmGmCmAmCmC |
| 2090. 45-7 | 2090SM7 | mUmGmCmUmAmCfU mCfUfGfGmUmAmUm UmUmCmCmUmAmA | 2090AM45 | VPU-SfUmAmGmGmAm AmAmUfAmCdCmAfGm AfGmUmAmGmCmAmC mC |
| 2090. 48-7 | 2090SM7 | mUmGmCmUmAmCfU mCfUfGfGmUmAmUm UmUmCmCmUmAmA | 2090AM48 | VPU-6fUmAmGmGmAm AmAmUfAmCdCmAfGm AfGmUmAmGmCmAmC mC |
| 2090. 49-7 | 2090SM7 | mUmGmCmUmAmCfU mCfUfGfGmUmAmUm UmUmCmCmUmAmA | 2090AM49 | VPU-7fUmAmGmGmAm AmAmUfAmCdCmAfGm AfGmUmAmGmCmAmC mC |

**Table 2 Modified siRNA Sequences Targeting CIDEB**

| Duplex Name | Modified Sense Strand Name | Modified Sense Strand Sequence 5'-3' | Modified Antisense Strand Name | Modified Antisense Strand Sequence 5'-3' |
|---|---|---|---|---|
| W4005. 23-24 | W4005SM24 | Invab*mC*mCmAmG mAmGmGmAfGmGfA mUfGmGmAmAmCm UmGmCmAInvabGalN Ac | W4005AM23 | VPU*fG*mCmAmGmUm UmCmCmAmUdCmCfUm CfCmUmCmU*mG*mG |
| W4005. 37-24 | W4005SM24 | Invab*mC*mCmAmG mAmGmGmAfGmGfA mUfGmGmAmAmCm UmGmCmAInvabGalN Ac | W4005AM37 | VPU-S*fG*mCmAmGmU mUmCmCmAmUdCmCfU mCfCmUmCmU*mG*mG |
| W4005. 38-24 | W4005SM24 | Invab*mC*mCmAmG mAmGmGmAfGmGfA mUfGmGmAmAmCm UmGmCmAInvabGalN Ac | W4005AM38 | VPU-12*fG*mCmAmGmU mUmCmCmAmUdCmCfU mCfCmUmCmU*mG*mG |
| W4005. 39-24 | W4005SM24 | Invab*mC*mCmAmG mAmGmGmAfGmGfA mUfGmGmAmAmCm UmGmCmAInvabGalN Ac | W4005AM39 | VPU-8*fG*mCmAmGmU mUmCmCmAmUdCmCfU mCfCmUmCmU*mG*mG |
| W4005. 45-24 | W4005SM24 | Invab*mC*mCmAmG mAmGmGmAfGmGfA mUfGmGmAmAmCm UmGmCmAInvabGalN Ac | W4005AM45 | VPU*fG*mCmAmGmUiso GNA-TmCmCmAmUdCm CfUmCfCmUmCmU*mG* mG |
| W4005. 46-24 | W4005SM24 | Invab*mC*mCmAmG mAmGmGmAfGmGfA mUfGmGmAmAmCm UmGmCmAInvabGalN Ac | W4005AM46 | VPU-S*fG*mCmAmGmUi soGNA-TmCmCmAmUdC mCfUmCfCmUmCmU*m G*mG |
| W4005. 47-24 | W4005SM24 | Invab*mC*mCmAmG mAmGmGmAfGmGfA mUfGmGmAmAmCm UmGmCmAInvabGalN Ac | W4005AM47 | VPU-9*fG*mCmAmGmU mUmCmCmAmUdCmCfU mCfCmUmCmU*mG*mG |
| W4005. 48-24 | W4005SM24 | Invab*mC*mCmAmG mAmGmGmAfGmGfA mUfGmGmAmAmCm UmGmCmAInvabGalN Ac | W4005AM48 | VPU-10*fG*mCmAmGmU mUmCmCmAmUdCmCfU mCfCmUmCmU*mG*mG |
| W4005. 49-24 | W4005SM24 | Invab*mC*mCmAmG mAmGmGmAfGmGfA mUfGmGmAmAmCm UmGmCmAInvabGalN Ac | W4005AM49 | VPU-11*fG*mCmAmGmU mUmCmCmAmUdCmCfU mCfCmUmCmU*mG*mG |

**Table 3 Modified siRNA Sequences Targeting ANGPTL3**

| Duplex Name | Modified Sense Strand Name | Modified Sense Strand Sequence 5'-3' | Modified Antisense Strand Name | Modified Antisense Strand Sequence 5'-3' |
|---|---|---|---|---|
| 7061. 1-11 | 7061SM11 | Invab*mG*mCmCmAm CmUmUmGfAfAdCmUf CmAmAmCmUmCmA mAmAInvab | 7061AM1 | VPU*fU*mUmGmAmGm UmUmGmAmGdTmUfCm AfAmGmUmG*mG*mC |
| 7061. 3-11 | 7061SM11 | Invab*mG*mCmCmAm CmUmUmGfAfAdCmUf CmAmAmCmUmCmA mAmAInvab | 7061AM3 | VPU-12*fU*mUmGmAm GmUmUmGmAmGdTmUf CmAfAmGmUmG*mG*m C |
| 7061. 4-11 | 7061SM11 | Invab*mG*mCmCmAm CmUmUmGfAfAdCmUf CmAmAmCmUmCmA mAmAInvab | 7061AM4 | VPU-S*fU*mUmGmAmG mUmUmGmAmGdTmUfC mAfAmGmUmG*mG*mC |
| 7061.7-1 1 | 7061SM11 | Invab*mG*mCmCmAm CmUmUmGfAfAdCmUf CmAmAmCmUmCmA mAmAInvab | 7061AM7 | VPU-8*fU*mUmGmAmG mUmUmGmAmGdTmUfC mAfAmGmUmG*mG*mC |
| 7061.8-1 1 | 7061SM11 | Invab*mG*mCmCmAm CmUmUmGfAfAdCmUf CmAmAmCmUmCmA mAmAInvab | 7061AM8 | mU*fU*mUmGmAmGmU mUmGmAmGdTmUfCmA fAmGmUmG*mG*mC |
| 7061.9-1 3 | 7061SM13 | Invab*mC*mGmCmUfU mGfAfAdCmUmCmAm AmCmUmCmAmAmAI nvab | 7061AM9 | VPU*fU*mUmGmAmGm UmUmGmAmGdTmUfCm AfAmGmCmG*mA*mC |
| 7061.11-13 | 7061SM13 | Invab*mC*mGmCmUfU mGfAfAdCmUmCmAm AmCmUmCmAmAmAI nvab | 7061AM11 | VPU-S*fU*mUmGmAmG mUmUmGmAmGdTmUfC mAfAmGmCmG*mA*mC |
| 7061.12-13 | 7061SM13 | Invab*mC*mGmCmUfU mGfAfAdCmUmCmAm AmCmUmCmAmAmAI nvab | 7061AM12 | VPU-9*fU*mUmGmAmG mUmUmGmAmGdTmUfC mAfAmGmCmG*mA*mC |
| 7061.13-13 | 7061SM13 | Invab*mC*mGmCmUfU mGfAfAdCmUmCmAm AmCmUmCmAmAmAI nvab | 7061AM13 | VPU-10*fU*mUmGmAm GmUmUmGmAmGdTmUf CmAfAmGmCmG*mA*m C |
| 7061.14-13 | 7061SM13 | Invab*mC*mGmCmUfU mGfAfAdCmUmCmAm AmCmUmCmAmAmAI nvab | 7061AM14 | VPU-11*fU*mUmGmAmG mUmUmGmAmGdTmUfC mAfAmGmCmG*mA*mC |
| 7079.12-13 | 7079SM13 | Invab*mC*mGmCmAfA mAfAfCdTmUmCmAm AmUmGmAmAmAmAI nvab | 7079AM12 | mU*fU*mUmUmCmAmU mUmGmAmAdGmUfUmU fUmGmUmG*mU*mU |
| 7079.11-13 | 7079SM13 | Invab*mC*mGmCmAfA mAfAfCdTmUmCmAm AmUmGmAmAmAmAI nvab | 7079AM11 | VPU*fU*mUmUmCmAm UmUmGmAmAdGmUfUm UfUmGmUmG*mU*mU |
| 7079.15-13 | 7079SM13 | Invab*mC*mGmCmAfA mAfAfCdTmUmCmAm AmUmGmAmAmAmAI nvab | 7079AM15 | VPU-10*fU*mUmUmCmA mUmUmGmAmAdGmUfU mUfUmGmUmG*mU*mU |

**Table 4: Modified siRNA Sequences Targeting PCSK9**

| Duplex Name | Modified Sense Strand Name | Modified Sense Strand Sequence 5'-3' | Modified Antisense Strand Name | Modified Antisense Strand Sequence 5'-3' |
|---|---|---|---|---|
| 11002.1-4 | 11002SM4 | Invab*mC*mUmAmCmA mGmCmCfAfAdCmUfU mUmUmCmUmAmGmA mAInvabGalNAc | 11002AM1 | VPU*fU*mCmUmAmGm AmAmAmAmGdTmUfGm GfCmUmGmU*mA*mG |
| 11002.3-4 | 11002SM4 | Invab*mC*mUmAmCmA mGmCmCfAfAdCmUfU mUmUmCmUmAmGmA mAInvabGalNAc | 11002AM3 | VPU-S*fU*mCmUmAmG mAmAmAmAmGdTmUfG mGfCmUmGmU*mA*mG |
| 11002.4-4 | 11002SM4 | Invab*mC*mUmAmCmA mGmCmCfAfAdCmUfU mUmUmCmUmAmGmA mAInvabGalNAc | 11002AM4 | VPU-12*fU*mCmUmAm GmAmAmAmAmGdTmUf GmGfCmUmGmU*mA*m G |
| 11040.1-7 | 11040SM7 | Invab*mC*mUmUmAmU mUmCmUfGmGfGmUfU mUmUmGmUmAmGmC mAInvabGalNAc | 11040AM1 | VPU*fG*mCmUmAmCm AmAmAmAmCdCmCfAm GfAmAmUmA*mA*mG |
| 11040.5-7 | 11040SM7 | Invab*mC*mUmUmAmU mUmCmUfGmGfGmUfU mUmUmGmUmAmGmC mAInvabGalNAc | 11040AM5 | VPU-S*fG*mCmUmAmC mAmAmAmAmCdCmCfA mGfAmAmUmA*mA*mG |
| 11040.6-7 | 11040SM7 | Invab*mC*mUmUmAmU mUmCmUfGmGfGmUfU mUmUmGmUmAmGmC mAInvabGalNAc | 11040AM6 | VPU-12*fG*mCmUmAm CmAmAmAmAmCdCmCf AmGfAmAmUmA*mA*m G |
| 11040.7-7 | 11040SM7 | Invab*mC*mUmUmAmU mUmCmUfGmGfGmUfU mUmUmGmUmAmGmC mAInvabGalNAc | 11040AM7 | VPU-8*fG*mCmUmAmC mAmAmAmAmCdCmCfA mGfAmAmUmA*mA*mG |
| 11040.8-7 | 11040SM7 | Invab*mC*mUmUmAmU mUmCmUfGmGfGmUfU mUmUmGmUmAmGmC mAInvabGalNAc | 11040AM8 | mU*fG*mCmUmAmCmA mAmAmAmCdCmCfAmG fAmAmUmA*mA*mG |

### Embodiment 14 In Vitro Activity Testing of siRNA in Hep3B Cells Using Liposome-mediated Transfection

Cell culture and 96-well plate transfection: In vitro experiments were conducted in Hep3B cells using MEM (BasalMedia, REF. L130KJ) + 10% FBS + 1X penicillin-streptomycin (Gibco, Cat. 15070-063) + 1X non-essential amino acids (Cell Cook, Cat. CM1008L). When cells reached 80% confluence, they were digested with trypsin. Cell density was determined using a Scepter automated cell counter (Millipore, #PHCC00000). Simultaneously, 2 µL of siRNA was mixed with 68 µL of Opti-MEM and 5 µL of INTERFERin (Polyplus transfection, REF.409-10) in a 96-well plate and the plate was incubated at room temperature for 10 min. Then, 125 µL of Hep3B cell complete culture medium (containing 7000 cells) was added to each well. The 96-well plate was placed in a 37°C, 5% CO₂ incubator and incubated for 24 h.

96-well plate RNA extraction and reverse transcription: mRNA was extracted from cells in the 96-well plate using the Dynabeads mRNA DIRECT Kit (Ambion). The culture medium was aspirated from the 96-well plate, and the wells were washed once with DPBS. 100 µL of cell lysis buffer was added to each well, followed by 20 µL of beads. The plate was shaken on an oscillator and then placed on a magnetic separation rack. The lysate was aspirated from the wells. 100 µL of wash buffer A was added to each well, pipetted up and down, and the plate was placed back on the magnetic separation rack. Wash buffer A was aspirated. The beads were then pipetted up with wash buffer B and transferred to a new 96-well plate. The plate was placed on the magnetic separation rack, wash buffer B was aspirated, and the beads were pipetted up with wash buffer B and transferred to a 96-well PCR plate. Simultaneously, the reverse transcription reagent was prepared. The 96-well PCR plate was placed on the magnetic separation rack, wash buffer B was aspirated, and 20 µL of reverse transcription reagent was added to each well. The plate was sealed with sealing film and incubated in a PCR instrument as follows: 25°C for 10 min, 37°C for 2 h, 85°C for 5 min, then cooled to 4°C to complete reverse transcription.

Real-time fluorescent quantitative PCR: After reverse transcription, the 96-well plate was placed on the magnetic separation rack until all beads were adsorbed to the bottom. The reverse transcription reagent was aspirated. The prepared QPCR system was added to the 96-well PCR plate. The plate was sealed with sealing film, and PCR was performed on a StepOnePlus Real-Time PCR System (Applied Biosystems).

Data were analyzed using the ΔΔCt method and normalized using cells transfected with 1 nM of the negative control sequence.
Negative control AD-1955 sequence: CUUACGCUGAGUACUUCGAdTdT;
UCGAAGUACUCAGCGUAAGdTdT.
Primers and probes for human genes are shown in Tables 5 and 6.

**Table 5 Primers for Human Genes**

| **Target Genes** | **Forward Primer** | **Reverse Primer** |
|---|---|---|
| hTMPRSS6 | CCGCCAAAGCCCAGAAG (SEQ ID NO:15) | GGTCCCTCCCCAAAGGAATAG (SEQ ID NO:16) |
| hCIDEB | CAGCGACCTTTCCGTGTCT (SEQ ID NO:17) | GGGTCTCCAATGCTTTGGCT (SEQ ID NO:18) |
| hAGT | ATTCTGCACACCGAGCTGAA (SEQ ID NO:19) | TCAAGCTCAAAAAAAATGCTGTTC (SEQ ID NO:20) |
| hANGPTL3 | ACCATTTATAACAGAGGTGA ACATACAAG(SEQ ID NO:21) | CCTGATATAACATCACAGTAGACA TGAAAA(SEQ ID NO:22) |
| hPCSK9 | ACGTGGCTGGCATTGCA (SEQ ID NO:23) | AAGTGGATCAGTCTCTGCCTCAA (SEQ ID NO:24) |

**Table 6 Probes for Human Genes**

| **Target Genes** | **Probes for TaqMan PCR Detection** | **Reporter Group** | **Quencher Group** |
|---|---|---|---|
| hTMPRSS6 | CAGCACCCGCCTGGGAACTTACTACAAC (SEQ ID NO:25) | 5'FAM | 3'MGB |
| hCIDEB | ACCATCCGGAAAGGCCTGACAGCTGCCA (SEQ ID NO:26) | 5'FAM | 3'BHQ1 |
| hAGT | CTGCAAAAATTGAGCAATGACCGCATC (SEQ ID NO:27) | 5'FAM | 3'MGB |
| hANGPTL3 | TGTATGCCATCAGACCCAGCAACTCTCA (SEQ ID NO:28) | 5'FAM | 3'BHQ1 |
| hPCSK9 | CATGATGCTGTCTGCCGAGCCG (SEQ ID NO:29) | 5'FAM | 3'BHQ1 |

**Table 7 Results of Liposome Delivery Activity of 0.1 nM siRNA in Hep3B Cells**

| siRNA | | | Residual mRNA Levels in Hep3B Cells (%) (relative to negative control cells) | |
|---|---|---|---|---|
| Target | Duplex Name | Special Modification | Mean | SD |
| | 2090.1-7 | No VPU | 67.5 | 2.1 |
| hTMPRSS6 | 2090.16-7 | VPU | 42.3 | 8.7 |
| | 2090.45-7 | VPU-S | 33.5 | 7.3 |

As shown in Table 7, introducing VPU-S into sequence 2090 significantly improved sequence activity in Hep3B cells, with a better improvement effect than that achieved by introducing VPU.

### Embodiment 15 In Vitro Activity Testing of siRNA in Wild-type Mice, AAV8-hTMPRSS6 Mice, AAV8-hCIDEB Mice, hANGPTL3 Mice or hPCSK9 Mice Using Liposomal-mediated Transfection or GalNAc Delivery

First, the hTMPRSS6 (human TMPRSS6) gene, hCIDEB (human CIDEB) gene or hANGPTL3 (human ANGPTL3) gene was individually integrated into a liver-targeting AAV8 expression vector. The virus [VectorBuilder, AAV8LP] was prepared and used to infect mice. After 14 days, transgenic mice stably expressing hANGPTL3, hTMPRSS6 or hCIDEB were obtained. hANGPTL3 or hPCSK9 mice were purchased from Shanghai Model Organisms.

Isolation of mouse primary hepatocytes: Mouse hepatocytes were isolated by collagenase digestion via inferior vena cava perfusion. After filtration through a 70 µm cell strainer (BIOLOGIX, 15-1070), viable mouse primary hepatocytes were obtained, resuspended in DMEM (BasalMedia, REF. L110KJ) + 10% FBS + 1X penicillin-streptomycin (Gibco, Cat.15070-063). Cell density was determined using a Scepter automated cell counter (Millipore, #PHCC00000). Cell plating and sample detection methods are described in Embodiment 14.

**Table 8 Results of GalNAc Delivery Activity of 1 nM siRNA in AAV8-hTMPRSS6 Mouse Primary Hepatocytes**

| siRNA | | | Residual mRNA Levels in AAV8-hTMPRSS6 Mouse Primary Hepatocytes (%) (relative to negative control cells) | |
|---|---|---|---|---|
| Target | Duplex Name | Special Modification | Mean | SD |
| | 2090.1-7 | No VPU | 4.0 | 4.2 |
| | 2090.16-7 | VPU | 2.0 | 2.1 |
| hTMPRSS6 | 2090.45-7 | VPU-S | 0.7 | 0.4 |
| | 2090.48-7 | VPU-6 | 0.7 | 0.3 |
| | 2090.49-7 | VPU-7 | 0.5 | 0.5 |

As shown in Table 8, introducing VPU-S, VPU-6, or VPU-7 into sequence 2090 significantly improved sequence activity compared to not introducing VPU in AAV8-hTMPRSS6 mouse primary hepatocytes, with a better improvement effect than that achieved by introducing VPU.

**Table 9 Results of GalNAc Delivery Activity of 0.1 nM siRNA in AAV8-hTMPRSS6 Mouse Primary Hepatocytes**

| siRNA | | | Residual mRNA Levels in AAV8-hTMPRSS6 Mouse Primary Hepatocytes (%) (relative to negative control cells) | |
|---|---|---|---|---|
| Target | Duplex Name | Special Modifica tion | Mean | SD |
| hTMPRSS6 | 2051.10-7 | No VPU | 48.7 | 31.9 |
| | 2051.20-7 | VPU | 20.0 | 8.7 |
| | 2051.36-7 | VPU-S | 6.5 | 1.6 |
| | 2051.39-7 | VPU-6 | 3.9 | 0.7 |
| | 2051.40-7 | VPU-7 | 5.7 | 0.8 |

As shown in Table 9, introducing VPU-S, VPU-6, or VPU-7 into sequence 2051 significantly improved sequence activity in AAV8-hTMPRSS6 mouse primary hepatocytes, with a better improvement effect than that achieved by introducing VPU.

**Table 10 Results of GalNAc Delivery Activity of 1 nM siRNA in AAV8-hCIDEB Mouse Primary Hepatocytes**

| siRNA | | | Residual mRNA Levels in AAV8-hCIDEB Mouse Primary Hepatocytes (%) (relative to negative control cells) | |
|---|---|---|---|---|
| Target | Duplex Name | Special Modification | Mean | SD |
| | W4005.23-24 | VPU | 10.45 | 1.58 |
| | W4005.37-24 | VPU-S | 5.72 | 0.75 |
| hCIDEB | W4005.47-24 | VPU-9 | 6.82 | 1.23 |
| | W4005.48-24 | VPU-10 | 7.87 | 0.58 |
| | W4005.49-24 | VPU-11 | 5.63 | 0.37 |

**Table 11 Results of Liposome Delivery Activity of 0.1 nM siRNA in AAV8-hCIDEB Mouse Primary Hepatocytes**

| siRNA | | | Residual mRNA Levels in AAV8-hCIDEB Mouse Primary Hepatocytes (%) (relative to negative control cells) | |
|---|---|---|---|---|
| Target | Duplex Name | Special Modificati on | Mean | SD |
| hCIDEB | W4005.45-24 | VPU | 15.66 | 4.81 |
| | W4005.46-24 | VPU-S | 8.9 | 2.08 |

As shown in Tables 10-11, introducing VPU-9, VPU-10, VPU-11 and VPU-S into sequence W4005 improved sequence activity compared to introducing VPU in AAV8-hCIDEB mouse primary hepatocytes.

**Table 12 Results of GalNAc Delivery Activity of 1 nM siRNA in hANGPTL3 Mouse Primary Hepatocytes**

| siRNA | | | Residual mRNA Levels in hANGPTL3 Mouse Primary Hepatocytes (%) (relative to negative control cells) | |
|---|---|---|---|---|
| Target | Duplex Name | Special Modification | Mean | SD |
| | 7061.1-11 | VPU | 55.8 | 8.6 |
| hANGPTL3 | 7061.3-11 | VPU-12 | 37.1 | 2.8 |
| | 7061.4-11 | VPU-S | 38.1 | 1.2 |

As shown in Table 12, introducing VPU-S and VPU-12 into sequence 7061 improved sequence activity compared to introducing VPU in hANGPTL3 mouse primary hepatocytes.

**Table 13 Results of GalNAc Delivery Activity of 1 nM siRNA in AAV8-hANGPTL3 Mouse Primary Hepatocytes**

| siRNA | | | Residual mRNA Levels in AAV8-hANGPTL3 Mouse Primary Hepatocytes (%) (relative to negative control cells) | |
|---|---|---|---|---|
| Target | Duplex Name | Special Modification | Mean | SD |
| | 7061.9-13 | VPU | 17.2 | 4.6 |
| | 7061.11-13 | VPU-S | 9.9 | 3.8 |
| hANGPTL3 | 7061.12-13 | VPU-9 | 12.2 | 1.5 |
| | 7061.13-13 | VPU-10 | 6.8 | 4.1 |
| | 7061.14-13 | VPU-11 | 9.5 | 1.2 |

**Table 14 Results of GalNAc Delivery Activity of 1 nM siRNA in AAV8-hANGPTL3 Mouse Primary Hepatocytes**

| siRNA | | | Residual mRNA Levels in AAV8-hANGPTL3 Mouse Primary Hepatocytes (%) (relative to negative control cells) | |
|---|---|---|---|---|
| Target | Duplex Name | Special Modification | Mean | SD |
| | 7061.8-11 | No VPU | 12.2 | 4.6 |
| hANGPTL3 | 7061.1-11 | VPU | 5.7 | 2.7 |
| | 7061.7-11 | VPU-8 | 17.7 | 5.0 |

As shown in Tables 13 and 14, introducing VPU-S, VPU-9, VPU-10 and VPU-11 into sequence 7061 improved sequence activity compared to introducing VPU in AAV8-hANGPTL3 mouse primary hepatocytes. Among these, VPU-10 resulted in the best activity of sequence 7061, while VPU-8 did not improve the activity of sequence 7061.

**Table 15 Results of GalNAc Delivery Activity of 1 nM siRNA in AAV8-hANGPTL3 Mouse Primary Hepatocytes**

| siRNA | | | Residual mRNA Levels in AAV8-hANGPTL3 Mouse Primary Hepatocytes (%) (relative to negative control cells) | |
|---|---|---|---|---|
| Target | Duplex Name | Special Modification | Mean | SD |
| | 7079.12-13 | No VPU | 19.4 | 4.0 |
| hANGPTL3 | 7079.11-13 | VPU | 14.3 | 4.5 |
| | 7079.15-13 | VPU-10 | 9.5 | 5.4 |

As shown in Table 15, introducing VPU-10 into sequence 7079 improved sequence activity in AAV8-hANGPTL3 mouse primary hepatocytes. In contrast, VPU resulted in a less significant improvement in the activity of sequence 7079 compared to VPU-10.

**Table 16 Results of GalNAc Delivery Activity of 1 nM siRNA in hPCSK9 Mouse Primary Hepatocytes**

| siRNA | | | Residual mRNA Levels in hPCSK9 Mouse Primary Hepatocytes (%) | |
|---|---|---|---|---|
| | | | (relative to negative control cells) | |
| Target | Duplex Name | Special Modificati on | Mean | SD |
| hPCSK9 | 11040.1-7 | VPU | 22.3 | 2.1 |
| | 11040.5-7 | VPU-S | 16.1 | 0.6 |
| | 11040.6-7 | VPU-12 | 15.4 | 1.0 |

As shown in Table 16, introducing VPU-S and VPU-12 into sequence 11040 significantly improved sequence activity compared to introducing VPU in hPCSK9 mouse primary hepatocytes.

### Embodiment 16 Evaluation of the Effects of Differently Modified 11040 on hPCSK9 Expression in the Serum of AAV8-hPCSK9 Mice

### 1. Adenovirus Integration of hPCSK9

Transgenic mice stably expressing hPCSK9 were obtained by infecting mice with 1x10¹¹ ultra-purified recombinant AAV8 virus particles [Vector Builder]. 100 µL of virus was added to 3.9 mL of DPBS, and 200 µL of the diluted virus solution was administered intravenously to each mouse.

### 2. Grouping and Administration

Blood was collected from the cut tails of mice 14 days after virus injection. Samples were allowed to stand at room temperature for 30 min, then centrifuged at 1000 × g for 10 min. The supernatant serum was collected, aliquoted, and frozen at -80°C. Serum samples were diluted 1000-fold and analyzed for hPCSK9 expression in mouse serum using a human PCSK9 sandwich ELISA kit (Proteintech, Catalog No.: KE00278). Mice were equally divided into groups based on hPCSK9 expression levels, with 4 mice per group. siRNA was dissolved in PBS to a concentration of 0.2 mg/mL, and mice were administered the siRNA solution subcutaneously at a dose of 1 mg/kg.

### 3. ELISA Test

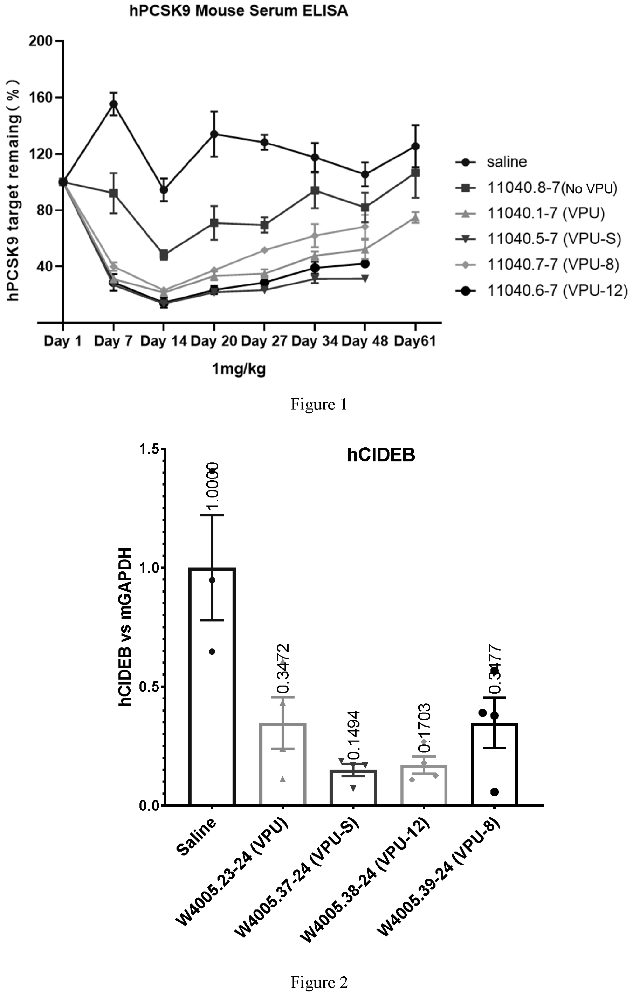

At specified days post-siRNA injection, as shown in Figure 1, a small amount of blood was collected from the cut tail. Samples were allowed to stand at room temperature for 30 min, and centrifuged at 1000 × g for 10 min. The supernatant serum was collected, diluted 1000-fold, and analyzed for hPCSK9 expression by ELISA.

As shown in Figure 1, compared to introducing VPU into sequence 11040, introducing VPU-S and VPU-12 more significantly improved the activity of sequence 11040. VPU-S exhibited a more obvious effect on improving the activity of sequence 11040 compared to VPU-12. VPU-8 also improved the activity of sequence 11040, but it was less obvious than VPU.

### Embodiment 17 Evaluation of the Effects of Differently Modified W4005 on hCIDEB Expression in the Liver of AAV8-hCIDEB Mice

### 1. Adenovirus Integration of hCIDEB

Transgenic mice stably expressing hCIDEB were obtained by injecting 1.5×10¹¹ purified recombinant AAV8 virus particles into 6-8 week-old female mice (C57BL/6).

### 2. Administration

The mice were equally divided into groups, with 4 mice per group, 14 days after virus injection. siRNA was dissolved in normal saline and administered subcutaneously at a dose of 1 mg/kg.

### 3. Liver Collection and Testing

Livers were collected 57 days after siRNA injection. RNA was extracted from liver tissue using TRI REAGENT (MRC, Cat. No.: TR118). The extracted RNA was reverse transcribed into cDNA using a PrimeScript RT Reagent Kit (Takara, Cat. No.: RR047A). The prepared QPCR system was added to a 96-well PCR plate, the plate was sealed with sealing film, and QPCR was conducted on a

StepOnePlus real-time PCR System (Applied Biosystems) to detect hCIDEB expression.

As shown in Figure 2, compared to introducing VPU into sequence W4005, introducing VPU-S and VPU-12 significantly improved the activity of sequence W4005, while VPU-8 exhibited comparable *in vivo* activity to VPU.

In summary, the present invention finds that introducing VPUS, VPU-6, VPU-7, VPU-9, VPU-10, VPU-11, and VPU-12 at the first position of the 5'-terminal of the siRNA antisense strand has a better effect on improving siRNA activity than VPU. VPU-8 exhibits comparable activity to VPU both *in vivo* and *in vitro*.

The technical features of the above embodiments can be combined arbitrarily. For a concise description, not all possible combinations of the technical features in the above embodiments are described. However, any combination of these technical features shall be deemed within the scope of the Description as long as no contradictions exist among the features.

The above embodiments only express several embodiments of the present invention. Although the description is relatively specific and detailed, it cannot be understood as limiting the patent scope of the present invention. It is to be pointed out that ordinarily skilled persons in the art may make several modifications and improvements without departing from the concept of the present invention, which all fall within the protection scope of the present invention. Therefore, the protection scope of the present invention shall be subject to the attached claims.

## Claims

1. A modified nucleotide compound having a structure shown in formula (I) or a pharmaceutically acceptable salt thereof or a stereoisomer thereof, 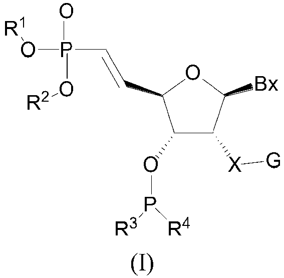 wherein,
R¹ and R² are each independently selected from: H, C₁-C₆ alkyl, C₂-C₆ alkenyl, C₂-C₆ alkynyl, C₃-C₈ cycloalkyl, and C₃-C₈ cycloalkylmethyl;
R³ is selected from: -OR⁵;
R⁴ is selected from: -N(R⁶)(R⁷);
R⁵ is selected from: H, C₁-C₆ alkyl, C₁-C₆ alkyl substituted with cyano, C₁-C₆ alkyl substituted with halogen, C₁-C₆ alkyl substituted with hydroxyl, C₁-C₆ alkyl substituted with C₁-C₆ alkoxy, C₁-C₆ alkyl substituted with amino, and C₁-C₆ alkyl substituted with C₁-C₆ alkylamino;
R⁶ and R⁷ are each independently selected from: C₁-C₆ alkyl, C₂-C₆ alkenyl, C₂-C₆ alkynyl, C₃-C₈ cycloalkyl, and C₃-C₈ cycloalkylmethyl;
X is selected from: -C(R⁸)(R⁹)-, -S-;
R⁸ and R⁹ are each independently selected from: H, C₁-C₆ alkyl, and halogen;
G is selected from: H, halogen, cyano, C₁-C₁₀ alkyl, C₂-C₁₀ alkenyl, C₂-C₁₀ alkynyl, C₃-C₈ cycloalkyl, C₃-C₈ cycloalkylmethyl, C₁-C₁₀ alkoxy, C₁-C₁₀ alkyl substituted with halogen, C₁-C₁₀ alkyl substituted with hydroxyl, C₁-C₁₀ alkyl substituted with C₁-C₆ alkoxy, C₁-C₁₀ alkyl substituted with amino, C₁-C₁₀ alkyl substituted with C₁-C₆ alkylamino, and C₁-C₁₀ alkyl substituted with cyano; Bx is a heterocyclic base.

2. The modified nucleotide compound or a pharmaceutically acceptable salt thereof or a stereoisomer thereof according to claim 1, **characterized in that** R¹ and R² are each independently selected from: H, C₁-C₃ alkyl, C₂-C₃ alkenyl, C₂-C₃ alkynyl, C₃-C₆ cycloalkyl, and C₃-C₆ cycloalkylmethyl.

3. The modified nucleotide compound or a pharmaceutically acceptable salt thereof or a stereoisomer thereof according to claim 2, **characterized in that** R¹ and R² are each independently selected from: H, methyl, ethyl, n-propyl, and isopropyl.

4. The modified nucleotide compound or a pharmaceutically acceptable salt thereof or a stereoisomer thereof according to claim 1, **characterized in that** R⁵ is selected from: H, C₁-C₃ alkyl, C₁-C₃ alkyl substituted with cyano, C₁-C₃ alkyl substituted with halogen, C₁-C₃ alkyl substituted with hydroxyl, C₁-C₃ alkyl substituted with C₁-C₃ alkoxy, C₁-C₃ alkyl substituted with amino, and C₁-C₃ alkyl substituted with C₁-C₃ alkylamino.

5. The modified nucleotide compound or a pharmaceutically acceptable salt thereof or a stereoisomer thereof according to claim 4, **characterized in that** R⁵ is selected from: H, methyl, ethyl, n-propyl, isopropyl, methyl substituted with cyano, ethyl substituted with cyano, and n-propyl substituted with cyano.

6. The modified nucleotide compound or a pharmaceutically acceptable salt thereof or a stereoisomer thereof according to claim 1, **characterized in that** R⁶ and R⁷ are each independently selected from: C₁-C₃ alkyl, C₂-C₃ alkenyl, C₂₋C₃ alkynyl, C₃-C₆ cycloalkyl, and C₃-C₆ cycloalkylmethyl.

7. The modified nucleotide compound or a pharmaceutically acceptable salt thereof or a stereoisomer thereof according to claim 6, **characterized in that** R⁶ and R⁷ are each independently selected from: methyl, ethyl, n-propyl, and isopropyl.

8. The modified nucleotide compound or a pharmaceutically acceptable salt thereof or a stereoisomer thereof according to claim 1, **characterized in that** X is selected from: -S-, -CH₂-, -C(H)(F)-, and -CF₂-.

9. The modified nucleotide compound or a pharmaceutically acceptable salt thereof or a stereoisomer thereof according to claim 1, **characterized in that** G is selected from: H, halogen, cyano, C₁-C₆ alkyl, C₂-C₆ alkenyl, C₂-C₆ alkynyl, C₃-C₆ cycloalkyl, C₃-C₆ cycloalkylmethyl, C₁-C₆ alkoxy, C₁-C₆ alkyl substituted with halogen, C₁-C₆ alkyl substituted with hydroxyl, C₁-C₆ alkyl substituted with C₁-C₆ alkoxy, C₁-C₆ alkyl substituted with amino, C₁-C₆ alkyl substituted with C₁-C₆ alkylamino, and C₁-C₆ alkyl substituted with cyano.

10. The modified nucleotide compound or a pharmaceutically acceptable salt thereof or a stereoisomer thereof according to claim 9, **characterized in that** G is selected from: H, halogen, cyano, C₁-C₃ alkyl, C₁-C₃ alkyl substituted with halogen, C₂-C₃ alkenyl, C₂-C₃ alkynyl, C₁-C₃ alkoxy, and C₁-C₃ alkyl substituted with C₁-C₃ alkoxy.

11. The modified nucleotide compound or a pharmaceutically acceptable salt thereof or a stereoisomer thereof according to claim 10, **characterized in that** G is selected from: H, methyl, ethyl, propyl, isopropyl, monofluoromethyl, difluoromethyl, trifluoromethyl, monofluoroethyl, difluoroethyl, vinyl, propenyl, methoxy, ethoxy, methoxymethyl, methoxyethyl, cyano, fluorine, and chlorine.

12. The modified nucleotide compound or a pharmaceutically acceptable salt thereof or a stereoisomer thereof according to claim 1, **characterized in that** X is -S-; G is selected from: C₁-C₃ alkyl.

13. The modified nucleotide compound or a pharmaceutically acceptable salt thereof or a stereoisomer thereof according to claim 1, **characterized in that** X is -CH₂-; G is selected from: C₁-C₃ alkyl, C₂-C₃ alkenyl, C₂-C₃ alkynyl, C₁-C₂ alkyl substituted with C₁-C₃ alkoxy, methyl substituted with fluorine, cyano, fluorine, and chlorine.

14. The modified nucleotide compound or a pharmaceutically acceptable salt thereof or a stereoisomer thereof according to claim 1, **characterized in that** the -X-G formed by X and G together is selected from: methylthio, ethylthio, propylthio, methyl, ethyl, propyl, butyl, allyl, methoxymethyl, methoxyethyl, methoxypropyl, ethoxymethyl, ethoxyethyl, ethoxypropyl, methyl substituted with cyano, ethyl substituted with cyano, monofluoromethyl, difluoromethyl, monofluoroethyl, and difluoroethyl.

15. The modified nucleotide compound or a pharmaceutically acceptable salt thereof or a stereoisomer thereof according to any one of claims 1-14, **characterized in that** Bx is selected from: adenine, guanine, cytosine, uracil, thymine, methyladenine, 1-methylguanine, isoguanine, 7-methylguanine, methylcytosine, isocytosine, pseudouracil, 5-hydroxymethylcytosine, hypoxanthine, Q-substituted adenine, Q-substituted guanine, Q-substituted cytosine, Q-substituted uracil, and Q-substituted thymine; wherein Q is selected from: H, benzoyl, and benzyloxymethyl.

16. The modified nucleotide compound or a pharmaceutically acceptable salt thereof or a stereoisomer thereof according to claim 1, **characterized in that** the modified nucleotide compound is selected from: 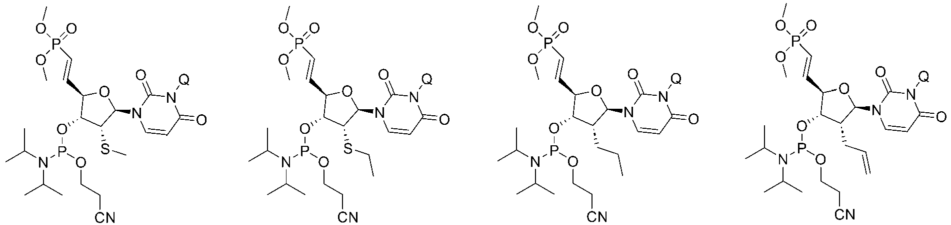 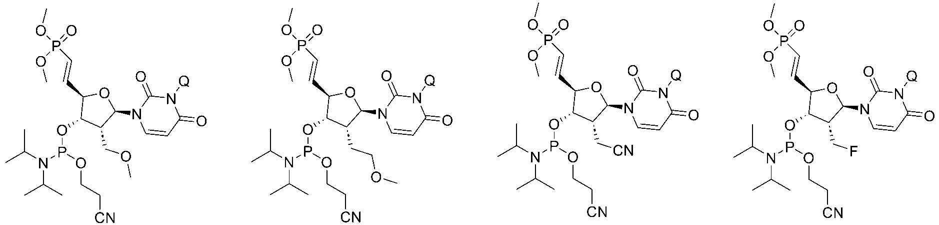 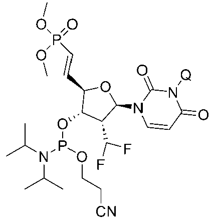 wherein, Q is selected from: H, benzoyl, and benzyloxymethyl.

17. An oligonucleotide, **characterized in that** at least one 5'-terminal nucleotide monomer is connected to the oligonucleotide by a modified nucleotide compound according to any one of claims 1-16 via a linking group; wherein the oligonucleotide has the structural formula as follows: 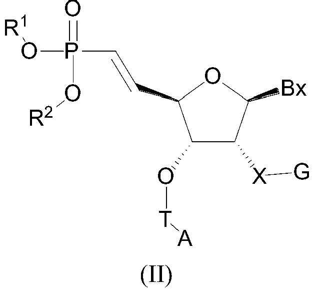
wherein, R¹, R², Bx, X, and G are as described in any one of claims 1-16;
A is the nucleotide chain in the oligonucleotide excluding the 5'-terminal nucleotide monomer;
T is the linking group connecting the modified nucleotide compound to the internucleoside of the oligonucleotide.

18. The oligonucleotide according to claim 17, **characterized in that** T is selected from: a phosphodiester bond, and a phosphorothioate bond.

19. The oligonucleotide according to claim 17 or 18, **characterized in that** the oligonucleotide is selected from: siRNA, shRNA, antisense oligonucleotide, microRNA, anti-microRNA, antimir, supermir, antagomir, ribozyme, triplex-forming oligonucleotide, bait oligonucleotide, splice-switching oligonucleotide, immunostimulatory oligonucleotide, RNA activator, U1 adaptor and CRISPR Cas, or a combination thereof.

20. The oligonucleotide according to claim 19, **characterized in that** the oligonucleotide is siRNA.

21. The oligonucleotide according to claim 20, **characterized in that** the modified nucleotide compound is connected to the 5'-terminus of the siRNA antisense strand via a T linking group.

22. Use of the modified nucleotide compound according to any one of claims 1-16 in the preparation of an oligonucleotide for inhibiting or activating gene expression.

23. The use according to claim 22, **characterized in that** the oligonucleotide is selected from: siRNA, shRNA, antisense oligonucleotide, microRNA, anti-microRNA, antimir, supermir, antagomir, ribozyme, triplex-forming oligonucleotide, bait oligonucleotide, splice-switching oligonucleotide, immunostimulatory oligonucleotide, RNA activator, U1 adaptor and CRISPR Cas, or a combination thereof.

24. The use according to claim 23, **characterized in that** the oligonucleotide is siRNA.

25. The use according to claim 24, **characterized in that** the modified nucleotide compound is connected to the 5'-terminus of the siRNA antisense strand via a covalent linking group.

26. The use according to claim 25, **characterized in that** the covalent linking group is a phosphodiester bond or a phosphorothioate bond.

27. Use of the modified nucleotide compound according to any one of claims 1-16, or the oligonucleotide according to any one of claims 17-21, in the preparation of a drug for inhibiting or activating gene expression.

28. A pharmaceutical preparation for inhibiting or activating gene expression, **characterized in that** it is prepared from an active ingredient and a pharmaceutically acceptable excipient, wherein the active ingredient comprises the oligonucleotide according to any one of claims 17-21.

29. A method for inhibiting or activating gene expression, **characterized in that** it comprises the following steps: administering a safe and effective amount of the oligonucleotide according to any one of claims 17-21 and/or the pharmaceutical preparation according to claim 28.
